# EUROPEAN PATENT APPLICATION

(11) **EP 4 682 154 A1**
(43) Date of publication of application: **21.01.2026**
(21) Application number: 24306204.9
(22) Date of filing: 17.07.2024
(51) Int. Cl.: C07H 15/18, C07H 1/00

(54) **PROTECTED TETRASACCHARIDES, THEIR PROCESS OF PREPARATION AND THEIR USE IN THE SYNTHESIS OF OLIGOSACCHARIDES REPRESENTING SEGMENTS OF O-ANTIGENS FROM DIVERSE SHIGELLA FLEXNERI SEROTYPES**

(71) Applicant: Institut Pasteur, 75015 Paris (FR); Centre National de la Recherche Scientifique, 75016 Paris (FR)
(72) Inventor: Mulard, Laurence, 75015 PARIS (FR); Le Heiget, Guillaume, 75015 PARIS (FR); Tamigney Kenfack, Marielle, 75015 PARIS (FR)
(74) Representative: Gevers & Orès

(57) **Abstract**

The present invention provides protected tetrasaccharides, their process of preparation and their use as common scaffold precursors in the synthesis of oligosaccharides representing segments of the O-antigens from diverse *S. flexneri* serotypes, comprising for example SF2a, SF3a, SF1b, and/or SFY.

## Description

The present invention provides protected tetrasaccharides, their process of preparation and their use as common scaffold precursors in the synthesis of oligosaccharides representing segments of the O-antigens from diverse *S. flexneri* serotypes, comprising for example SF2a, SF3a, SF1b, and/or SFY.

Carbohydrates displayed at the surface of cells and pathogens are of great therapeutic potential. On the one hand, the human glycome is being scrutinized in detail, on the other hand increasing knowledge on microbial carbohydrates and carbohydrate binding proteins offers new openings for therapeutic and prophylactic interventions. Among a large diversity of applications, carbohydrates are actively investigated as vaccine components. In this context, synthetic carbohydrates represent an attractive alternative to carbohydrate antigens of biological origin. The licensing of QuimiHib^{®}, over a decade ago, demonstrated feasibility. Other vaccine candidates derived from synthetic oligosaccharides are under development whether targeting infectious diseases or non-transmittable diseases such as cancer.

Owing to an increasing interest in well-defined carbohydrates, progress in synthetic methodologies to complex oligosaccharides evolve rapidly. Reports on the use of scaffolds compatible with customized modifications opening the way to a diversity of targets have emerged, especially related to the synthesis of highly diverse complex N-glycans. Chemo-enzymatic strategies, mostly relying on the use of glycosyltransferases at the latest stages of the synthesis, are highly attractive. These two approaches were successfully combined to deliver a library of N-glycans. Similarly, glycorandomization/glycodiversification find wide applications. Yet, drawbacks in the use of glycosyltransferases include enzyme availability, added to cost and availability of the sugar-nucleotide donors.

An alternative strategy consists in the use of "engineered transglycosidase/low cost donor" systems adapted to the customization of non-natural acceptors for the chemo-enzymatic synthesis of carbohydrates and glycoconjugates. This strategy was explored in the context of *Shigella flexneri.* Using mono-, di- and tetrasaccharides, feasibility was demonstrated for non-natural acceptors. This method provides an access to a number of targets, However, subsequent site-specific modification and/or chain elongation of the products of enzymatic glycosylation would require complex enzyme engineering and/or sophisticated chemical manipulation.

Shigellosis is a major diarrheal disease. It is responsible for a large burden of disease worldwide and is a leading cause of diarrheal deaths due to bacterial infection. *Shigella* affects especially children under 5 years of age in low- and middle-income countries (LMICs). Besides direct mortality and morbidity, it is a direct cause of stunting in this population. Shigellosis is a general problem also causing disease among travelers, military and non-governmental organization staff deployed in endemic areas. In high-income countries, it occurs in the form of outbreaks with some communities, such as young children in daycare and people in locations with poor sanitation, being particularly affected. Of additional concern in terms of public health threat is the intercontinental spread of antimicrobial resistant strains, in particular by travelers, including multidrug resistant strains.

Disease is caused by Gram-negative bacteria of the genus *Shigella.* Bacteria are transmitted via the fecal-oral route, through direct person-to-person contact or indirectly upon contact with contaminated food or water. The *Shigella* bacillus comprises four species, or groups A-D, which are subdivided in more than 50 serotypes and subtypes. *S. flexneri* (group B) consists of 15 or more serotypes and subtypes, whereas *S. sonnei* (group D) features only 1 serotype. In combination, these two species (*S*. *flexneri* and *S. sonnei*) account for approximately 90% of all cases of endemic shigellosis worldwide. While the latter dominates in transitional and high-income countries, *S. flexneri* remains prevalent in LMICs, which makes it the most important species globally. The spread of resistance among *Shigella* isolates increasingly compromises antibiotic treatment and emphasizes the need for alternatives among which disease prevention through vaccination is an attractive option. Altogether, epidemiological data call for a broad serotype coverage vaccine conferring primarily protective immunity to the pediatric population in LMICs. This is a challenging issue considering the diversity of circulating strains responsible for disease.

*Shigella* serotypes are differentiated by their surface polysaccharide and, in particular, by the O-antigen (O-Ag) component, or O-specific polysaccharide (O-SP) part, of their lipopolysaccharide (LPS). The O-Ag consists of oligomers and polymers of oligosaccharide repeating units. Naturally acquitted protective immunity against shigellosis is thought to be largely serotype-specific.

Despite a diversity of vaccine candidates being designed to prevent infection by *Shigella* and evaluated for safety and immunogenicity in clinical trials, many of which are currently ongoing, there is yet no licensed *Shigella* vaccine.

A major challenge resides in the need for a vaccine that is well tolerated, immunogenic and efficacious against moderate-to-severe diarrhea caused by circulating *Shigella* strains representative of a large diversity of serotypes, in particular among infants and children under 5 years of age in LMICs.

Accordingly, it is an object of the present invention to provide versatile core precursors, namely, specifically protected tetrasaccharide scaffolds, able to provide access to a large diversity of *Shigella flexneri* O-antigen segments in a highly efficient divergent manner. Indeed, key repeating units (RU) building blocks featuring serotype-specific substitutions and functionalized to enable subsequent chain extension were built from these single orthogonally protected scaffolds.

Another aim of the present invention is to provide a way to a large variety of selected targets in the context of vaccine development against shigellosis. In particular, the present invention relates to a chemically synthesized specifically protected tetrasaccharide designed as a common scaffold precursor to conjugates comprising oligosaccharides representing segments of the O-antigens from diverse *S. flexneri* serotypes, comprising for example SF2a, SF3a, SFlb and/or SFY.

Thus, in one aspect, the present invention relates to a tetrasaccharide compound of following formula (O) : 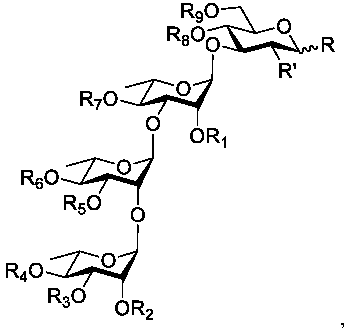 in particular 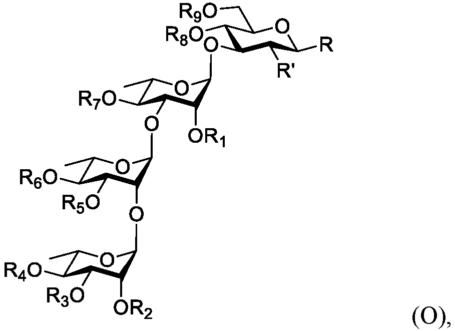 wherein :
R is O-allyl (All), O-triisopropylsilyl (TIPS), O-*tert*-butyldiphenylsilyl (TBDPS), *O-tert*butyldimethylsilyl (TBS or TBDMS), O-thexyl dimethyl silyl (TDS), *para*-methoxyphenyl (PMP) or SR₀, with R₀ being as the compound is a thioglycoside;
R' is NHC(O)CCl₃, NHC(O)CHCl₂, NAc₂, NHTroc with Troc being 2,2,2-trichloroethoxycarbonyl, a carbamate such as NHCbz, with Cbz being carboxybenzyl, NAlloc, with Alloc being allyloxycarbonyl, NHC(O)CF₃, tetrachlorophtalimido, phtalimido, or azido; R₁ is chloroacetyl (CA), bromoacetyl (BA), or acetyl (Ac), and R₂ is levulinoyl (Lev), fluorenylmethoxycarbonyl (Fmoc), or pentafluorophenyl ester (PFP);
or R₂ is chloroacetyl (CA), bromoacetyl (BA), or acetyl (Ac), and R₁ is levulinoyl (Lev), fluorenylmethoxycarbonyl (Fmoc), or pentafluorophenyl ester (PFP);
R₄, R₅ and R₆ are independently chosen from arylmethyl protecting groups, in particular benzyl (Bn), *para*-chlorobenzyl (PCB), *para*-bromobenzyl (PBB), *para*-nitrobenzyl or *ortho-*nitrobenzyl;
R₃, R₇ and R₈/R₉ are protecting groups that are orthogonal to each other and orthogonal to R, R₁, R₂, R₄, R₅, R₆ and R'.

Alternatively, R₂ is a protecting group that is orthogonal to R, R₁, R₂, R₃, R₄, R₅, R₆, R₇, R₈/R₉ and R', and capable of assistance, in particular anchimeric assistance and/or by steric hindrance.

In particular, R' is NHC(O)CCl₃, NHC(O)CHCl₂, NAc₂, NHTroc, tetrachlorophtalimido, or azido, more particularly NHC(O)CCl₃.

In particular, R is O-allyl (All), and/or R' is NHC(O)CCl₃.

In another aspect, the present invention relates to a tetrasaccharide compound of following formula (O') : 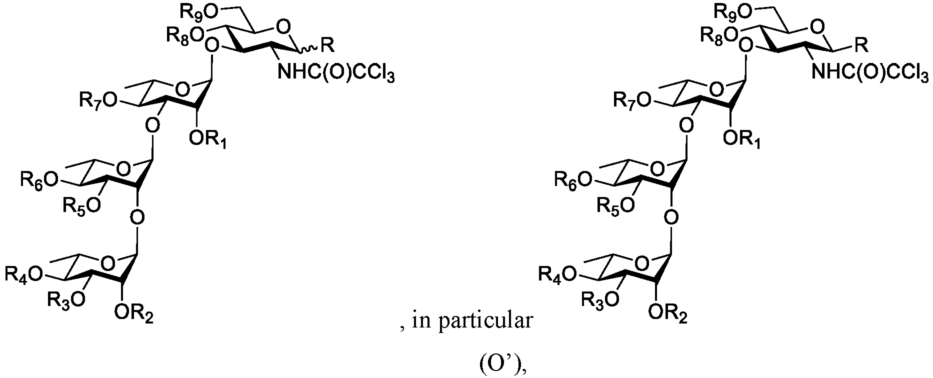 wherein :
R is O-allyl (All), O-triisopropylsilyl (TIPS), O-tert-butyldiphenylsilyl (TBDPS), *O-tert*butyldimethylsilyl (TBS or TBDMS), O-thexyl dimethyl silyl (TDS), para-methoxyphenyl (PMP) or SR₀, with R₀ being as the compound is a thioglycoside;
R₁ is chloroacetyl (CA), bromoacetyl (BA), or acetyl (Ac), and R₂ is levulinoyl (Lev), fluorenylmethoxycarbonyl (Fmoc), or pentafluorophenyl ester (PFP);
or R₂ is chloroacetyl (CA), bromoacetyl (BA), or acetyl (Ac), and R₁ is levulinoyl (Lev), fluorenylmethoxycarbonyl (Fmoc), or pentafluorophenyl ester (PFP);
R₄, Rs and R₆ are independently chosen from arylmethyl protecting groups, in particular benzyl (Bn), *para*-chlorobenzyl (PCB), *para*-bromobenzyl (PBB), *para*-nitrobenzyl or *ortho-*nitrobenzyl;
R₃, R₇ and R₈/R₉ are protecting groups that are orthogonal to each other and orthogonal to R, R₁, R₂, R₄, R₅, R₆ and NHC(O)CCl₃.

Alternatively, R₃, R₇ and R₈ are protecting groups that are orthogonal to each other and orthogonal to R, R₁, R₂, R₄, R₅, R₆ and NHC(O)CCl₃, and R₉ is Bn.

Orthogonality and orthogonal protecting groups in carbohydrate chemistry are well known from the skilled in the art, and are in particular described in Agoston et al. (Tetrahedron: Asymmetry 27 (2016) 707-728).

Thioglycosides are well known from the skilled in the art. Reference is made for example to Advances in Carbohydrate Chemistry and Biochemistry, Volume 52, 1997, Pages 179-205.

In a particular embodiment, R₀ is chosen from:
- C₁-C₁₂-alkyl, in particular Me or Et;
- C₁-C₁₂-alkyl-Ar, wherein Ar is an aryl, optionally substituted, notably by one or more groups chosen from C₁-C₆ alkyl, O-C₁-C₆ alkyl, NO₂, in particular (CH₂)₃-Ph, CH₂-(*tert*-butyl-Ph) (MBP),
- C₁-C₁₂-alkyl-Het, wherein Het is a heteroaryl, optionally substituted, notably by one or more groups chosen from C₁-C₆ alkyl, O-C₁-C₆ alkyl, NO₂,
- C₁-C₁₂-alkenyl, in particular Me or Et;
- C₁-C₁₂-alkenyl-Ar, wherein Ar is an aryl, optionally substituted, notably by one or more groups chosen from C₁-C₆ alkyl, O-C₁-C₆ alkyl, NO₂, in particular 4-(*p-*Methoxyphenyl)-4-pentenyl (MPTG),
- C₁-C₁₂-alkenyl-Het, wherein Het is a heteroaryl, optionally substituted, notably by one or more groups chosen from C₁-C₆ alkyl, O-C₁-C₆ alkyl, NO₂,
- aryl, optionally substituted, notably by one or more groups chosen from C₁-C₆ alkyl, O-C₁-C₆ alkyl, NO₂, in particular phenyl, tolyl, -Ph-NO₂, 2-*tert*-butyl-5-methylphenyl;
- heteroaryl, optionally substituted, notably by one or more groups chosen from C₁-C₆ alkyl, O-C₁-C₆ alkyl, NO₂, in particular pyridyl, indolyl, benzoxazolyl (Box); and
- glycosyl;
- or is such as SR₀ is an alkoxythioimidate;

R₀ being more particularly phenyl, tolyl, ethyl or 2-*tert*-butyl-5-methylphenyl.

In a particular embodiment, the invention also concerns a tetrasaccharide compound of following formula (I₀) : 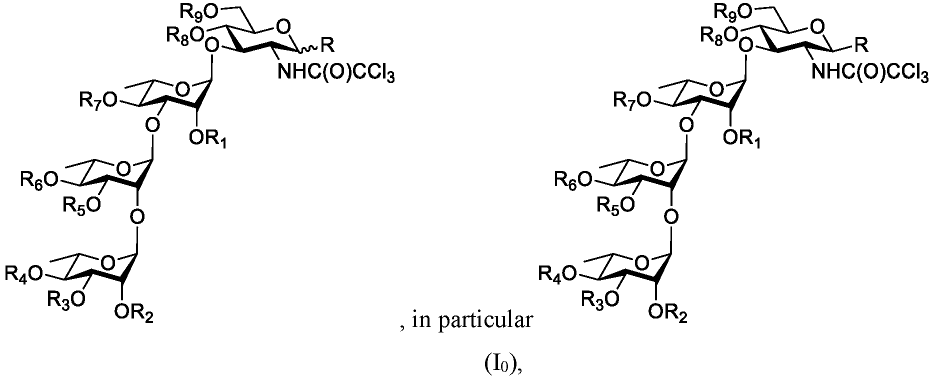 wherein :
R is O-allyl (All), O-triisopropylsilyl (TIPS), O-*tert*-butyldiphenylsilyl (TBDPS), *O-tert*butyldimethylsilyl (TBS or TBDMS), O-thexyl dimethyl silyl (TDS), para-methoxyphenyl (PMP) or SR₀, with R₀ being as the compound is a thioglycoside;
R₁ is chloroacetyl (CA), bromoacetyl (BA), or acetyl (Ac), and R₂ is levulinoyl (Lev), fluorenylmethoxycarbonyl (Fmoc), or pentafluorophenyl ester (PFP);
or R₂ is chloroacetyl (CA), bromoacetyl (BA), or acetyl (Ac), and R₁ is levulinoyl (Lev), fluorenylmethoxycarbonyl (Fmoc), or pentafluorophenyl ester (PFP);
R₃ is *tert*-butyldimethyl silyl (TBS), triethylsilyl (TES), O-triisopropylsilyl (TIPS), 2-methylnaphthyl (Nap), *para*-methoxybenzyl (PMB), *para*-bromobenzyl (PBB), *para-*chlorobenzyl (PCB),*para*-nitrobenzyl, *ortho*-nitrobenzyl, 2-pyridylmethyl (picolinyl), picoloyl ester (pico), or allyl (All);
R₄, R₅ and R₆ are independently chosen from arylmethyl protecting groups, in particular benzyl (Bn), *para*-chlorobenzyl (PCB), *para*-bromobenzyl (PBB), *para*-nitrobenzyl or *ortho-*nitrobenzyl;
R₇ is 2-methylnaphthyl (Nap), *para*-methoxybenzyl (PMB), *para*-bromobenzyl (PBB), *para-*chlorobenzyl (PCB), *tert*-butyldimethyl silyl (TBS), triethylsilyl (TES), O-triisopropylsilyl (TIPS), *para*-nitrobenzyl, *ortho*-nitrobenzyl, 2-pyridylmethyl (picolinyl), picoloyl ester (pico), allyloxycarbonyl (Alloc) or allyl (All);
none or only one of R, OR₃ and OR₇ being OAll;
R₇ being different from PBB and PCB when at least one of R₄, R₅ and R₆ is PBB or PCB; with R₃ being TBS or TES, and R being different from TIPS, TBDPS, TBS and TDS, when R₇ is Nap, PMB or PBB,
with R₃ being Nap, PMB or PBB, and R being different from TIPS, TBDPS, TBS and TDS, when R₇ is TBS or TES;
with R₃ being Nap and R₇ being PMB or PBB, or R₇ being Nap and R₃ being PMB or PBB, when R is TIPS, TBDPS, TBS or TDS,
with R₃ being TBS, TES, TIPS, PMB, PCB, PBB, *para*-nitrobenzyl, *ortho*-nitrobenzyl, picolinyl, or pico, when R₇ is Nap, and R₃ being Nap, PMB, PCB or PBB, *para*-nitrobenzyl, *ortho*-nitrobenzyl, picolinyl, or pico, when R₇ is TBS, TES, or TIPS;
and with R₇ being TBS, TES, TIPS, PMB, PCB, PBB, *para*-nitrobenzyl, *ortho*-nitrobenzyl, picolinyl, or pico, when R₃ is Nap, and R₇ being Nap, PMB, PCB or PBB, *para*-nitrobenzyl, *ortho*-nitrobenzyl, picolinyl, or pico, when R₃ is TBS, TES, or TIPS;
R₈ and R₉ form together a benzylidene acetal (Bzl), cyclohexylidene acetal or isopropylidene acetal, or, when R₃ and R₇ are not Nap, *para*-nitrobenzylidene acetal or naphthylidene acetal.

In a particular embodiment, R₁ is chloroacetyl (CA), bromoacetyl (BA), or acetyl (Ac), and R₂ is levulinoyl (Lev), fluorenylmethoxycarbonyl (Fmoc), or pentafluorophenyl ester (PFP).

In a particular embodiment, R₂ is levulinoyl (Lev).

In a particular embodiment, R₁ is chloroacetyl (CA), bromoacetyl (BA), or acetyl (Ac), and R₂ is levulinoyl (Lev).

In a particular embodiment, R₄, R₅ and R₆ are Bn.

In a particular embodiment, R₄, R₅ and R₆ are *para*-chlorobenzyl (PCB), *para-*bromobenzyl (PBB), *para*-nitrobenzyl or *ortho*-nitrobenzyl, and R₃ and R₇ are not PCB, PBB, *para*-nitrobenzyl, or *ortho*-nitrobenzyl.

In a particular embodiment, R₄, R₅ and R₆ are identical.

In a particular embodiment, the invention also concerns a tetrasaccharide compound of following formula (I) : 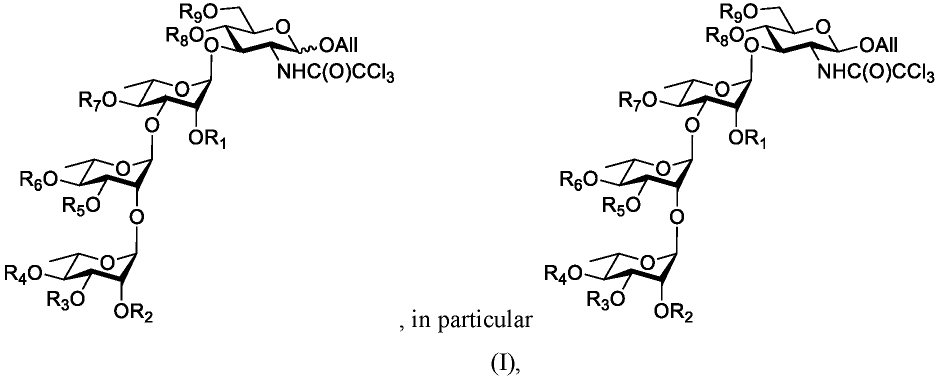 wherein :
All is allyl;
R₁ is chloroacetyl (CA), bromoacetyl (BA), or acetyl (Ac);
R₂ is levulinoyl (Lev) or fluorenylmethoxycarbonyl (Fmoc) or pentafluorophenyl ester (PFP), in particular Lev or Fmoc, even more particularly Lev;
R₃ is *tert*-butyldimethyl silyl (TBS), triethylsilyl (TES), 2-methylnaphthyl (Nap), *para-*methoxyphenyl (PMB) or *para*-bromobenzyl (PBB);
R₄, R₅ and R₆ are independently chosen from arylmethyl protecting groups, in particular benzyl (Bn), *para*-chlorobenzyl (PCB), *para*-bromobenzyl (PBB), *para*-nitrobenzyl or *ortho-*nitrobenzyl;
R₇ is 2-methylnaphthyl (Nap), *para*-methoxyphenyl (PMB), *para*-bromobenzyl (PBB), *tert-*butyldimethyl silyl (TBS) or triethylsilyl (TES);
R₇ being different from PBB and PCB when at least one of R₄, R₅ and R₆ is PBB or PCB; with R₃ being TBS or TES, and R being different from TIPS, TBDPS, TBS and TDS, when R₇ is Nap, PMB or PBB,
with R₃ being Nap, PMB or PBB when R₇ is TBS or TES, and R being different from TIPS, TBDPS, TBS and TDS;
with R₃ being Nap and R₇ being PMB or PBB, or R₇ being Nap and R₃ being PMB or PBB, when R is TIPS, TBDPS, TBS or TDS,
with R₃ being TBS, TES, TIPS, PMB, PCB, PBB, *para*-nitrobenzyl, *ortho*-nitrobenzyl, picolinyl, or pico, when R₇ is Nap, and R₃ being Nap, PMB, PCB or PBB, *para*-nitrobenzyl, *ortho*-nitrobenzyl, picolinyl, or pico, when R₇ is TBS, TES, or TIPS;
and with R₇ being TBS, TES, TIPS, PMB, PCB, PBB, *para*-nitrobenzyl, *ortho*-nitrobenzyl, picolinyl, or pico, when R₃ is Nap, and R₇ being Nap, PMB, PCB or PBB, *para*-nitrobenzyl, *ortho*-nitrobenzyl, picolinyl, or pico, when R₃ is TBS, TES, or TIPS;
R₈ and R₉ form together a benzylidene acetal (Bzl), cyclohexylidene acetal or isopropylidene acetal, or, when R₃ and R₇ are not Nap, *para*-nitrobenzylidene acetal or naphthylidene acetal.

In a particular embodiment, the invention also concerns a compound as defined above, of following formula (I₁) : 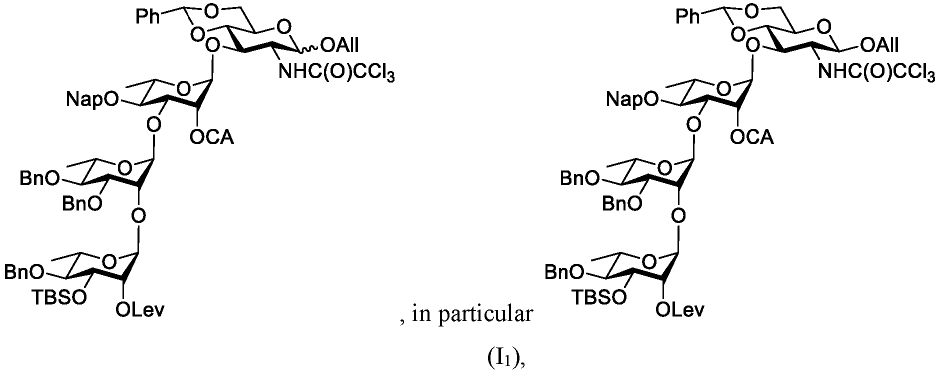 wherein :
All is allyl;
CA is chloroacetyl;
Lev is levulinoyl;
TBS is *tert*-butyldimethyl silyl;
Bn is benzyl;
Nap is 2-methylnaphthyl.

In another aspect, the invention also concerns a process of preparation of a compound as defined above, comprising a step of contacting a donor of formula (I_{A}) with an acceptor of formula (I_{B}) to yield said compound of formula (I),
said formula (I_{A}) being as follows: 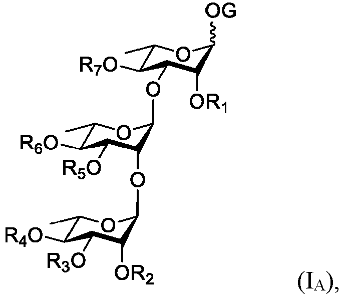 wherein:
G is an activating group, in particular A-phenyltrifluoroacetimidyl (PTFA) or trichloroacetimidyl (TCA);
R₁-R₇ being as defined above;
said formula (I_{B}) being as follows: 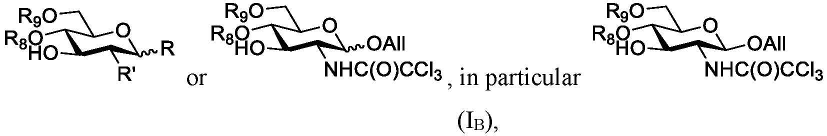
R₈-R₉ being as defined above.

G is in particular chosen from PTFA, TCA, imidates, thioimidates, for example S-benzimidazolyl (SBiz) imidate, S-thiazolinyl (STaz) imidate, O-benzoxazolyl (OBox) imidates, 3,3-difluoro-3H-indol-2-ylthio (SFox) imidates, thioglycosides, sulfoxides, alkoxythioimidates, phosphates, halides, alkynyl benzoates.

G is for example described by:
- B. Yu and J. Sun (Chem. Commun., 2010, 46, 4668-4679),
- Schmidt and al. (Adv. Carbohydr. Chem. Biochem. 1994, 50, 21 ; *or* Angew. Chem., Int. Ed. Engl. 1986, 25, 212),
- Org. Biomol. Chem., 2024, 22, 5214-5223,
- Carbohydrate Research, 2015, 403, 115-122,
- Acc. Chem. Res. 2018, 51, 2, 507-516.

G may also represent H, the corresponding compound being in this case a hemiacetal. This hemiacetal can be activated into a donor, for example an imidate donor (anomeric OPTFA or OTCA substitution) (J. Org. Chem. 2015, 80, 11237-57), or into an alkynyl benzoate donor (Acc. Chem. Res. 2018, 51, 507-516), or into a diphenyl oxosulfonium intermediate (J. Am. Chem. Soc. 2000, 122, 4269-4279).

In another aspect, the invention also concerns a penta- or hexasaccharide compound of following formula (II) : 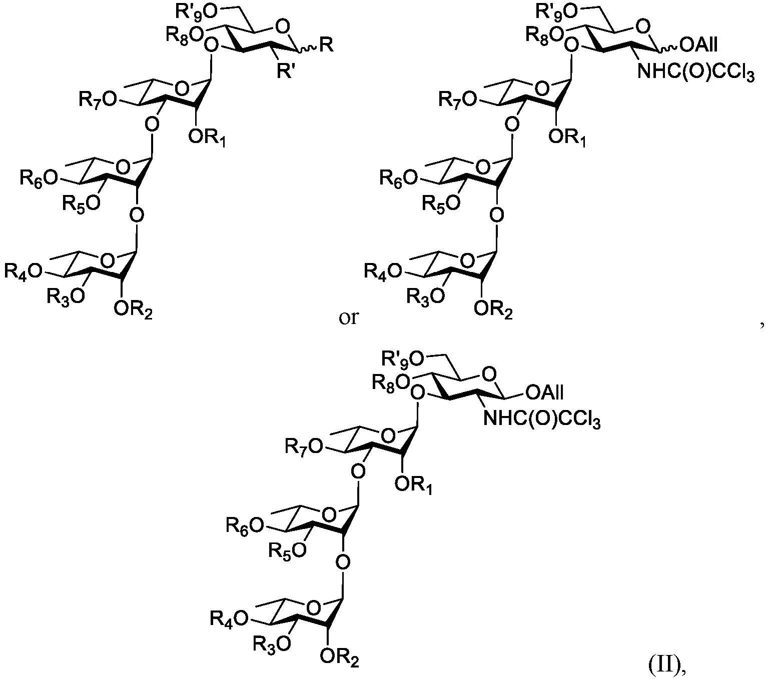 in particular wherein :
one or two of R₈, R₇ and R₃ represent(s) a protected residue α-D-Glc*p*-;
the other R₁-R₇ groups are as defined above;
when R₈ represents a protected residue α-D-Glc*p*-, R'₉ is a protecting group that is orthogonal R₂, R₃ and R₇;
and when R₈ does not represent a protected residue α-D-Glc*p*-, R₈ and R'₉ form together a benzylidene acetal (Bzl) or isopropylidene acetal.

In particular, one of R₈, R₇ and R₃ represents a protected residue α-D-Glc*p*-. The compound of formula (II) is in this case a pentasaccharide.

In particular, when R₈ represents a protected residue α-D-Glc*p*-, R'₉ is a primary hydroxyl protecting group, in particular a protecting group which is selective for primary hydroxyl group.

In particular, when R₈ represents a protected residue α-D-Glc*p*-, R'₉ is chosen from arylmethyl protecting groups, in particular Bn, PCB, PBB, PMB, *para*-nitrobenzyl or *ortho-*nitrobenzyl, Nap, hindered esters, Ac, and, when R₃ is not TBS or TES, TBDPS, TES, TBS and thexyl (TDS), notably TBDPS and TES.

Alternatively, one of R₇ and R₃ represents a protected residue α-D-Glc*p*-, R₈ represents an arylmethyl protecting group, in particular Bn, PCB, PBB, PMB, para-nitrobenzyl or *ortho-*nitrobenzyl, and R'₉ is chosen from Nap, hindered esters, and Ac.

Alternatively, one of R₇ and R₃ represents a protected residue α-D-Glc*p*-, R₈ represents Bn, and R'₉ is chosen from PCB, PBB, PMB, para-nitrobenzyl or ortho-nitrobenzyl, Nap, hindered esters, and Ac.

In another aspect, the invention also concerns a pentasaccharide compound of following formula (II) : 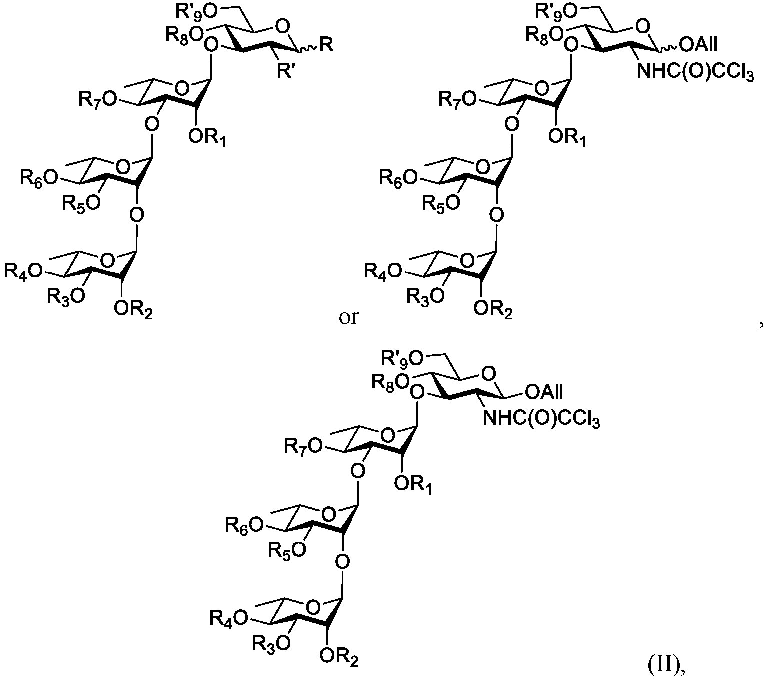 in particular wherein :
R₈, R₇ or R₃ represents a protected residue α-D-Glc*p*-;
the other R₁-R₇ groups are as defined above;
when R₈ represents a protected residue α-D-Glc*p*-, R'₉ is chosen from Bn,Nap, PMB, PBB, and, when R₃ is not TBS or TES, TBDPS, TES, TBS and thexyl (TDS), notably TBDPS and TES, and when R₈ does not represent a protected residue α-D-Glc*p*-, R₈ and R₉ form together a benzylidene acetal (Bzl), cyclohexylidene acetal or isopropylidene acetal, or, when R₃ and R₇ are not Nap, *para*-nitrobenzylidene acetal or naphthylidene acetal.

By "protected residue α-D-Glc*p*-" is in particular meant a residue of the following formula: 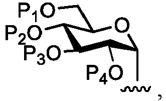 wherein P₁, P₂, P₃ and P₄ are independently chosen from arylmethyl protecting groups, in particular Bn, PCB, PBB, PMB, *para*-nitrobenzyl or *ortho*-nitrobenzyl, and Nap.

In particular, P₁, P₂, P₃ and P₄ are identical.

In a particular embodiment, the invention also concerns a pentasaccharide compound as defined above, of following formula : 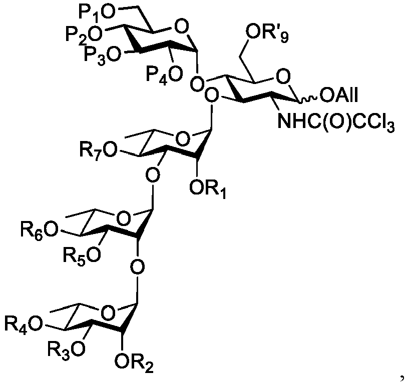 in particular 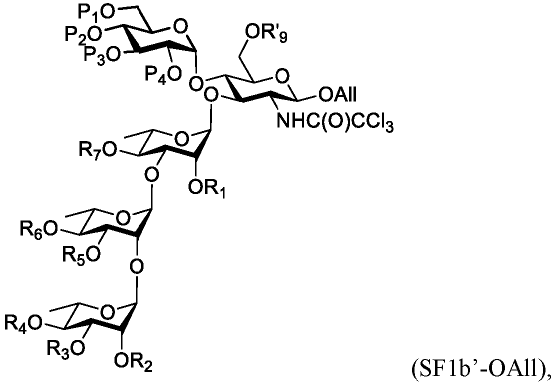 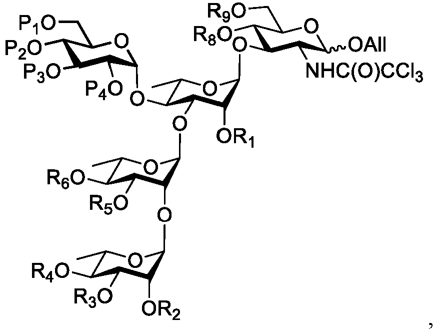 in particular 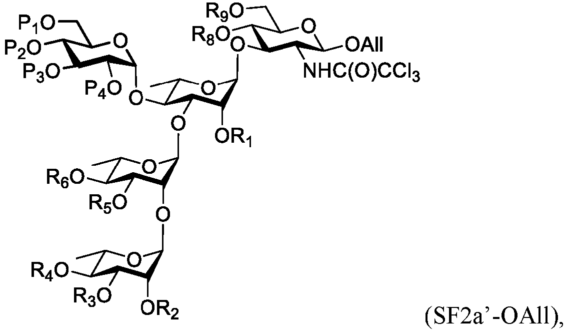 or 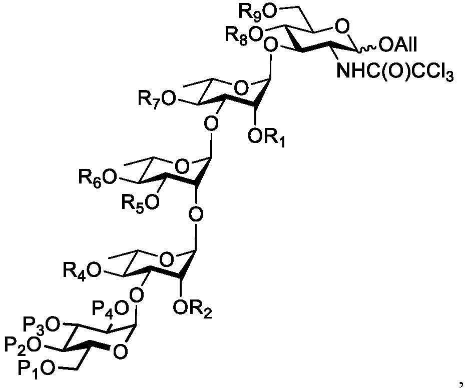 in particular 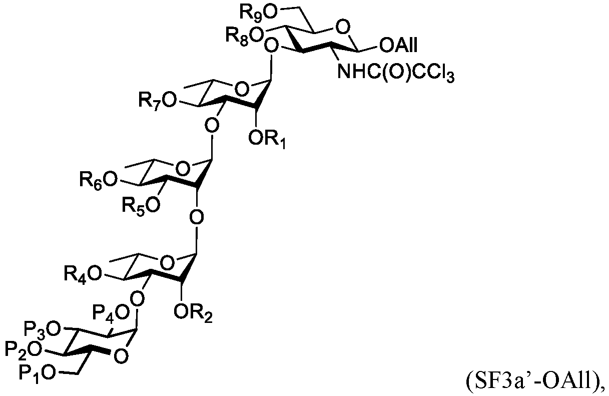 wherein :
P₁, P₂, P₃ and P₄ are independently chosen from arylmethyl protecting groups, in particular Bn, PCB, PBB, PMB, *para*-nitrobenzyl or ort*h*o-nitrobenzyl, and Nap,
R₁-R₉ and R'₉ being as defined above,
and, for SF1b', wherein R₃ may also represent Bn.

In another aspect, the invention also concerns a process of preparation of a pentasaccharide SF1b'-OAll, wherein:
SFIb is ABC(E)D, with A is 2)-α-L-Rha*p*-(1→ , B is 2)-α-L-Rha*p*-(1→ , C is 3)-α-L-Rha*p*-(1→ , D is 3)-α-D-Glc*p*NAc-(1→ or 3)-β-D-Glc*p*NAc-(1→ , E represents a residue α-D-Glc*p*-;
SFlb being in particular *O*-acetylated in position 2c; and ' denotes that the pentasaccharide is protected as defined below,
said process comprising the following steps:
   (i) A step of deprotection of the R₈ group of a compound of formula (I), in particular by deprotecting both R₈ and R₉ using for example SnCl₄, camphorsulfonic acid (CSA) or trifluoroacetic acid (TFA), and then by protecting the obtained compound with a R'₉ group using for example R'₉-Br and Taylor reagent, R'₉-Br with Bu₂SnO activation, R'₉-TCA or R'₉-PTFA, R'₉ being as defined above, to obtain an acceptor compound of following formula: 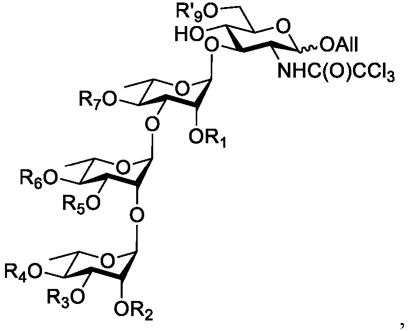 in particular 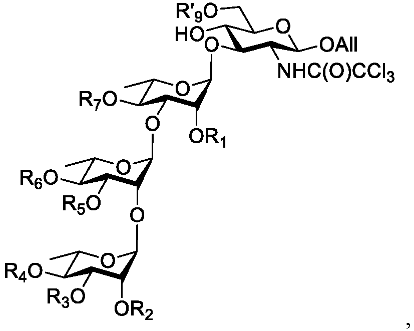
      wherein R₁-R₇ and R'₉ are as defined above;
      and wherein R₃ may also represent Bn;
   (ii) A step of reacting the acceptor obtain in step (i) with the donor compound E-G of following formula: 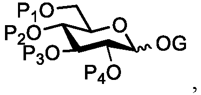 wherein :
      P₁, P₂, P₃ and P₄ are as defined above,
      G is an activating group, in particular *N*-phenyltrifluoroacetimidyl (PTFA), or trichloroacetimidyl (TCA),
      in particular in presence of an additive, for example dimethylformamide (DMF),
      to yield the pentasaccharide SF1b'-OAll of following formula: 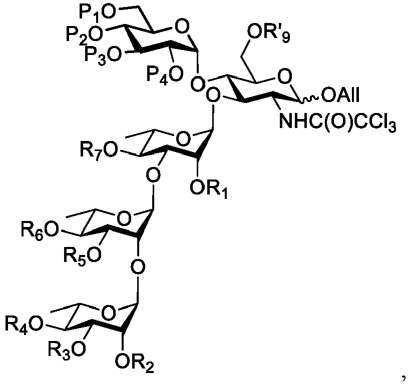 in particular 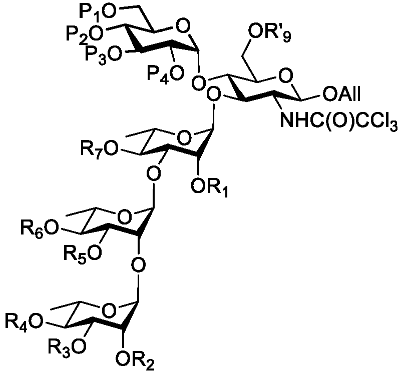
      wherein R₁-R₇ and R'₉, P₁, P₂, P₃ and P₄ are as defined above,
      and wherein R₃ may also represent Bn.

Such additives are for examples described in Angew. Chem. Int. Ed. 2011, 50, 7315 - 7320.

In another aspect, the invention also concerns a process of preparation of a pentasaccharide SF2a'-OAll, wherein:
SF2a is AB(E)CD, with A is 2)-α-L-Rha*p*-(1→ , B is 2)-α-L-Rha*p*-(1→ , C is 3)-α-L-Rha*p*-(1→ , D is 3)-α -D-Glc*p*NAc-(1→ or 3)-β-D-Glc*p*NAc-(1→ , E represents a residue α-D-Glc*p*- ; and ' denotes that the pentasaccharide is protected as defined below,
said process comprising the following steps:
   (i) A step of deprotection of the R₇ group of a compound of formula (I), for example using 2,3-dichloro-5,6-dicyano-p-benzoquinone (DDQ) and optionally β-pinene when R₇ is Nap, to obtain an acceptor compound of following formula: 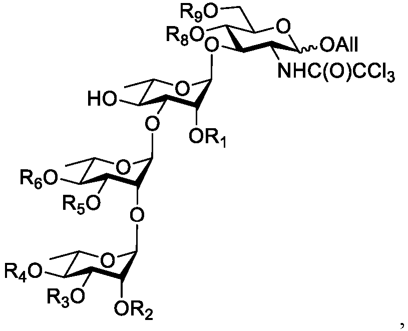 in particular 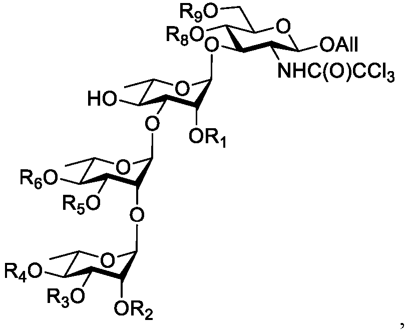 wherein R₁-R₆ and R₈-R₉ are as defined above ;
   (ii) A step of reacting the acceptor obtain in step (i) with the donor compound E-G of following formula: 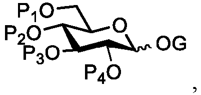 wherein :
      P₁, P₂, P₃ and P₄ are as defined above,
      G is an activating group, in particular N-phenyltrifluoroacetimidyl (PTFA), or trichloroacetimidyl (TCA),
      in particular in presence of an additive, for example dimethylformamide (DMF),
      to yield the pentasaccharide SF2a'-OAll of following formula: 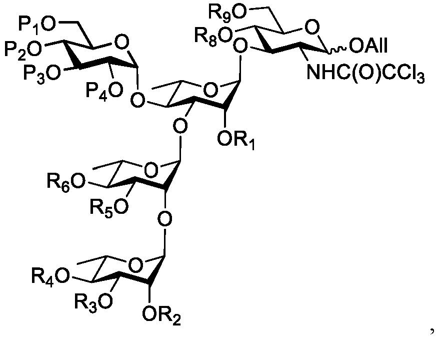 in particular 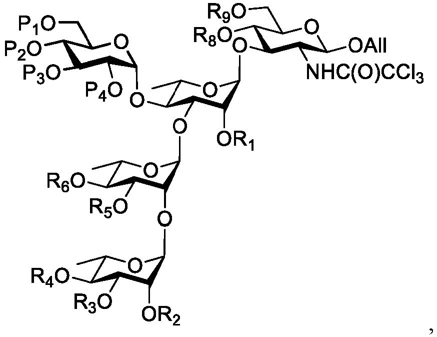 wherein R₁-R₆, R₈-R₉, P₁, P₂, P₃ and P₄ are as defined above.

In another aspect, the invention also concerns a process of preparation of a pentasaccharide SF3a'-OAll, wherein:
SF3a is (E)ABCD, with A is 2)-α-L-Rha*p*-(1→ , B is 2)-α-L-Rha*p*-(1→ , C is 3)-α-L-Rhap-(1→ , D is 3)-α -D-Glc*p*NAc-(1→ or 3)-β-D-Glc*p*NAc-(1→ , E represents a residue α-D-Glc*p*- ;
SF3a being in particular *O*-acetylated in position 2c; and
' denotes that the pentasaccharide is protected as defined below,
said process comprising the following steps:
   (i) A step of deprotection of the R₃ group of a compound of formula (I), for example using triethylamine trihydrofluoride (TEA.3HF) when R₃ is TBS, to obtain an acceptor compound of following formula: 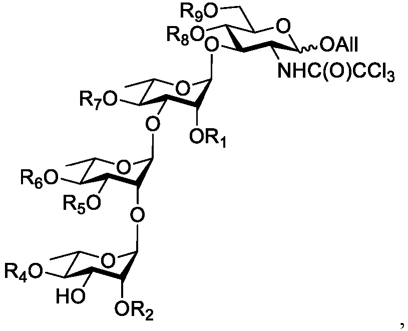 in particular 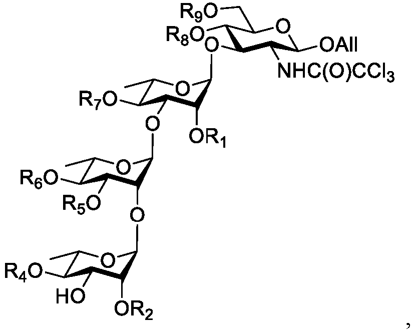 wherein R₁-R₂, and R₄-R₉ are as defined above;
   (ii) A step of reacting the acceptor obtain in step (i) with the donor compound E-G of following formula: 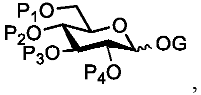 wherein :
      P₁, P₂, P₃ and P₄ are as defined above,
      G is an activating group, in particular *N*-phenyltrifluoroacetimidyl (PTFA), or trichloroacetimidyl (TCA),
      in particular in presence of an additive, for example dimethylformamide (DMF),
      to yield the pentasaccharide SF3a'-OAll of following formula: 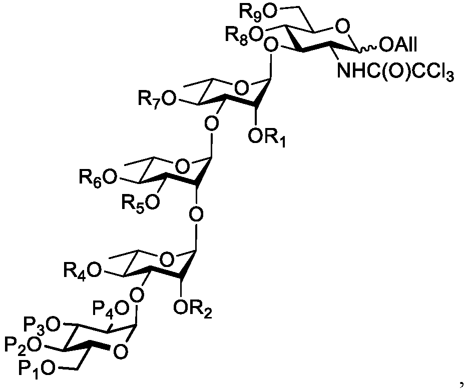 in particular 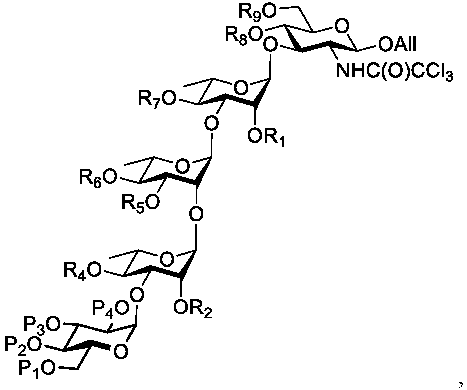 wherein R₁-R₂, R₄-R₉, P₁, P₂, P₃ and P₄ are as defined above.

In another aspect, the invention also concerns a pentasaccharide acceptor compound of following formula (III_{A}) : 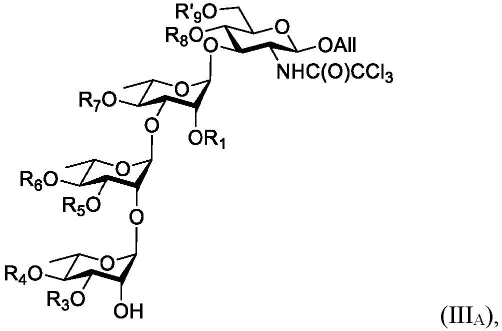 wherein :
R₈, R₇ or R₃ represents a protected residue α-D-Glcp- ;
the other R₁, R₃-R₈ and R'₉ being as defined above.

In a particular embodiment, the invention also concerns a pentasaccharide acceptor compound as defined above, of following formula : 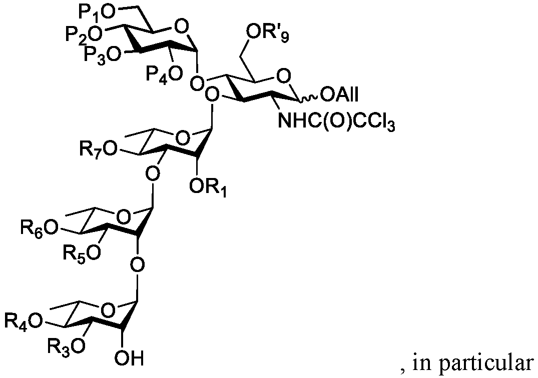 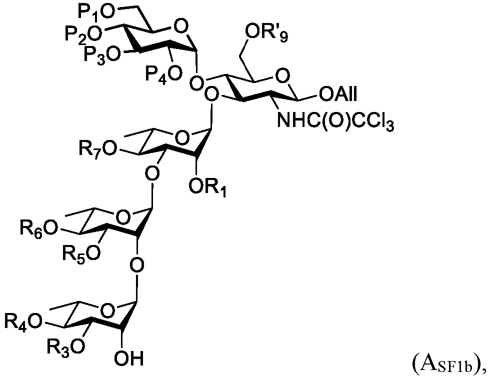 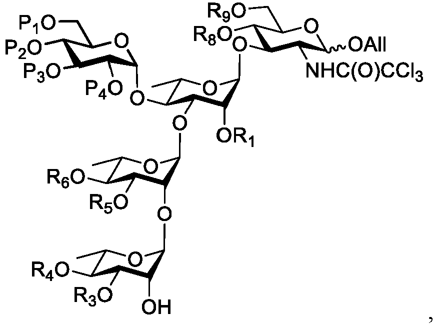 in particular 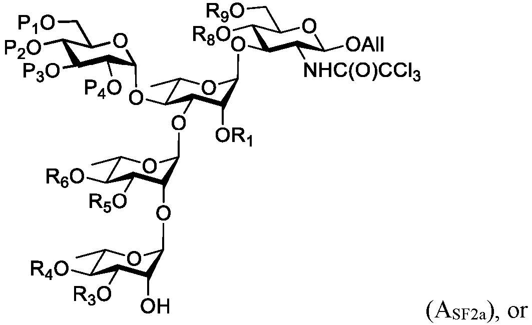 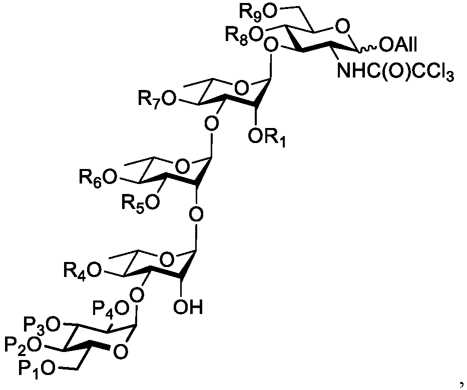 in particular 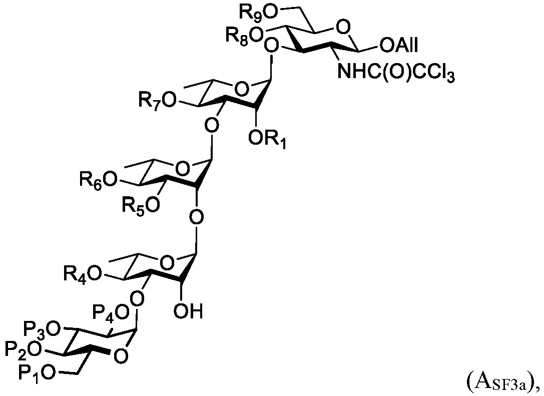 wherein R₁, R₂-R₉ and R'₉, P₁, P₂, P₃ and P₄ are as defined above,
and, for SF1b', wherein R₃ may also represent Bn.

In another aspect, the invention also concerns a process of preparation of a pentasaccharide acceptor compound of formula (III_{A}) as defined above, comprising a step of deprotection of group R₂ of a compound of formula (II), in particular using hydrazine acetate or hydrazine in a mixture pyridine/acetic acid, in particular in a 1:1 (v/v) pyridine/acetic acid mixture when R₂ is Lev.

In another aspect, the invention also concerns a pentasaccharide compound of following formula (III_{DHF}) : 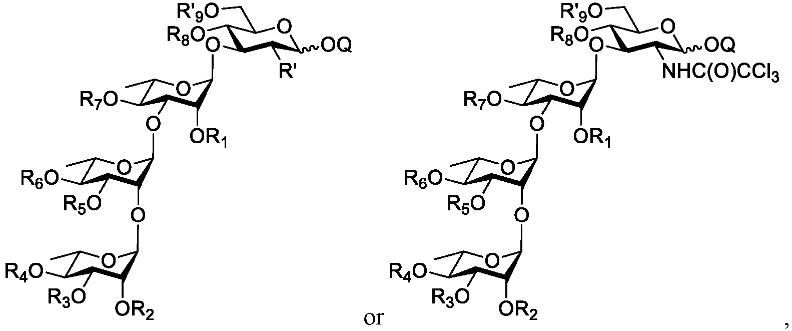 in particular 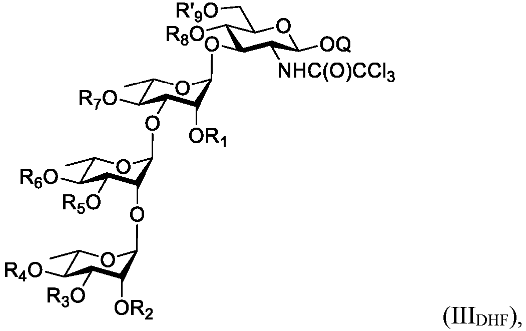 wherein :
Q is H, G or LZ,
G is an activating group, in particular N-phenyltrifluoroacetimidyl (PTFA), or trichloroacetimidyl (TCA),
L, which is optionally protected is:
   - a single bond,
   - a divalent C₁-C₁₂ alkyl chain optionally interrupted by one or more heteroatoms, notably selected from an oxygen atom, a sulphur atom or a nitrogen atom, said nitrogen and sulphur atoms being optionally oxidized, and the nitrogen atom being optionally involved in an acetamide bond, or
   - a divalent C₁-C₁₂ alkyl chain substituted by at least one -OH group, being in particular of the following formula -(CH₂-CH₂-C(OH))_{q}-(CH₂-CH₂)ᵢ, wherein i is 0 or 1 and q ranges from 1 to 10,
Z is a terminal function or group, optionally protected, able to form a covalent bond with a compound enabling to extend said LZ chain, a carrier and/or a solid support, or a multivalent scaffold; an anchor; a mono-, oligo- or polysaccharide; or a dye or fluorescent residue.

The protection of Z, if any, may be adapted to the R', and R₁-R'₉ groups as defined above.

In particular, Z is N₃, NHCbz, or NBnCbz group, in particular when R' is NHC(O)CCl₃, or a protected ester, for example COOBn.

In particular, LZ is -(CH₂)ₚ-N₃, -(CH₂)ₚ-NHCbz, or -(CH₂)ₚ-NBnCbz or or -(CH₂)ₚ-COOBn, wherein p ranges from 1 to 10, in particular from 2 to 8, more particularly 2, 3, 4, 5 or 6.
R₈, R₇ or R₃ represents a protected residue α-D-Glc*p*- ;
the other R₁-R₈ groups are as defined above;
R'₉ is as defined above.

In a particular embodiment, the invention also concerns a pentasaccharide compound as defined above, of following formula : 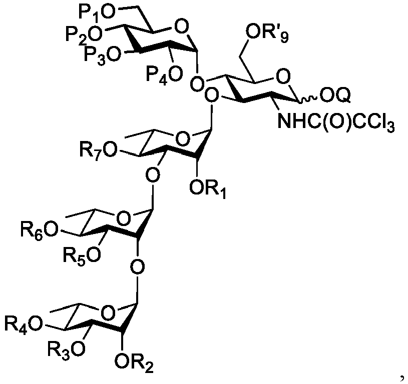 in particular 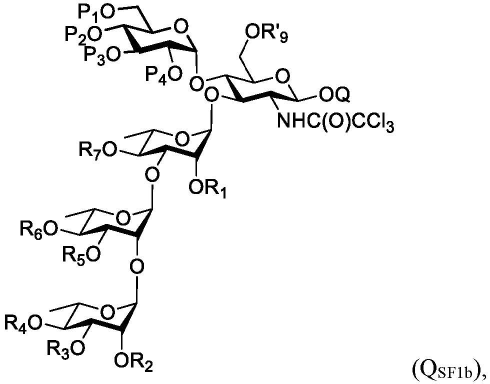 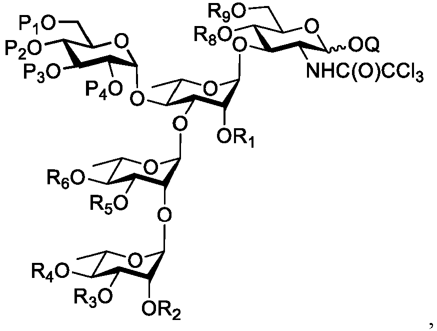 in particular 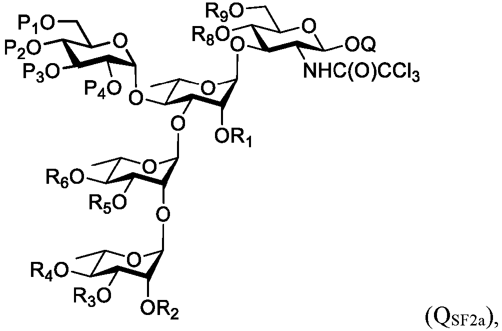 or 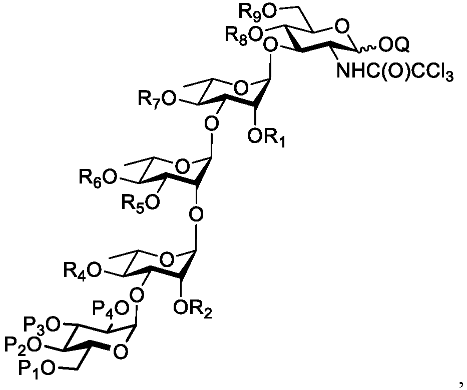 in particular 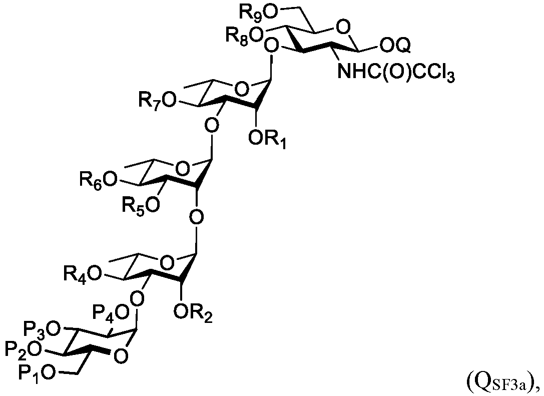 wherein R₁-R₉ and R'₉, P₁, P₂, P₃, P₄ and Q are as defined above,
and, for SF1b', wherein R₃ may also represent Bn.

In another aspect, the invention also concerns a pentasaccharide donor compound of following formula (III_{D}) : 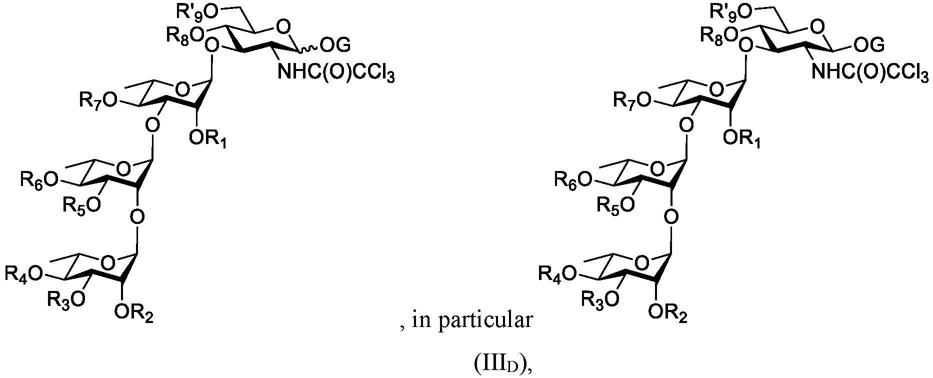 wherein :
G is an activating group, in particular *N*-phenyltrifluoroacetimidyl (PTFA), or trichloroacetimidyl (TCA),
R₈, R₇ or R₃ represents a protected residue α-D-Glc*p*- ;
the other R₁-R₈ and R'₉ groups are as defined above.

In a particular embodiment, the invention also concerns a pentasaccharide donor compound as defined above, of following formula : 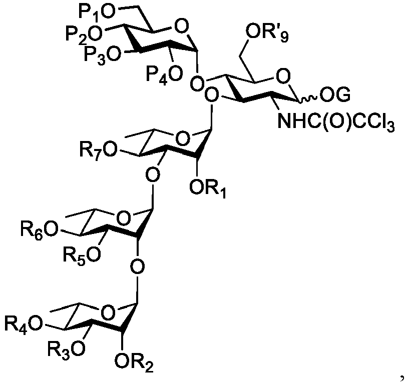 in particular 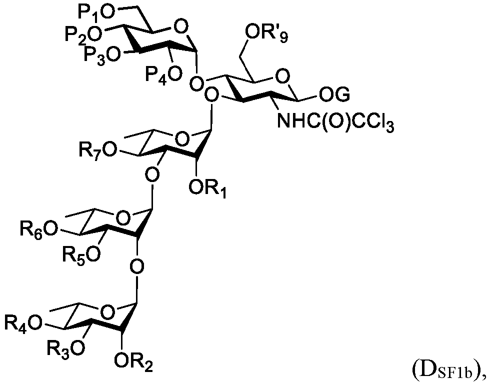 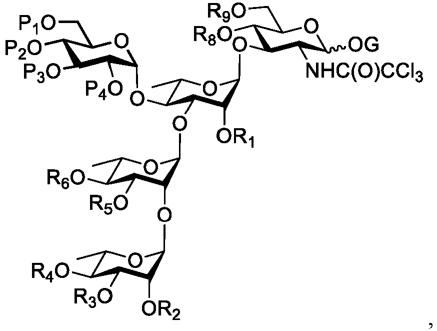 in particular 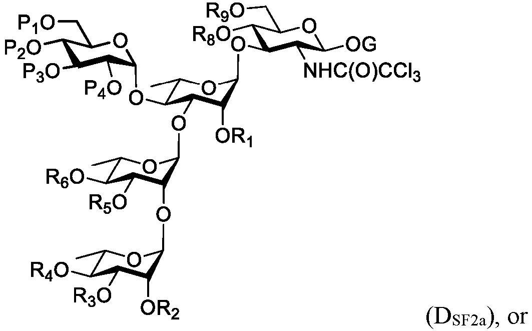 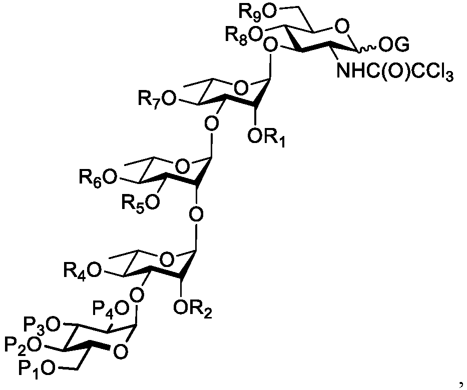 in particular 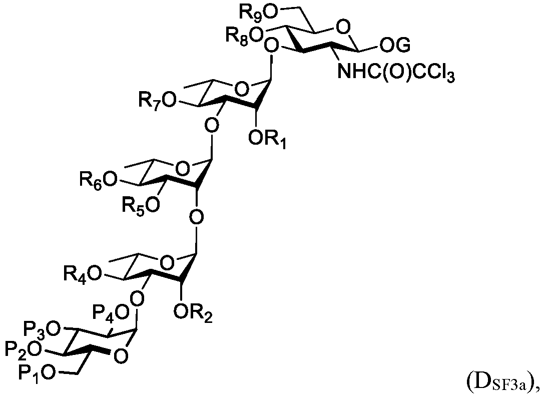 wherein R₁-R₉ and R'₉, P₁, P₂, P₃, P₄ and G are as defined above,
and, for SF1b', wherein R₃ may also represent Bn.

In another aspect, the invention also concerns a process of preparation of a pentasaccharide donor compound of formula (III_{D}) as defined above, comprising a first step of deprotection of the All group, in particular using PdCl₂ and then N-iodosuccinimide (NIS), or using iridium, to obtain a compound of formula (III_{H}), and a second step of contacting the obtained compound with a compound of type G-LG wherein G is as defined above, and LG is a leaving group, for example PTFA-Cl when G is PTFA.

In another aspect, the invention also concerns a pentasaccharide compound of following formula (III_{H}) : 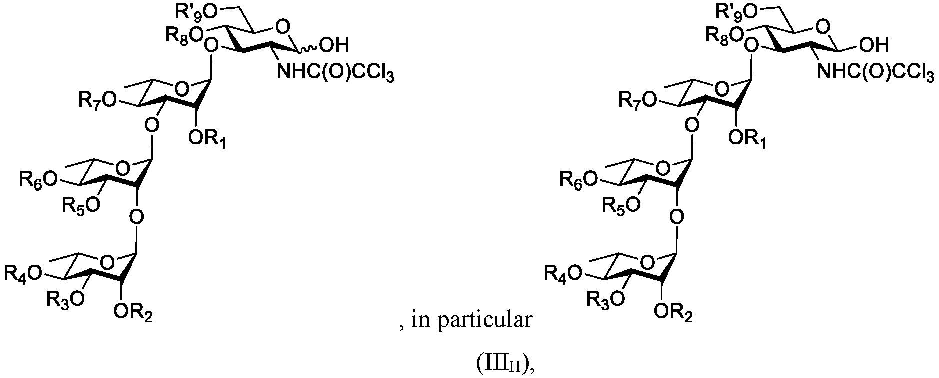 wherein :
R₈, R₇ or R₃ represents a protected residue α-D-Glc*p*- ;
the other R₁-R₈ and R'₉ groups are as defined above.

In a particular embodiment, the invention also concerns a pentasaccharide compound as defined above, of following formula : 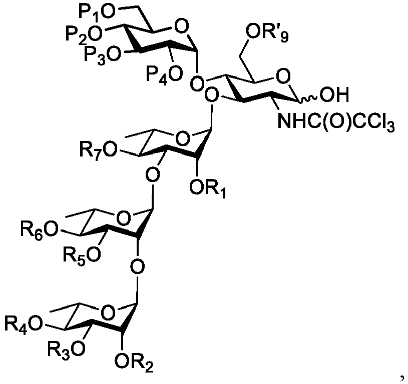 in particular 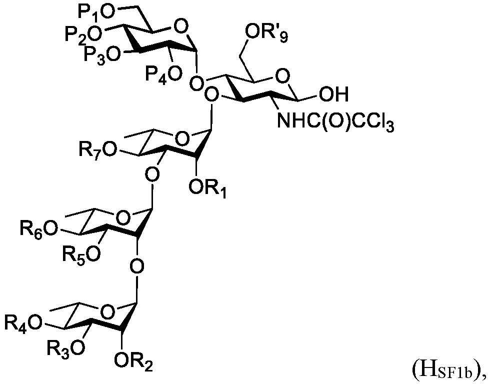 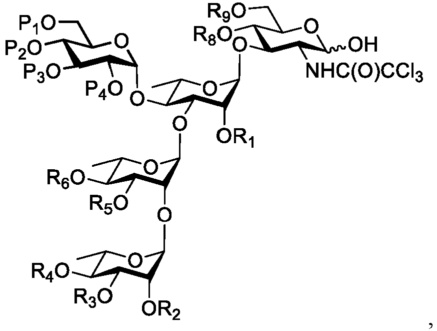 in particular 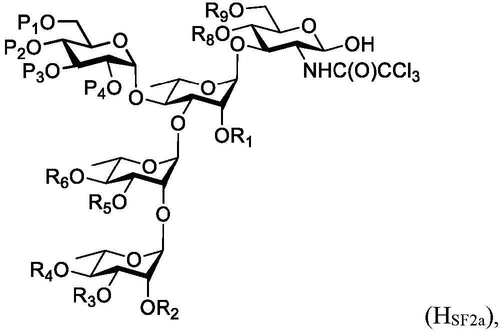 or 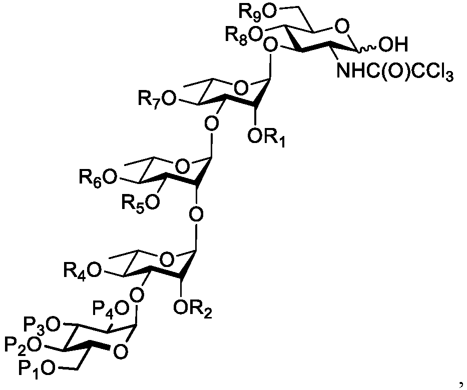 in particular 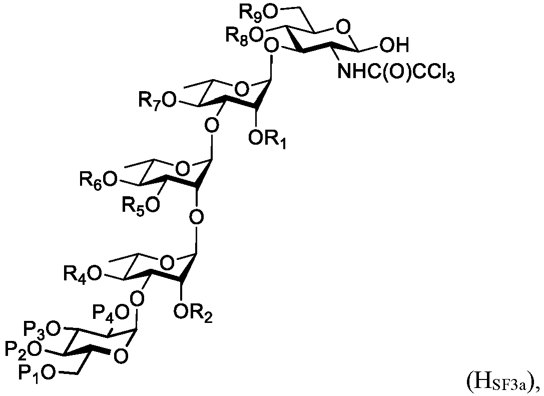 wherein R₁-R₉ and R'₉, P₁, P₂, P₃, and P₄ are as defined above,
and, for SF1b', wherein R₃ may also represent Bn.

In another aspect, the invention also concerns a functionalized pentasaccharide compound of following formula (III_{F}) : 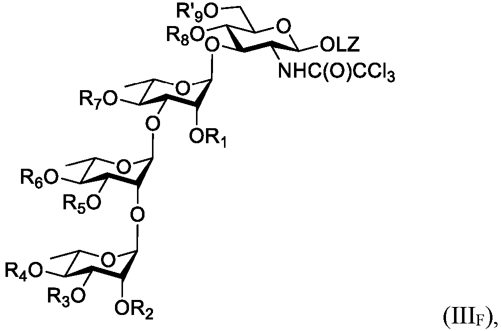 wherein :
L and Z are as defined above;
R₈, R₇ or R₃ represents a protected residue α-D-Glc*p*- ;
the other R₁-R₈ and R'₉ groups are as defined above.

In a particular embodiment, the invention also concerns a functionalized pentasaccharide as defined above, wherein Z is Hal, biotin, C₂-C₆ alkenyl, C₂-C₆ alkynyl, azido, alkoxy, epoxyde, acetal, C(=O)H, SR₁, S(=O)₂-R₁, NHCbz, or NBnCbz, or COOBn,
R₁ being H, C(=O)CH₃ or SR₂, and
R₂ being a C₁-C₆ alkyl, a C₆-C₁₀ aryl, or a 5 to 7 membered heteroaryl, such as pyridyl, or any group allowing to convert SSR₂ into SH.

In a particular embodiment, the invention also concerns a functionalized compound as defined above, of following formula : 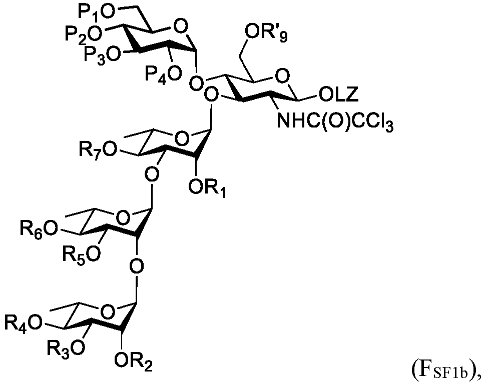 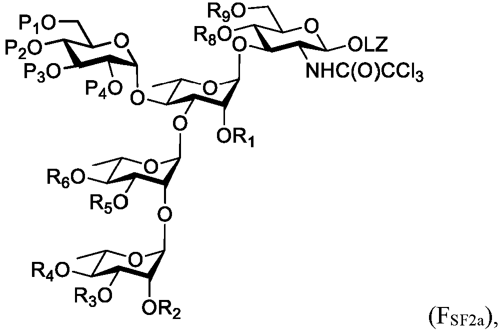 or 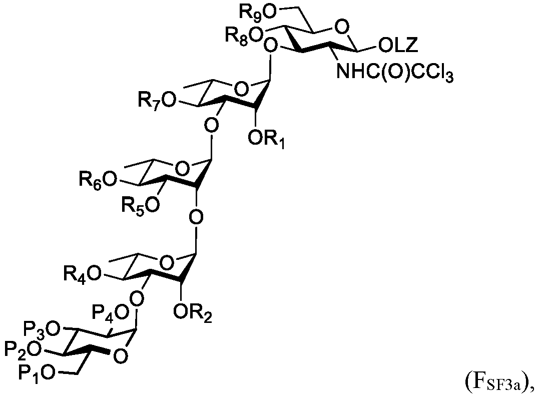 wherein R₁-R₉ and R'₉, P₁, P₂, P₃, P₄, L and Z are as defined above,
and, for SF1b', wherein R₃ may also represent Bn.

In another aspect, the invention also concerns a process of preparation of a functionalized pentasaccharide compound of following formula (III_{F}), comprising a step of contacting a pentasaccharide donor compound of formula (III_{D}), wherein G is in particular PTFA or TCA, with a compound of formula HO-LZ', wherein Z' is Z, optionally protected, or a group enabling to form Z, in particular in presence of TMSOTf, TfOH, TBSOTf, AgOTf, Tf₂O, Bi(OTf)₃, Yb(OTf)₃, I₂/Et₃SiH, TMSB(C₆F₅)₄, acid-washed molecular sieves, ZnBr₂, or BF₃-Et₂O, notably in catalytical conditions.

In another aspect, the invention also concerns a functionalized pentasaccharide acceptor compound of following formula (III_{AF}) : 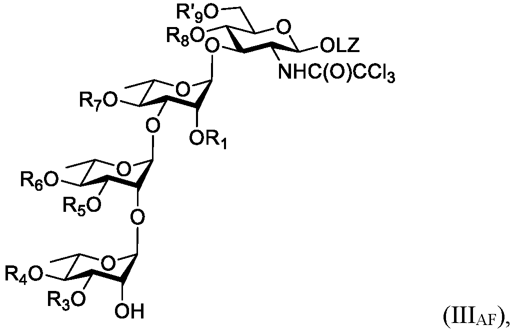 wherein Rs, R₇ or R₃ represents a protected residue α-D-Glc*p*- ;
the other R₁-R₈ and R'₉ groups, L and Z are as defined above.

In a particular embodiment, the invention also concerns a functionalized pentasaccharide acceptor compound as defined above, of following formula : 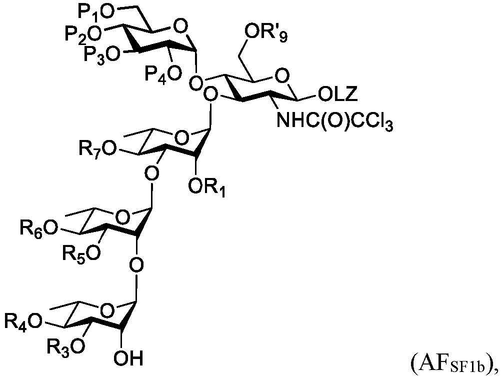 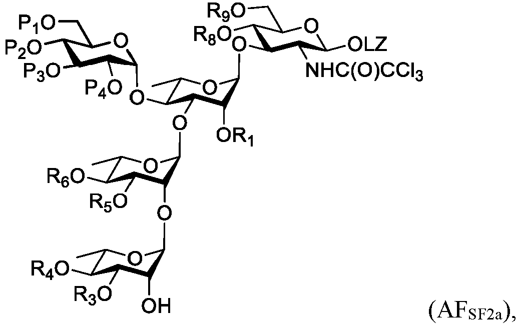 or 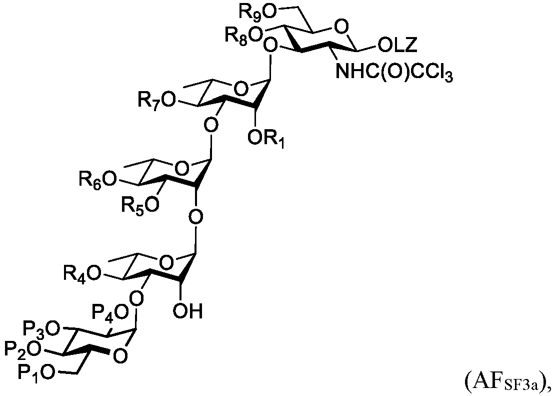 wherein R₁, R₃-R₉ and R'₉, P₁, P₂, P₃, P₄, L and Z are as defined above,
and, for SF1b', wherein R₃ may also represent Bn.

In another aspect, the invention also concerns a process of preparation of a functionalized pentasaccharide acceptor compound of following formula (III_{AF}), comprising a step of deprotection of the group R₂ of a compound of formula (III_{F}), in particular using hydrazine acetate or hydrazine in a mixture pyridine/acetic acid, in particular in a 1:1 (v/v) pyridine/acetic acid mixture when R₂ is Lev.

In another aspect, the invention also concerns a polysaccharide compound of formula R₂-P'-OQ', wherein P' is constituted of or comprises at least two units, and in particular at most nine or ten units, chosen from -SF1b'-, -SF2a'-, and/or -SF3a'- of following formulae: 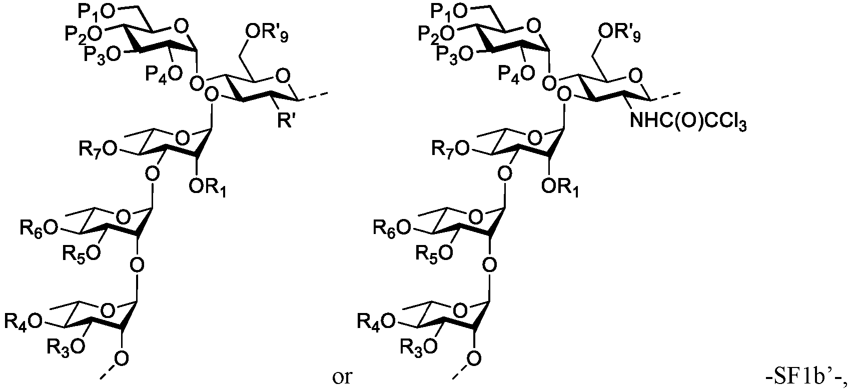 R₁ being in particular Ac, more particularly with P' being constituted of or comprising at least two units chosen from -SF1b'-, -SF2a'-, and -SF3a'-, at least two of these units being different, 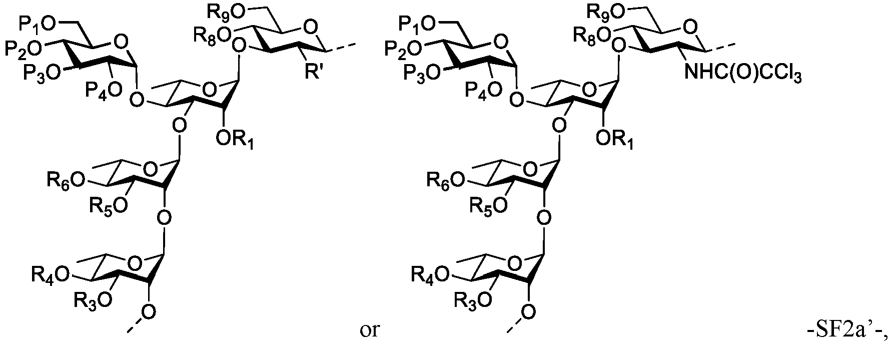 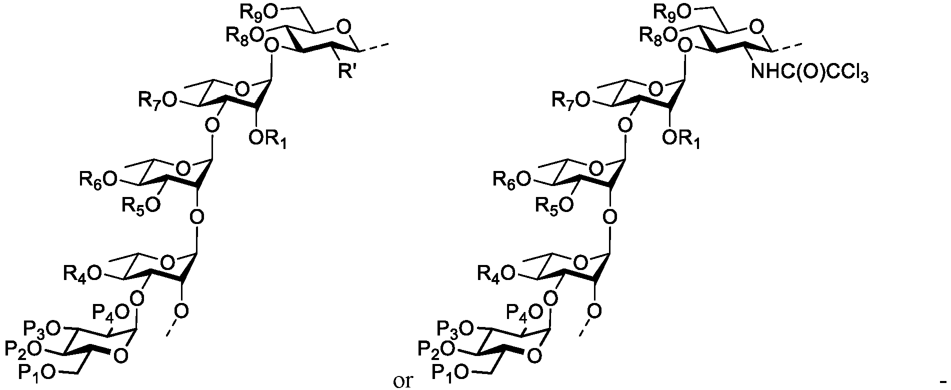 SF3a'-, R₁ being in particular Ac, more particularly with P' being constituted of or comprising at least two units chosen from -SF1b'-, -SF2a'-, and -SF3a'-, at least two of these units being different,
wherein R₁-R₉ and R'₉, P₁, P₂, P₃, and P₄ are as defined above,
and wherein Q' is H, All, or LZ as defined above,
and, for SF1b', wherein R₃ may also represent Bn.

In a particular embodiment, the invention also concerns a polysaccharide compound as defined above, being a homopoly-pentasaccharide of formula R₂-P'-OQ', wherein P' is constituted of or comprises at least two units, and in particular at most 6 units, more particularly 2 or 3, of formula -SF1b'-, -SF2a'-, or -SF3a', R₂ and Q' being as defined above.

In a particular embodiment, the invention also concerns a polysaccharide compound as defined above, being a heteropoly-pentasaccharide (or chimeric pentasaccharide) of formula R₂-P'-OQ', wherein P' is constituted of or comprises at least two units, and in particular at most 6 units, more particularly 2, 3, 4 or 5, chosen from -SF1b'-, -SF2a'-, and -SF3a'-, at least two of these units being different.

In a particular embodiment, the invention also concerns a polysaccharide compound as defined above, which is selected from the following poly-pentasaccharides:

R₂-[(SF2a')ₓ-(SF3a')_{y}-(SF1b')_{z}]ₙ-OQ';

R₂-[(SF2a')ₓ-(SF1b')_{z}-(SF3a')_{y}]ₙ-OQ';

R₂-[(SF3a')_{y}-(SF2a')ₓ-(SF1b')_{z}]ₙ-OQ';

R₂-[(SF3a')_{y}-(SF1b')_{z}-(SF2a')ₓ]ₙ-OQ';

R₂-[(SF1b')_{z}-(SF3a')_{y}-(SF2a')ₓ]ₙ-OQ';

R₂-[(SF1b')_{z}-(SF2a')ₓ-(SF3a')_{y}]ₙ-OQ';

wherein x, y and z are independently chosen from 0, 1 and 2, with at least two of x, y and z being 1 or 2.

In a particular embodiment, the invention also concerns apolysaccharide compound as defined above, which is selected from the following poly-pentasaccharides:

R₂-[SF1b']ₙ-OQ';

wherein n is in particular from 2 to 6, more particularly 2 or 3;

R₂-[SF2a']ₙ-OQ';

wherein n is in particular from 2 to 6, more particularly 2 or 3;

R₂-[SF3a']ₙ-OQ';

wherein n is in particular from 2 to 6, more particularly 2 or 3;

R₂-[SF2a'-SF1b']ₙ-OQ';

wherein n is in particular 1;

R₂-[(SF2a')₂-SF1b']ₙ-OQ';

wherein n is in particular 1;

R₂-[(SF2a')₂-(SF1b')₂]ₙ-OQ';

wherein n is in particular 1;

R₂-[SF3a'-SF1b']ₙ-OQ';

wherein n is in particular 1;

R₂-[(SF3a')₂-SF1b']ₙ-OQ'; wherein n is in particular 1;

R₂-[SF3a'-SF2a']ₙ-OQ';

wherein n is in particular 1;

R₂-[SF2a'-SF3a']ₙ-OQ';

wherein n is in particular 1;

R₂-[(SF2a')₂-SF3a']ₙ-OQ';

wherein n is in particular 1;

R₂-[(SF2a')₂-(SF3a')₂]ₙ-OQ';

wherein n is in particular 1;

R₂-[SF3a'-(SF2a')₂]ₙ-OQ';

wherein n is in particular 1;

R₂-[(SF3a')₂-(SF2a')₂]ₙ-OQ'; wherein n is in particular 1;

R₂-[SF3a'-SF2a'-SF1b']ₙ-OQ';

wherein n is in particular 1;

R₂-[SF3a'-(SF2a')₂-SF1b']ₙ-OQ';

wherein n is in particular 1;

R₂-[SF2a'-SF3a'-SF1b']ₙ-OQ';

wherein n is in particular 1;

R₂-[(SF2a')₂-(SF3a')₂-SF1b']ₙ-OQ';

wherein n is in particular 1;

R₂-[(SF3a')₂-(SF2a')₂-SF1b']ₙ-OQ';

wherein n is in particular 1; wherein:
Q' is H, All, or LZ as defined above;
SF1b', SF2a', and SF3a' are as defined above.

In a particular embodiment, the invention also concerns a polysaccharide compound as defined above, wherein P' is constituted of or comprises at least two units chosen from -SF1b'-, -SF2a'-, -SF3a'- and -ABCD'- of following formula: 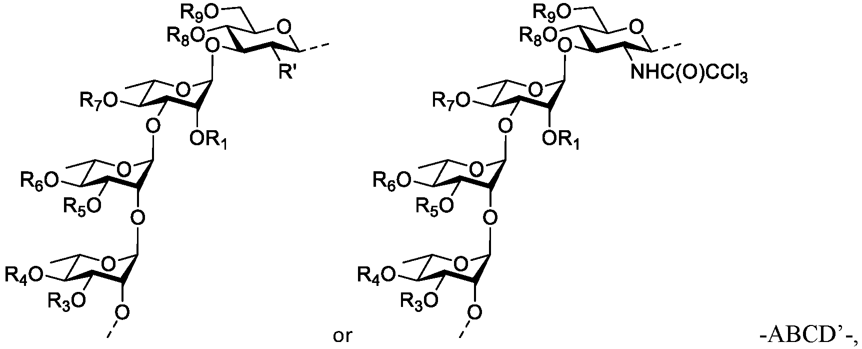 wherein R₁ and R₃-R₉ are as defined above,
P' comprising at least one unit, in particular at least two units chosen from -SF1b'-, -SF2a'-, - SF3a'-, and at least one and -ABCD'- unit, in particular one -ABCD'- unit, more particularly between two units chosen from -SF1b'-, -SF2a'-, -SF3a'-.

The ABCD unit may be seen as the RU of the SFY serotype.

In a particular embodiment, the invention also concerns a polysaccharide compound as defined above, which is selected from the following polysaccharides:

R₂-[SF2a'-ABCD'-SF3a']ₙ-OQ';

R₂-[SF2a'-ABCD'-SF1b']ₙ-OQ';

wherein:
n is 1;
Q' is H, All, or LZ as defined above;
SF1b', SF2a', and SF3a' are as defined above.

In another aspect, the invention also concerns a process of preparation of a polysaccharide as defined above, comprising:
a) a step of contacting a pentasaccharide acceptor compound of formula (III_{A}) or (III_{AF}), or a ABCD' acceptor, and a pentasaccharide donor compound of formula (III_{D}) or a ABCD' donor;
   and optionally:
b) a step:
   - of converting the polysaccharide obtained in a previous step into an acceptor by deprotecting the R₂ group, in particular using hydrazine acetate or hydrazine in a mixture pyridine/acetic acid, in particular in a 1:1 (v/v) pyridine/acetic acid mixture when R₂ is Lev; or
   - of converting the polysaccharide obtained in a previous step into a donor by first deprotecting the All group, in particular in particular using PdCl₂ and then N-iodosuccinimide (NIS), and then of contacting the obtained compound with a compound of type D'-LG wherein D' is a donor group and LG is a leaving group, for example PTFA-Cl when D' is PTFA;
      and
c) a step of contacting the acceptor or donor obtained in step b) with a pentasaccharide donor compound of formula (III_{D}) or a ABCD' donor; or a pentasaccharide acceptor compound of formula (III_{A}) or (III_{AF}) or a ABCD' acceptor, respectively, or an acceptor or donor obtained in one of the previous steps, respectively;
   step b) and c) being repeated if necessary;
   and/or
d) a step of functionalizing if necessary the polysaccharide obtained in a previous step by deprotecting the All group, in particular using PdCl₂ and then N-iodosuccinimide (NIS), contacting the obtained compound with a compound of type D'-LG wherein D' is a donor group and LG is a leaving group, for example PTFA-Cl when D' is PTFA, and then contacting the obtained donor with a compound of formula HO-LZ, in particular in presence of TMSOTf, TfOH, TBSOTf, AgOTf, Tf₂O, Bi(OTf)₃, Yb(OTf)₃, I₂/Et₃SiH, TMSB(C₆F₅)₄, acid-washed molecular sieves, ZnBr₂, or BF₃-Et₂O, notably in catalytical conditions;
   step d) taking place after step c), or after one of steps c) if step c) is repeated), in particular after last step c).

In a particular embodiment, the invention also concerns a process of preparation of a polysaccharide as defined above, comprising:
**a)** a step of contacting a pentasaccharide acceptor compound of formula (III_{A}), or a ABCD' acceptor, and a pentasaccharide donor compound of formula (III_{D}) or a ABCD' donor;
   and optionally:
**b)** a step:
   - of converting the polysaccharide obtained in a previous step into an acceptor by deprotecting the R₂ group, in particular using hydrazine acetate or hydrazine in a mixture pyridine/acetic acid, in particular in a 1:1 (v/v) pyridine/acetic acid mixture when R₂ is Lev; or
   - of converting the polysaccharide obtained in a previous step into a donor by first deprotecting the All group, in particular in particular using PdCl₂ and then N-iodosuccinimide (NIS), and then of contacting the obtained compound with a compound of type D'-LG wherein D' is a donor group and LG is a leaving group, for example PTFA-Cl when D' is PTFA;
   and
**c)** a step of contacting the acceptor or donor obtained in step b) with a pentasaccharide donor compound of formula (III_{D}) or a ABCD' donor; or a pentasaccharide acceptor compound of formula (III_{A}) or a ABCD' acceptor, respectively, or an acceptor or donor obtained in one of the previous steps, respectively;
   step b) and c) being repeated if necessary;
   and comprising :
**d)** a step of functionalizing if necessary the polysaccharide obtained in a previous step by deprotecting the All group, in particular using PdCl₂ and then N-iodosuccinimide (NIS), contacting the obtained compound with a compound of type D'-LG wherein D' is a donor group and LG is a leaving group, for example PTFA-Cl when D' is PTFA, and then contacting the obtained donor with a compound of formula HO-LZ, in particular in presence of TMSOTf, TfOH, TBSOTf, AgOTf, Tf₂O, Bi(OTf)₃, Yb(OTf)₃, I₂/Et₃SiH, TMSB(C₆F₅)₄, acid-washed molecular sieves, ZnBr₂, or BF₃-Et₂O, notably in catalytical conditions;
   step d) taking place after step c), or after the last of steps c) if step c) is repeated).

In a particular embodiment, the invention also concerns a process of preparation of a polysaccharide as defined above, being a poly-pentasaccharide comprising:
**a)** a step of contacting a pentasaccharide acceptor compound of formula (III_{A}) or (III_{AF}) and a pentasaccharide donor compound of formula (III_{D});
   and optionally:
**b)** a step:
   - of converting the poly-pentasaccharide obtained in a previous step into an acceptor by deprotecting the R₂ group , in particular using hydrazine acetate or hydrazine in a mixture pyridine/acetic acid, in particular in a 1:1 (v/v) pyridine/acetic acid mixture when R₂ is Lev; or
   - of converting the poly-pentasaccharide obtained in a previous step into a donor by first deprotecting the All group, in particular in particular using PdCl₂ and then N-iodosuccinimide (NIS), and then of contacting the obtained compound with a compound of type D'-LG wherein D' is a donor group and LG is a leaving group, for example PTFA-Cl when D' is PTFA;
   and
**c)** a step of contacting the acceptor or donor obtained in step b) with a pentasaccharide donor compound of formula (III_{D}) or a pentasaccharide acceptor compound of formula (III_{A}) or (III_{AF}), respectively, or an acceptor or donor obtained in one of the previous steps, respectively;
   step b) and c) being repeated if necessary;
   and/or
**d)** a step of functionalizing if necessary the poly-pentasaccharide obtained in a previous step by deprotecting the All group, in particular using PdCl₂ and then N-iodosuccinimide (NIS), contacting the obtained compound with a compound of type D'-LG wherein D' is a donor group and LG is a leaving group, for example PTFA-Cl when D' is PTFA, and then contacting the obtained donor with a compound of formula HO-LZ, in particular in presence of TMSOTf, TfOH, TBSOTf, AgOTf, Tf₂O, Bi(OTf)₃, Yb(OTf)₃, I₂/Et₃SiH, TMSB(C₆F₅)₄, acid-washed molecular sieves, ZnBr₂, or BF₃-Et₂O, notably in catalytical conditions;
step d) taking place after step c), or after one of steps c) if step c) is repeated), in particular after last step c).

In a particular embodiment, the invention also concerns a process of preparation of a polysaccharide as defined above, comprising:
**a)** a step of contacting a pentasaccharide acceptor compound of formula (III_{A}), and a pentasaccharide donor compound of formula (III_{D}) or a ABCD' donor;
   and optionally:
**b)** a step:
   - of converting the polysaccharide obtained in a previous step into an acceptor by deprotecting the R₂ group, in particular using hydrazine acetate or hydrazine in a mixture pyridine/acetic acid, in particular in a 1:1 (v/v) pyridine/acetic acid mixture when R₂ is Lev; or
   - of converting the polysaccharide obtained in a previous step into a donor by first deprotecting the All group, in particular in particular using PdCl₂ and then N-iodosuccinimide (NIS), and then of contacting the obtained compound with a compound of type D'-LG wherein D' is a donor group and LG is a leaving group, for example PTFA-Cl when D' is PTFA;
   and
**c)** a step of contacting the acceptor or donor obtained in step b) with a pentasaccharide donor compound of formula (III_{D}); or a pentasaccharide acceptor compound of formula (III_{A}), respectively, or an acceptor or donor obtained in one of the previous steps, respectively;
   step b) and c) being repeated if necessary;
   and comprising :
**d)** a step of functionalizing if necessary the polysaccharide obtained in a previous step by deprotecting the All group, in particular using PdCl₂ and then N-iodosuccinimide (NIS), contacting the obtained compound with a compound of type D'-LG wherein D' is a donor group and LG is a leaving group, for example PTFA-Cl when D' is PTFA, and then contacting the obtained donor with a compound of formula HO-LZ, in particular in presence of TMSOTf, TfOH, TBSOTf, AgOTf, Tf₂O, Bi(OTf)₃, Yb(OTf)₃, I₂/Et₃SiH, TMSB(C₆F₅)₄, acid-washed molecular sieves, ZnBr₂, or BF₃-Et₂O, notably in catalytical conditions;
step d) taking place after step c), or after the last of steps c) if step c) is repeated).

In a particular embodiment, the invention also concerns a process as defined above, further comprising a step of conversion of the R₁ protecting group into an acetyl group (Ac), on one, some or all of the donors and acceptors before their use, in particular on one, some or all of the pentasaccharide acceptor compounds of formula (III_{A}) or (III_{AF}) and pentasaccharide donor compounds of formula (III_{D}) before their use.

In a particular embodiment, the invention also concerns a process as defined above, further comprising:
- after the corresponding deprotection, a step of acetylation of the 3_{A} position and/or the 6_{D} position to obtain a 3_{A}- and/or 6_{D}-*O*-acetylated compound, in particular in 3_{A} and 6_{D} for SF2a (and for SF6), 3_{A} for SF1b, and in 6_{D} for SF3a;
- a step of cleavage of the ClAc or BrAc protecting group borne by C to obtain a 2c-hydroxylated compound, said step being in particular followed by an acetylation; or
- a conversion of the masking group borne by D' into an acetyl group (Ac), in particular when the masking group is not Cl₃Ac.

In another aspect, the invention also concerns a process of preparation of a glycoconjugate of formula H-P-OLZ', wherein:
P is constituted of or comprises at least two units chosen from -SF1b-, -SF2a-, -SF3a- and - ABCD-, wherein:
SFlb is ABC(E)D, SF2a is AB(E)CD, SF3a is (E)ABCD, with A is 2)-α-L-Rhap-(1→ , B is 2)-α-L-Rha*p*-(1→ , C is 3)-α-L-Rha*p*-(1→ , D is 3)-β-D-Glc*p*NAc-(1→, or, when D is in terminal position, 3)-β-D-Gl*c*pNAc-(1→ or 3)-α -D-Glc*p*NAc-(1→ , E represents a residue α-D-Glc*p*-;
L is:
   - a single bond,
   - a divalent C₁-C₁₂ alkyl, C₂-C₁₂ alkenyl or C₂-C₁₂ alkynyl chain optionally interrupted by one or more heteroatoms, notably selected from an oxygen atom, a sulphur atom or a nitrogen atom, said nitrogen and sulphur atoms being optionally oxidized, and the nitrogen atom being optionally involved in an acetamide bond, or
   - a divalent C₁-C₁₂ alkyl, C₂-C₁₂ alkenyl or C₂-C₁₂ alkynyl chain substituted by at least one -OH group, being in particular of the following formula -(CH₂-CH₂-C(OH))_{q}-(CH₂-CH₂)ᵢ, wherein i is 0 or 1 and q ranges from 1 to 10,
Z' is Z₁ or F₁-L₂-Z₂,
Z₁ is a terminal function or group, optionally protected, able to form a covalent bond with a carrier and/or a solid support, or a multivalent scaffold; an anchor; a mono-, oligo- or polysaccharide; or a dye or fluorescent residue.
F₁ is any group enabling to bond the linker L to the linker L₂, F₁ being in particular chosen from the -C(=O)-, -C(=O)-C(=O)-, -C(=O)-C(=O)-NH-, -NHC(=O)-C(=O)-, -NHC(=O)-C(=O)-NH-, -C(=O)-C(H)=N-NH-, -NH-C(=O)-C(H)=N-NH-, ester, amide, amine, -CH₂-, ether, thioether, imine, thio-succinimide, oxime, hydrazone, hydrazonamide, -C(=O)CH₂-NH-, -NH-CH₂-C(=O)-, triazole functions or groups, and from the following: 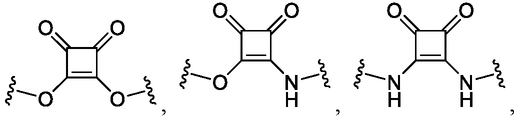
L₂ is a single bond, divalent C₁-C₁₂ alkyl, C₂-C₁₂ alkenyl or C₂-C₁₂ alkynyl chain optionally interrupted by one or more heteroatoms, notably selected from an oxygen atom, a sulphur atom or a nitrogen atom, said nitrogen and sulphur atoms being optionally oxidized, and the nitrogen atom being optionally involved in an acetamide bond,
Z₂ is Z₁ or F₂-L₃-Z₁,
F₂ is any group enabling to bond the linker L to the linker L₃, F₁ being in particular chosen from the -C(=O)-, -C(=O)-C(=O)-, -C(=O)-C(=O)-NH-, -NHC(=O)-C(=O)-, -NHC(=O)-C(=O)-NH-, -C(=O)-C(H)=N-NH-, -NH-C(=O)-C(H)=N-NH-, ester, amide, amine, -CH₂-, ether, thioether, imine, thio-succinimide, oxime, hydrazone, hydrazonamide, -C(=O)CH₂-NH-, -NH-CH₂-C(=O)-, triazole functions or groups, and from the following: 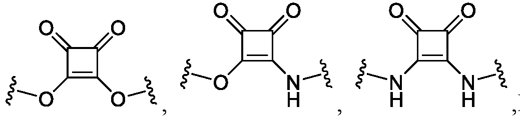
L₃ is single bond, a divalent C₁-C₁₂ alkyl, C₂-C₁₂ alkenyl or C₂-C₁₂ alkynyl chain optionally interrupted by one or more heteroatoms, notably selected from an oxygen atom, a sulphur atom or a nitrogen atom, said nitrogen and sulphur atoms being optionally oxidized, and the nitrogen atom being optionally involved in an acetamide bond,
said process comprising :
   **a)** One or more steps of full deprotection of a polysaccharide of formula R₂-P'-LZ as defined above, to obtain a H-P-OLZ₁ compound or a H-P-OLF₁ compound, L and Z₁ being as defined above, F₁' being a precursor of F₁ as defined above;
   **b)** Optionally, a step of contacting the fully deprotected polysaccharide obtained in the previous step with:
      - a compound of following formula F₁"-L₂-Z₁, F₁" being a precursor of F₁ as defined above, L₂ and Z₁ being as defined above, or
      - a compound of following formula F₁"-L₂-F₂', F₁" being a precursor of F₁ as defined above, F₂' being a precursor of F₂ as defined above, L₂ being as defined above, followed by contacting the obtained compound with a compound of following formula F₂"-L₃-Z₁, wherein F₂" is a precursor of F₂ as defined above, and L₃ and Z₁ being as defined above.

The LZ group may be of one of the following formulae:
- L-Z₁;
- L-F₁-L₂-Z₁; or
- L-F₁-L₂-F₂-L₃-Z₁.

In a particular embodiment, Z₁ is a terminal reactive function or group, optionally protected, able to form a covalent bond with a carrier and/or a solid support.

In particular embodiment, the carrier and/or a solid support presents on its surface functionalities, more particularly primary amino functionalities, notably of lysine residues, that are able to react with Z₁.

In another particular embodiment, the carrier and/or a solid support presents on its surface functionalities, more particularly primary amino functionalities, notably of lysine residues, linked to an interconnecting molecule, which is able to react with the Z₁ group of the oligo- and polysaccharides of the invention.

In a particular embodiment, Z₁ is a multivalent scaffold; an anchor; a mono-, oligo- or polysaccharide; or a dye or fluorescent residue.

In a particular embodiment, L₂ is a single bond, and F₁ and Z₂ or Z₁ are one and only group.

In a particular embodiment, L₃ is a single bond, and F₂ and Z₁ are one and only group.

By anchor is in particular meant a residue able to form a non-covalent type attachment with a carrier and/or a solid support. Said anchor is for example biotine, able to form non-covalent bonds with streptavidine bound to a solid support.

By multivalent scaffold is in particular meant a scaffold able to form at least two bonds, each one with one compound of formula (II) of the present invention. Said multivalent scaffold is for example a linear polymer, a dendrimer, a monosaccharide, a cyclic peptide, or a (poly)-lysine scaffold.

In a particular embodiment, Z₁ is a terminal reactive function or group, optionally protected, able to form a covalent bond with a carrier and/or a solid support.
F₁' and F₁" are for example:
   - -C(=O)-C(=O) and an amine, forming a -C(=O)-C(=O)-NH- group,
   - -C(=O)-C(=O)H and an amine, forming a -C(=O)-C(H)=N-NH- group,
   - A carboxylic acid or an activated ester and an alcohol, forming an ester,
   - an activated ester and an amine, forming an amide,
   - an amine and a leaving group, forming an amine,
   - a thiol and a leaving group, forming thioether,
   - a thiol and an allyl,
   - an amine and an aldehyde, forming an imine,
   - an amine and an aldehyde, forming an amine, in particular by reductive amination,
   - a thiol and a maleimide, forming a thio-succinimide,
   - an azide and an alkyne, forming a triazole, in particular by click chemistry,
   - C(=O)CH₂-Hal, in particular Br, and an amine, forming a C(=O)CH₂-NH group,
   - NHC(=O)CH₂-Hal, in particular Br, and an amine, forming a NHC(=O)CH₂-NH group,
   - C(=O)CH₂-Hal, in particular Br, and a thiol, forming a C(=O)CH₂-SH group,
   - NHC(=O)CH₂-Hal, in particular Br, and a thiol, forming a NHC(=O)CH₂-SH group, 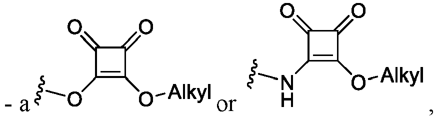 and an amine or a alcohol, forming 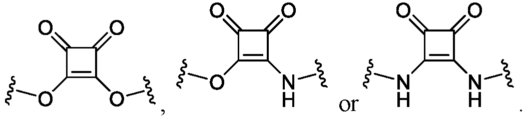
F₂' and F₂" are for example:
   - -C(=O)-C(=O) and an amine, forming a -C(=O)-C(=O)-NH- group,
   - -C(=O)-C(=O)H and an amine, forming a -C(=O)-C(H)=N-NH- group,
   - A carboxylic acid or an activated ester and an alcohol, forming an ester,
   - an activated ester and an amine, forming an amide,
   - an amine and a leaving group, forming an amine,
   - a thiol and a leaving group, forming thioether,
   - a thiol and an allyl,
   - an amine and an aldehyde, forming an imine,
   - an amine and an aldehyde, forming an amine, in particular by reductive amination,
   - a thiol and a maleimide, forming a thio-succinimide,
   - an azide and an alkyne, forming a triazole, in particular by click chemistry,
   - C(=O)CH₂-Hal, in particular Br, and an amine, forming a C(=O)CH₂-NH group,
   - NHC(=O)CH₂-Hal, in particular Br, and an amine, forming a NHC(=O)CH₂-NH group,
   - C(=O)CH₂-Hal, in particular Br, and a thiol, forming a C(=O)CH₂-SH group,
   - NHC(=O)CH₂-Hal, in particular Br, and a thiol, forming a NHC(=O)CH₂-SH group,
   - a 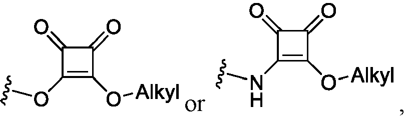 and an amine or a alcohol, forming

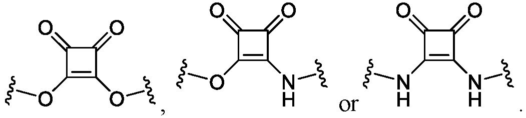

Reference is also made regarding glyoxylyl group to Bioconjugate Chem. 2013, 24, 735 -765

In a particular embodiment, F₁" and F₂' are orthogonal (i.e. in particular they do react together).

In a particular embodiment, L is a divalent C₁-C₁₂ alkyl or alkenyl chain optionally interrupted by one or more heteroatoms, notably selected from an oxygen atom, a sulphur atom or a nitrogen atom, said nitrogen and sulphur atoms being optionally oxidized, and the nitrogen atom being optionally involved in an acetamide bond, and F₁' is before deprotection a N₃, NHCbz, or NBnCbz group, L-F₁' being before deprotection notably a PEG chain bearing a N₃, NHCbz, NBnCbz, or SBn group (for this latter, see for example Chem. Sci. 2014, 5, 1992).

In a particular embodiment, the F₁' group reacts with F₁"-L₂-Z₁ which is a compound bearing a first reactive function that will react with the F₁' residue to form the F₁ function. In a particular embodiment, the F₁' group reacts with F₁"-L₂-F₂' that further comprises a second reactive function F₂', which is orthogonal to the first reactive function F₁".

In a more particular embodiment, L-Z₁ or L-F₁' is -(CH₂)ₚ-NH₃⁺or -(CH₂)ₚ-NH₂, wherein p ranges from 1 to 10, in particular from 2 to 8, more particularly 2, 3, 4, 5 or 6, and F₁"-L₂-Z₁ or F₁"-L₂-F₂' is an activated version, in particular an activated ester of the compound of the following formula:

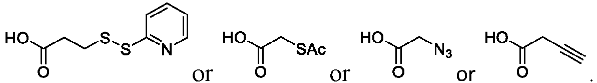

In another more particular embodiment, HO-L-Z₁ or HO-L-F₁' is HO-(CH₂)ₚ-CH₂=CH₂, and F₁"-L₂-Z₁ is a thiol, for example HS-Bn. Reference is in particular made to Angew. Chem. Int. Ed. 2014, 53, 3894 -3898.

In another more particular embodiment, L-Z₁ or L-F₁' is -(CH₂)ₚ-SH (reference is in particular made for this latter case to Chem. Eur. J. 2004, 10, 4265 - 4282), wherein p ranges from 1 to 10, in particular from 2 to 8, more particularly 2, 3, 4, 5 or 6.

In a particular embodiment, L-Z₁ or L-F₁' is -(CH₂)ₚ-OH, wherein p ranges from 1 to 10, in particular from 2 to 8, more particularly 2, 3, 4, 5 or 6. The introduced primary alcohol may for example be converted into an aldehyde moiety upon selective oxidation, and then react with a F₁"-L₂-Z₁ or F₁"-L₂-F₂' compound comprising a hydrazide-, an oxime- or a derivative. F₁"-L₂-Z₁ or F₁"-L₂-F₂' optionally further comprises a second reactive function, which is orthogonal to the first reactive function, and may be selected from alkene, alkyne and masked thiol groups.

In a particular embodiment, L-Z₁ or L-F₁' is -CH₂-C(OH)-CH₂-OH, and F₁"-L₂-Z₁ or F₁"-L₂-F₂' is of the following formula:

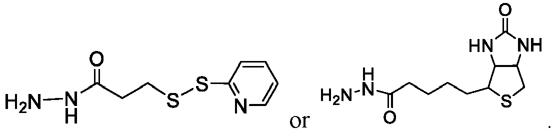

In another aspect, the invention also concerns a glycoconjugate of formula H-P-OLZ', as defined above, wherein P is constituted of or comprises:
- at least two different units chosen from -SF1b-, -SF2a-, -SF3a-;
- at least one -SF1b- unit, with LZ' being different from propyl when P is constituted of or comprises one -SF1b- unit; or
- at least one -ABCD- unit, in particular one -ABCD- unit, and at least one unit chosen from -SF1b-, -SF2a-, -SF3a-, and
And wherein:
- the ABCD units are optionally substituted by at least one phosphoethanolamine (PEtN-modified); and/or
- the units, in particular the -SF1b-, -SF2a-, -SF3a- units are optionally acetylated, said acetylation being partial or stoichiometric.

In a particular embodiment, the invention also concerns aglycoconjugate as defined above, being of one of the following formulae:

H-[SF1b]ₙ-OLZ';

H-[SF2a-SF1b]ₙ-OLZ';

H-[(SF2a)₂-SF1b]ₙ-OLZ';

H-[(SF2a)2-(SF1b)₂]ₙ-OLZ';

H-[SF3a-SF1b]ₙ-OLZ';

H-[(SF3a)₂-SF1b]ₙ-OLZ';

H-[SF3a-SF2a]ₙ-OLZ';

H-[SF2a-SF3a]ₙ-OLZ';

H-[(SF2a)₂-SF3a]ₙ-OLZ';

H-[(SF2a)₂-(SF3a)₂]ₙ-OLZ';

H-[SF3a-(SF2a)₂]ₙ-OLZ';

H-[(SF3a)₂-(SF2a)₂]ₙ-OLZ';

H-[SF3a-SF2a-SF1b]ₙ-OLZ';

H-[SF3a-(SF2a)₂-SF1b]ₙ-OLZ';

H-[SF2a-SF3a-SF1b]ₙ-OLZ';

H-[(SF2a)₂-(SF3a)₂-SF1b]ₙ-OLZ';

H-[SF2a-ABCD-SF3a]ₙ-OLZ';

H-[SF2a-ABCD-SF1b]ₙ-OLZ'.

And wherein:
- the ABCD units are optionally substituted by at least one phosphoethanolamine (PEtN-modified); and/or
- the units, in particular the -SF1b-, -SF2a-, -SF3a- units are optionally O-acetylated, said acetylation being partial or stoichiometric, -SF3a- being in particular stoichiometrically *O*-acetylated in position 2c, -SF1b- being in particular O-acetylated, more particularly partially, in position 2c.

In a particular embodiment, the glycoconjugate is H-[SF1b]ₙ-OLZ', which is partially *O*-acetylated.

In a particular embodiment, the glycoconjugate is H-[SF1b]ₙ-OLZ', which is partially *O*-acetylated, in particular on position 2c.

In a particular embodiment, the glycoconjugate is H-[SF1b]ₙ-OLZ', which is O-acetylated, in particular on position 2c, when n=1, or *O*-acetylated or partially *O*-acetylated, in particular on position 2c, when n is not 1.

Such acetylation is for example described in FEMS Immunol Med Microbiol 66, 201 (2012), and Biochemistry (Moscow), 2015, Vol. 80, No. 7, pp. 901-914 and 1072-1087.

In another aspect, the invention also concerns a glycoconjugate of formula H-P-OLZ', as defined above, wherein P is constituted of or comprises at least one -SF2a- or -SF3a- units.

In a particular embodiment, the number of -SF2a- or -SF3a- units is 3 or 4 or more, and in particular less than 100, 50, 20, or 10.

In a particular embodiment, the number of -SF2a- or -SF3a- units is 1, 2 or 3, and LZ' is different from propyl, aminoethyl and aminopropyl.

In a particular embodiment, the number of -SF3a- units is 1, 2 or 3, and LZ' is different from propyl, aminoethyl and aminopropyl.

In a particular embodiment, the number of -SF3a- units is 1, 2 or 3, and LZ' is different from aminoethyl and aminopropyl.

In another aspect, Z₁ is a terminal function or group, forming a covalent bond with a carrier and/or a solid support, or a multivalent scaffold; an anchor; a mono-, oligo- or polysaccharide; or a dye or fluorescent residue, an adjuvant.

Covalent linkage of synthetic oligo- and polysaccharides to proteins is known in the art and may for example be achieved by targeting the ε-amines of lysines, the carboxylic groups of aspartic/glutamic acids, the sulfhydryls of cysteines, or tyrosines. A reactive group, for example an amine, can also be introduced at the oligosaccharide reducing termini, directly or via a linker, to be used finally for insertion of a bifunctional linker for conjugation to the carrier.

For example, the oligo- or polysaccharide may be conjugated to the carrier through the reaction between a maleimido or haloacetyl group, in particular bromoacetyl group, bound to the oligo- or polysaccharide, in particular via a linker, and a thiol or a NH₂ group bound to the carrier, in particular via a linker; or through the reaction between a maleimido or haloacetyl group, in particular bromoacetyl group, bound to the carrier, in particular via a linker, and a thiol or a NH₂ group bound to the oligo- or polysaccharide, in particular via a linker.

To conjugate with a linker or crosslinking agent, either or both of the oligo- or polysaccharide and the carrier may be covalently bound to one or more linkers first. The linkers or crosslinking agents are homobifunctional or heterobifunctional molecules, *e.g*., adipic dihydrazide, ethylenediamine, cystamine, *N-*succinimidyl 3-(2-pyridyldithio)propionate (SPDP), *N*-acetyl-DL-homocysteine thiolactone, *N*'-succinimidyl-[*N*-(2-iodoacetyl)-β-alanyl] propionate (SIAP), 3,3'-dithiodipropionic acid, squarates and their derivatives, and the like.

According to the type of linkage between the oligo- or polysaccharide and the carrier, there is the possibility of preparing a conjugate wherein the ratio of the oligo- or polysaccharide versus the carrier can in particular vary between 1:1 and 500:1, notably between 1:1 and 200:1. More particularly, this ratio is comprised between 1:1 and 30:1, preferably between 5:1 and 25:1, more preferably between 8:1 and 30:1, or between 5:1 and 20:1, notably when the carrier is tetanus toxoid or a fragment thereof.

A carrier can be a natural, modified-natural, synthetic, semi-synthetic or recombinant material containing one or more functional groups, for example primary and/or secondary amino groups, azido groups, thiol, alkynyl, alkenyl, or carboxyl group. The carrier can be water soluble or insoluble. Carriers that fulfil these criteria are well-known to those of ordinary skill in the art.

Suitable carriers according to the present invention notably include proteins, peptides, lipopeptides, zwitterionic polysaccharides, lipid aggregates (such as oil droplets or liposomes), inactivated virus particles, nanoparticles, in particular gold nanoparticles (reference is for example made to Bioorganic Chemistry 99 (2020) 103815, or Nanomedicine 2012, 7:651-662), virus-like particles, for example bacteriophage Qβ (VLPs Methods Enzymol 2017;597:359-376) and Generalized Modules for Membrane Antigens (GMMA; reference is for example made to: Vaccines 2020, 8, 540; Vaccines (Basel). 2020 Apr 3;8(2):160).

In a particular embodiment, the carrier is a protein.

In this case, the term "carrier" refers in particular to a protein to which the oligo- or polysaccharide is coupled or attached or conjugated, typically for the purpose of enhancing or facilitating detection of the antigen by the immune system. Oligosaccharides are T-independent antigens that are poorly immunogenic and do not lead to long-term protective immune responses. Conjugation of the oligosaccharide antigen to a protein carrier changes the context in which immune effector cells respond to oligosaccharides. The term carrier protein is intended to cover both small peptides and large polypeptides (>10 kDa). In a particular embodiment, the carrier is an immunocarrier.

Immunocarriers are carriers chosen to increase the immunogenicity of the oligo- or polysaccharide and/or to raise antibodies against the carrier which are medically beneficial.

Suitable immunocarriers according to the present invention notably include proteins, glycosphingolipids, peptides, lipopeptides, lipid aggregates containing T-helper peptides (at least one), inactivated virus particles, nanoparticles, in particular gold nanoparticles, and Generalized Modules for Membrane Antigens (GMMA).

In a particular embodiment, the conjugate of the invention is covalently bound to a protein or a peptide comprising at least one T-helper epitope.

Protein carriers known to have potent T-helper epitopes, include but are not limited to bacterial toxoids such as tetanus, diphtheria and cholera toxoids, *Staphylococcus* exotoxin or toxoid, *Pseudomonas aeruginosa* Exotoxin A and recombinantly produced, genetically detoxified variants thereof, outer membrane proteins (OMPs) of *Neisseria meningitidis* and *Shigella* proteins. The recombinantly-produced, non-toxic mutant strains of *P. aeruginosa* Exotoxin A (rEPA) are described and used in polysaccharide-protein conjugate vaccines (Infect Immun 1993, 61, 1023-1032). The CMR197 carrier is a well characterized non-toxic diphtheria toxin mutant that is useful in glycoconjugate vaccine preparations intended for human use ( a) Adv Exp Med Biol 1989, 251, 175-180; b) Vaccine 1992, 10, 691-698). Other exemplary protein carriers include the Fragment C of tetanus toxin (WO 2005/000346, WO 2005/000346). Also CRM9 carrier has been disclosed for human immunisation (Pediatr Infect Dis J2003, 22, 701-706).

Useful carrier proteins include bacterial toxins or toxoids, such as diphtheria toxoid or tetanus toxoid. Fragments of toxins or toxoids can also be used e.g. fragment C of tetanus toxoid. The CRM 197 mutant of diphtheria toxin is a particularly useful with the invention. Other suitable carrier proteins include the *Neisseria meningitidis* outer membrane protein, synthetic peptides, heat shock proteins, pertussis proteins , cytokines, lymphokines, hormones, growth factors, human serum albumin (preferably recombinant) in particular for diagnostic aspects, artificial proteins comprising multiple human CD4+ T cell epitopes from various pathogen-derived antigens such as N19, protein D from *Haemophilus influenzae,* pneumococcal surface protein PspA, pneumolysin, iron-uptake proteins, toxin A or B from *Clostridium difficile,* recombinant *P. aeruginosa* exoprotein A (rEPA), a GBS protein, and the like.

Particularly suitable carrier proteins include CRM 197, tetanus toxoid (TT), tetanus toxoid fragment C, protein D, non-toxic mutants of tetanus toxin and diphtheria toxoid (DT). Other suitable carrier proteins include protein antigens GBS80, GBS67 and GBS59 from *Streptococcus agalactiae* and fusion proteins, for example, GBS59(6xD3) disclosed in WO2011/121576 and GBS59(6xD3)-1523 disclosed in EP14179945.2. The use as other suitable carrier proteins of proteins antigens that are common to several *Shigella* serotypes such as IpaD, IpaB, MxiH and all their possible combinations may also be advantageous. Another carrier could be genetically modified OMVs (GMMA). Synthetic peptides bearing immunodominant T-helper cell epitopes can also act as carriers in polysaccharide and oligosaccharide conjugates. The peptide carriers include polypeptides containing multiple T-helper epitopes addressing the extensive polymorphism of HLA molecules (Pediatrics 1993, 92, 827-832), and universal T-helper epitopes compatible with human use. Exemplary T-helper epitopes, include but are not limited to natural epitopes characterized from tetanus toxoid (J Immunol 1992, 149, 717-721), and non-natural epitopes or engineered epitopes such as the pan HLA DR-binding epitope PADRE (Immunity 1994, 1, 751-761).

In a particular embodiment of the present invention, the immunocarrier is selected among a protein or a peptide comprising at least one T-helper epitope, or a derivative thereof. In a particular aspect, the immunocarrier is the peptide PADRE.

In a particular embodiment of the present invention, the immunocarrier is tetanus toxoid (TT) or a fragment thereof.

In another particular embodiment of the present invention, the immunocarrier is CRM 197.

The term "toxoid" as used herein refers to a bacterial toxin (usually an exotoxin), whose toxicity has been inactivated or suppressed either by chemical (formalin) or heat treatment, while other properties, typically T-helper properties and/or immunogenicity, are maintained. A mutated toxoid as used herein is a recombinant bacterial toxin, which has been amended to be less toxic or even non-toxic by amending the wild-type amino acid sequence. Such a mutation could be a substitution of one or more amino acids. Such a mutated toxoid presents on its surface a functionality that can react with the functional group of the interconnecting molecule to provide a modified toxoid. Said functionality is known to the person skilled in the art and includes, but is not restricted to the primary amino functionality of a lysine residue that can react with activated esters, an isocyanate group or an aldehyde in presence of a reducing agent, to the carboxylate functionality of a glutamate or aspartate residue that can be activated by carbodiimides or to the thiol functionality of a cysteine residue.

Activated esters include, but are not restricted to N-(y-maleimidobutyryloxy) succinimide ester (GMBS), N-(y-maleimidobutyryloxy) sulfosuccinimide ester (sulfo-GMBS), succinimidyl (4-iodoacetyl) aminobenzoate (sulfo-SIAB), succinimidyl-3-(bromoacetamido)propionate (SBAP), disuccinimidyl glutarate (DSG), disuccinimidyl adipate (DSA), 2-pyridyldithiol-tetraoxatetradecane-N-hydroxysuccinimide (PEG-4-SPDP), bis-(4-nitrophenyl) adipate and bis-(4-nitrophenyl) succinate. Preferred activated esters are for example N-(y-maleimidobutyryloxy) succinimide ester (GMBS), N-(y-maleimidobutyryloxy) sulfosuccinimide ester (sulfo-GMBS), succinimidyl (4-iodoacetyl) aminobenzoate (sulfo-SIAB), succinimidyl-3-(bromoacetamido)propionate (SBAP).

The cysteine residue on the carrier protein can be converted to the corresponding dehydroalanine that can be further reacted with a suitable interconnecting molecule to provide modified carrier protein having on their surface the functional group of the interconnecting molecule.

For example, the inventive saccharides described herein are conjugated to the non-toxic mutated diphtheria toxin CRM₁₉₇ presenting as a functionality a primary amine functionality of a lysine residue.

CRM197 like wild-type diphtheria toxin is a single polypeptide chain of 535 amino acids (58 kD) consisting of two subunits linked by disulfide bridges having a single amino acid substitution of glutamic acid for glycine. It is utilized as a carrier protein in a number of approved conjugate vaccines for diseases such as Prevnar.

It is especially preferred that inventive saccharides described herein are conjugated to tetanus toxoid (TT) or a fragment thereof presenting as a functionality a primary amine functionality of a lysine residue.

It is especially preferred that inventive saccharides described herein are conjugated to CRM197 presenting as a functionality a primary amine functionality of a lysine residue.

Thus, in a preferred embodiment of the present invention the carrier protein presents on its surface primary amino functionalities of lysine residues that are able to react with the functional group of the interconnecting molecule to provide modified carrier protein having on their surface said functional group of the interconnecting molecule, which is able to react with the Z group of the oligo- and polysaccharides of the invention.

Said functional group of the interconnecting molecules is for example selected from the group comprising or consisting of maleimide; α-iodoacetyl; α-bromoacetyl; and N-hydroxysuccinimide ester (NHS), aldehyde, imidoester, carboxylic acid, alkyl sulfonate, sulfonyl chloride, epoxide, anhydride, carbonate.

Other types of carrier include but are not limited to biotin or liposomes. As regards the use of a liposome as a carrier, in particular those, which do not imply covalent linkages, reference could be made to the International Application WO 2010/136947.

In a particular embodiment of the present invention, the carrier is biotin (as an anchor) or biotin/avidin complex.

In a particular embodiment of the present invention, Z₁ is an adjuvant, for example PAMCys3(Vaccine 27 (2009) 5419-5426).

In a particular embodiment of the present invention, the carrier is a multivalent scaffold, i.e. a carrier that enables multiple presentation of the oligo- or polysaccharide of the invention, in particular a scaffold able to form at least two bonds, each one with an oligo- or polysaccharide of the invention. Said multivalent scaffold is for example a linear polymer, a dendrimer, a monosaccharide, a cyclic peptide, or a (poly)-lysine scaffold, for example MAP (Multiple Antigen Peptide), as for example described in Proc Natl Acad Sci U S A. 2013; 110(33): 13564-13569.

Compositions may include a small amount of free carrier. When a given carrier protein is present in both free and conjugated form in a composition of the invention, the unconjugated form is preferably no more than 5% of the total amount of the carrier protein in the composition as a whole, and more preferably present at less than 2% by weight.

After conjugation, free and conjugated oligosaccharides can be separated. There are many suitable methods, including hydrophobic chromatography, tangential ultrafiltration, diafiltration, etc.

In another object, the invention provides an immunogenic composition comprising a conjugate according to the invention and a physiologically acceptable vehicle.

All the embodiments related to the conjugate apply here as well, alone or in combination.

The immunogenic (or vaccine) composition includes one or more pharmaceutically acceptable excipients or vehicles such as water, saline, glycerol, or ethanol. Additionally, auxiliary substances, such as wetting or emulsifying agents, pH buffering substances, and the like, may be present in such vehicles.

The glycoconjugates of the present invention which induce protective antibodies against *S. flexneri* infection are administered to a mammal subject, preferably a human, in an amount sufficient to prevent or attenuate the severity, extent of duration of the infection by *S. flexneri.*

Immunogenic compositions are suitable for administration to animal (and, in particular, human) patients, and thus include both human and veterinary uses. They may be used in a method of raising an immune response in a patient, comprising the step of administering the composition to the patient.

The immunogenic compositions of the present invention may be administered before a subject is exposed to a *S. flexneri* and/or after a subject is exposed to a *S. flexneri.*

Immunogenic compositions may be prepared in unit dose form. In some embodiments a unit dose may have a volume of between 0.1-1.0 mL e.g. about 0.5 mL.

The invention also provides a delivery device (e.g. syringe, nebulizer, sprayer, inhaler, dermal patch, etc.) containing an immunogenic composition of the invention e.g. containing a unit dose. This device can be used to administer the composition to a vertebrate subject.

The invention also provides a sterile container (e.g. a vial) containing an immunogenic composition of the invention e.g. containing a unit dose, or a multidoses sterile container.

The invention also provides a unit dose of an immunogenic composition of the invention.

The invention also provides a hermetically sealed container containing an immunogenic composition of the invention. Suitable containers include e.g. a vial.

Immunogenic compositions of the invention may be prepared in various forms. For example, the immunogenic compositions may be prepared as injectables, either as liquid solutions or suspensions. Solid forms suitable for solution in, or suspension in, liquid vehicles prior to injection can also be prepared (e.g. a lyophilized composition or a spray-freeze dried composition). The composition may be prepared for topical administration e.g. as an ointment, cream or powder. The composition may be prepared for oral administration e.g. as a tablet or capsule, as a spray, or as a syrup (optionally flavored). The composition may be prepared for pulmonary administration e.g. by an inhaler, using a fine powder or a spray. The composition may be prepared as a suppository. The composition may be prepared for nasal, aural or ocular administration e.g. as a spray or drops. Injectables for intramuscular administration are typical.

The pharmaceutical compositions may comprise an effective amount of an adjuvant i.e. an amount which, when administered to an individual, either in a single dose or as part of a series, is effective for enhancing the immune response. This amount can vary depending upon the health and physical condition of the individual to be treated, age, the taxonomic group of individual to be treated (e.g. non-human primate, primate, etc.), the capacity of the individual's immune system to synthesize antibodies, the degree of protection desired, the formulation of the immunogenic composition, the treating doctor's assessment of the medical situation, and other relevant factors. The amount will fall in a relatively broad range that can be determined through routine trials.

Techniques for the formulation and administration of the immunogenic composition of the present invention may be found in "Remington's Pharmaceutical Sciences" Mack Publishing Co., Easton PA.Each vaccine dose comprises a therapeutically effective amount of oligo- or polysaccharide conjugate.

A therapeutically effective dosage of one conjugate according to the present invention or of one saccharide of general formula (I) refers to that amount of the compound that results in an at least a partial immunization against a disease. Toxicity and therapeutic efficacy of such compounds can be determined by standard pharmaceutical, pharmacological, and toxicological procedures in cell cultures or experimental animals. The dose ratio between toxic and therapeutic effect is the therapeutic index. The actual amount of the composition administered will be dependent on the subject being treated, on the subject's weight, the severity of the affliction, the manner of administration and the judgement of the prescribing physician.

Such amount will vary depending on the capacity of the subject to synthesize antibodies against the oligo- or polysaccharide, the degree of protection desired, the particular oligo- or polysaccharide conjugate selected and its mode of administration, among other factors. An appropriate effective amount can be readily determined by one oskilled in the art. A therapeutically effective amount may vary in a wide range that can be determined through routine trials.

More particularly the oligo- or polysaccharide conjugate of the invention will be administered in a therapeutically effective amount that comprises from 0.1 µg to 100 µg, notably from 0.5 µg to 50 µg of oligo- or polysaccharide, preferably 1 µg to 10 µg. An optimal amount for a particular vaccine can be ascertained by methods known from the skilled in the art, in particular standard studies involving measuring the anti-*S*. *flexneri* antibody titers in subjects, more accurately protective antibody titers.

Methods of administering the immunogenic compositions of the invention are well known from the skilled in the art. Briefly, the immunogenic compositions of the invention may be administered in single or multiple doses. The inventors have found that the administration of a single dose of the immunogenic compositions of the invention may be sufficient. Alternatively, one unit dose followed by a second unit dose may be effective. Typically, the second (or third, fourth, fifth etc.) unit dose is identical to the first unit dose. The second unit dose may be administered at any suitable time after the first unit dose, in particular after 1, 2 or 3 months. In particular, following an initial administration, subjects may receive one or two booster injections at about four week intervals. For infants less than 12 months of age, two doses at not less than two month intervals can be administered, the first dose not being administered before 2 months of age. The immunogenic composition of the invention may include one or more adjuvants. However, the use of unadjuvanted compositions is also envisaged, for example, it may be advantageous to omit adjuvants in order to reduce potential toxicity. Accordingly, immunogenic compositions that do not contain any adjuvant or that do not contain any aluminium salt adjuvant are envisaged.

Adjuvants generally combined with glycoconjugate vaccines allow to strengthen the antibody response and hence the B response. Adjuvants can be added directly to the vaccine compositions or can be administered separately, either concurrently with or shortly after, administration of the vaccine.

Adjuvants are well known from the person skilled in the art. Reference is for instance made to Current Opinion in Immunology 2020, 65:97-101. Classically recognized examples of adjuvants include:
- mineral-containing compositions, including calcium salts and aluminium salts (or mixtures thereof). Calcium salts include calcium phosphate. Aluminium salts include hydroxides, phosphates, sulfates, etc., with the salts taking any suitable form (e.g. gel, crystalline, amorphous, etc.). Adsorption to these salts is preferred. The mineral containing compositions may also be formulated as a particle of metal salt. The adjuvants known as aluminium hydroxide and aluminium phosphate may be also used. The invention can use any of the "hydroxide" or "phosphate" adjuvants that are in general used as adjuvants. The adjuvants known as "aluminium hydroxide" are typically aluminium oxyhydroxide salts, which are usually at least partially crystalline. The adjuvants known as "aluminium phosphate" are typically aluminium hydroxyphosphates, often also containing a small amount of sulfate (i. e. aluminium hydroxyphosphate sulfate). They may be obtained by precipitation, and the reaction conditions and concentrations during precipitation influence the degree of substitution of phosphate for hydroxyl in the salt. Mixtures of both an aluminium hydroxide and an aluminium phosphate can be employed in the formulation according to the present invention;

- saponins, which are a heterologous group of sterol glycosides and triterpenoid glycosides that are found in the bark, leaves, stems, roots and even flowers of a wide range of plant species. Saponins from the bark of the Quillaia saponaria, Molina tree have been widely studied as adjuvants. Saponins can also be commercially obtained from Smilax ornata (sarsaprilla), Gypsophilla paniculata (brides veil), and Saponaria oficianalis (soap root). Saponin adjuvant formulations include purified formulations, such as QS21, as well as lipid formulations, such as ISCOMs. Saponin compositions have been purified using HPLC and RP-HPLC. Specific purified fractions using these techniques have been identified, including QS7, QS 17, QS 18, QS2 1, QH-A, QH-B and QH-C. Saponin formulations may also comprise a sterol, such as cholesterol. Combinations of saponins and cholesterols can be used to form unique particles called immunostimulating complexes (ISCOMs). ISCOMs generally include a phospholipid such as phosphatidylethanolamine or phosphatidylcholine. Any known saponin can be used in ISCOMs. Preferably, the ISCOM includes one or more of QuilA, QHA & QHC;
- microparticles (i.e. a particle of 100 nm to 150 pm in diameter, more preferably 200 nm to 30 pm in diameter, or 500 nm to 10 pm in diameter) formed from materials that are biodegradable and non-toxic. Such non-toxic and biodegradable materials include, but are not restricted to poly(α-hydroxy acid), polyhydroxybutyric acid, polyorthoester, polyanhydride, polycaprolactone;
- CD1d ligands, such as an α-glycosylceramide, phytosphingosine-containing α-glycosylceramides, OCH, KRN7000 [(2S,3S,4R)-1-O-(α-D-galactopyranosyl)-2-(N-hexacosanoylamino)-1,3,4-octadecanetriol], CRONY- 101, 3"-sulfo-galactosyl-ceramide;
- immunostimulatory oligonucleotides, such CpG motif containing ones (a dinucleotide sequence containing an unmethylated cytosine residue linked by a phosphate bond to a guanosine residue), or Cpl motif containing ones (a dinucleotide sequence containing cytosine linked to inosine), or a double-stranded RNA, or an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded;
- compounds containing lipids linked to a phosphate-containing acyclic backbone, such as the TLR4 antagonist E5564;
- oil emulsions (e.g. Freund's adjuvant), in particular for diagnostic uses.

In particular, such adjuvants may be chosen from aluminium salts (aluminium hydroxide, aluminium phosphate), oil-in-water emulsion formulations with or without specific stimulating agents such as TLR agonists, muramyl peptides, saponin adjuvants, cytokines, detoxified mutants of bacterial toxins such as the cholera toxin, the pertussis toxin, or the *E*. *coli* heatlabile toxin.

The immunogenic composition of the invention may be administered with other immunogens or immunoregulatory agents, for example, immunoglobulins, cytokines, lymphokines and chemokines.

Immunogenic compositions are preferably in aqueous form, particularly at the point of administration, but they can also be presented in non-aqueous liquid forms or in dried forms e.g. as gelatin capsules, or as lyophilisates, etc.

Immunogenic compositions may include one or more preservatives, such as thiomersal or 2-phenoxyethanol. Mercury-free compositions are preferred, and preservative-free vaccines can be prepared.

Immunogenic compositions may include a physiological salt, such as a sodium salt e.g. to control tonicity. Sodium chloride (NaCl) is typical and may be present at between 1 and 20 mg/ml. Other salts that may be present include potassium chloride, potassium dihydrogen phosphate, disodium phosphate dehydrate, magnesium chloride, calcium chloride, etc.

Immunogenic compositions can have an osmolality of between 200 mOsm/kg and 400 mOsm/kg.

Immunogenic compositions may include compounds (with or without an insoluble metal salt) in plain water (e.g. w.f.i.), but will usually include one or more buffers. Typical buffers include: a phosphate buffer; a Tris buffer; a borate buffer; a succinate buffer; a histidine buffer (particularly with an aluminium hydroxide adjuvant); or a citrate buffer. Buffer salts will typically be included in the 5-20 mM range.

Immunogenic compositions typically have a pH between 5.0 and 9.5 e.g. between 6.0 and 8.0.

Immunogenic compositions are preferably sterile and gluten free.

Typically, the immunogenic compositions are prepared as injectables either as liquid solutions or suspensions; or as solid forms suitable for solution or suspension in a liquid vehicle prior to injection. The preparation may be emulsified or encapsulated in liposomes for enhanced adjuvant effect. In this respect, reference could be made to International Application WO 2010/136947.

Once formulated, the immunogenic compositions may be administered parenterally, by injection, either subcutaneous, intramuscular or intradermal.

Typically, the immunogenic compositions of the invention may be administered intramuscularly, e.g. by intramuscular administration to the high or the upper arm. Alternative formulations suitable for other mode of administration include oral and intranasal formulations.

In another aspect, the invention concerns a conjugate or an immunogenic composition as defined above for use in vaccination.

Protection and deprotection techniques (i.e. protecting group introduction and cleavage) are for instance described by P. G. M. Wuts and T. W. Greene (Greene's Protective Groups in Organic Synthesis, Fourth Edition; Wiley-Interscience, 2006; or Greene's Protective Groups in Organic Synthesis, fifth Edition; Wiley-Interscience, 2014, by P. Wuts, DOI: 10.1002/9781118905074). Reference is also made to "Recent Advances Toward Robust N-Protecting Groups for Glucosamine as Required for Glycosylation Strategies", Mohamed Ramadan El Sayed Aly and El Sayed H. El Ashry, Advances in Carbohydrate Chemistry and Biochemistry, Volume 73, p. 117-224 (2016).

### Definitions

The following terms and expressions contained herein are defined as follows:
As used herein, the term "alkyl" refers to a straight-chain, or branched alkyl group having 1 to 6 carbon atoms, such as methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, hexyl, etc. The alkyl moiety of alkyl-containing groups, such as aralkyl or O-alkyl groups, has the same meaning as alkyl defined above. Lower alkyl groups, which are preferred, are alkyl groups as defined above which contain 1 to 4 carbons. A designation such as "C1-C4 alkyl" refers to an alkyl radical containing from 1 to 4 carbon atoms.

As used herein, the term "aryl" refers to a substituted or unsubstituted, mono- or bicyclic hydrocarbon aromatic ring system having 6 to 10 ring carbon atoms. Examples include phenyl and naphthyl. Preferred aryl groups include unsubstituted or substituted phenyl and naphthyl groups. Included within the definition of "aryl" are fused ring systems, including, for example, ring systems in which an aromatic ring is fused to a cycloalkyl ring. Examples of such fused ring systems include, for example, indane, indene, and tetrahydronaphthalene.

As used herein, the term "heteroaryl" refers to an aromatic group containing 5 to 10 ring carbon atoms in which one or more ring carbon atoms are replaced by at least one hetero atom such as -O-, -N-, or -S-. Examples of heteroaryl groups include pyrrolyl, furanyl, thienyl, pirazolyl, imidazolyl, thiazolyl, isothiazolyl, isoxazolyl, oxazolyl, oxathiolyl, oxadiazolyl, triazolyl, oxatriazolyl, furazanyl, tetrazolyl, pyridyl, pyrazinyl, pyrimidinyl, pyridazinyl, triazinyl, indolyl, isoindolyl, indazolyl, benzofuranyl, isobenzofuranyl, purinyl, quinazolinyl, quinolyl, isoquinolyl, benzoimidazolyl, benzothiazolyl, benzothiophenyl, thianaphthenyl, benzoxazolyl, benzisoxazolyl, cinnolinyl, phthalazinyl, naphthyridinyl, and quinoxalinyl. Included within the definition of "heteroaryl" are fused ring systems, including, for example, ring systems in which an aromatic ring is fused to a heterocycloalkyl ring. Examples of such fused ring systems include, for example, phthalamide, phthalic anhydride, indoline, isoindoline, tetrahydroisoquinoline, chroman, isochroman, chromene, and isochromene.

As used herein, the term "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

In another aspect, the present invention is directed to pharmaceutically acceptable salts of the compounds described above. As used herein, "pharmaceutically acceptable salts" includes salts of compounds of the present invention derived from the combination of such compounds with non-toxic acid.

Acid addition salts include inorganic acids such as hydrochloric, hydrobromic, hydroiodic, sulfuric, nitric and phosphoric acid, as well as organic acids such as acetic, citric, propionic, tartaric, glutamic, salicylic, oxalic, methanesulfonic, *para*-toluenesulfonic, succinic, and benzoic acid, and related inorganic and organic acids.

In addition to pharmaceutically-acceptable salts, other salts are included in the invention. They may serve as intermediates in the purification of the compounds, in the preparation of other salts, or in the identification and characterization of the compounds or intermediates.

The pharmaceutically acceptable salts of compounds of the present invention can also exist as various solvates, such as with water, methanol, ethanol, dimethylformamide, ethyl acetate and the like. Mixtures of such solvates can also be prepared. The source of such solvate can be from the solvent of crystallization, inherent in the solvent of preparation or crystallization, or adventitious to such solvent. Such solvates are within the scope of the present invention.

It is recognized that compounds of the present invention may exist in various stereoisomeric forms. As such, the compounds of the present invention include both diastereomers and enantiomers. The compounds are normally prepared as racemates and can conveniently be used as such, but individual enantiomers can be isolated or synthesized by conventional techniques if so desired. Such racemates and individual enantiomers and mixtures thereof form part of the present invention.

It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; Wilen, S.H. Stereochemistry of Organic Compounds; Wiley: New York, 1994, and Jacques, J, et al. Enantiomers, Racemates, and Resolutions; Wiley: New York, 1981, each incorporated by reference herein in their entireties.

As used herein, the term "oligosaccharide" more particularly refers to a saccharide containing from 2 to 10 monosaccharides (simple sugars).

As used herein, the term "polysaccharide" more particularly refers to a saccharide containing more than 10 monosaccharides (simple sugars).

As used herein, a range of values in the form "x-y" or "x to y", or "x through y", include integers x, y, and the integers there between. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

As used herein, the term "donor" more particularly refers to a mono-, oligo- or polysaccharide bearing a leaving group at the anomeric position.

As used herein, the term "acceptor" more particularly refers to a mono-, oligo- or polysaccharide having at least a free hydroxyl group, in general other than the anomeric hydroxyl, preferably at least the free hydroxyl group corresponding to the elongation site of the growing chain.

By "divalent C₁-C₁₂ alkyl, C₂-C₁₂ alkenyl or C₂-C₁₂ alkynyl chain" is in particular meant a C₁-C₁₂ alkane diyl, C₂-C₁₂ alkene diyl or C₂-C₁₂ alkyne diyl chain, respectively.

### FIGURES

**Figure 1** displays immunization assays according to example 20, with:

| | | | | |
|---|---|---|---|---|
| NEW 1 | - | SF1b-TT5 | - | [AB_{Ac}C(E)D]₁₆-TT |
| NEW 2 | - | SF1b-TT10 | - | [(AB_{Ac}C(E)D)₂]_{21.5}-TT |
| NEW 3 | - | SF1b-TT15 | - | [(AB_{Ac}C(E)D)₃]_{23.5}-TT |

| | | | | |
|---|---|---|---|---|
| Wherein TT is tetanus toxoid. | | | | |

**Figure 2** displays immunization assays according to example 20, with:

| | | | | |
|---|---|---|---|---|
| NEW 6 | - | SF2aSF1b-TT10 | - | [(AB(E)CD)(AB_{Ac}C(E)D)]₂₁-TT |
| NEW 7 | - | SF3aSF1b-TT10 | - | [((E)AB_{Ac}CD)(AB_{Ac}C(E)D]₁₉-TT |
| NEW 8 | - | SF3aSF2a-TT10 | - | [((E)AB_{Ac}CD)(AB(E)CD]_{18.5}-TT |
| NEW 9 | - | SF3aSF2aSF2a-TT15 | - | [(AB(E)CD)(AB_{Ac}C(E)D)₂]₂₅-TT |

| | | | | |
|---|---|---|---|---|
| Wherein TT is tetanus toxoid. | | | | |

### EXAMPLES

### Example 1: Synthesis of donor A

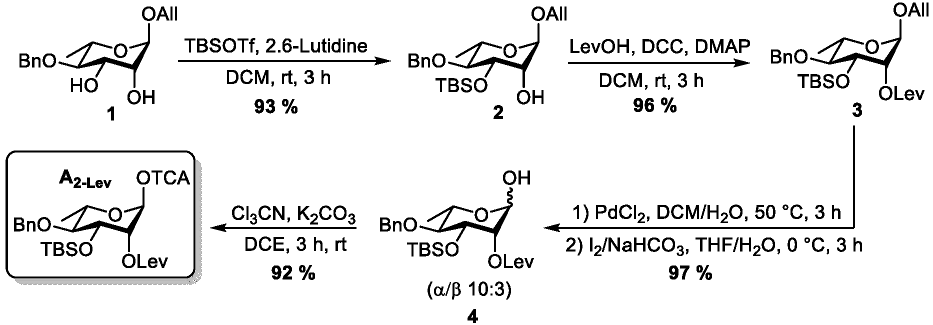

### Allyl 4-O-Benzyl-3-O-tert-butyldimethylsilyl-α-L-rhamnopyranoside (2).

To a solution of diol 1 (5 g, 16.9 mmol, 1.0 equiv.) in anhydrous DCM (170 mL) stirred at -20 °C under Ar, were added 2,6-lutidine (4.0 mL, 34.0 mmol, 2.0 equiv.) and TBSOTf (5.8 mL, 25.5 mmol, 1.5 equiv.). The mixture was stirred from -20 °C to 0 °C for 1.5 h. Then, MeOH was added and the mixture was stirred for 15 min and a precipitate appeared. The suspension was filtered through a pad of Celite, washed with a generous amount of MeOH and the filtrate was concentrated under reduced pressure. The crude product was purified by flash chromatography (cHex/EtOAc 95:5 to 8:2) to give the desired alcohol 2 (6.5 g, 93%) as a yellow oil.

**¹H NMR (400 MHz, CDCl₃) δ (ppm):** 7.36-7.29 (m, 5H, C*H*-Ar), 5.94 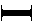 5.86 (m, 1H, H-2_{All}), 5.29 (ddd, *J =* 17.1, 3.7, 1.6 Hz, 1H, H-3a_{All}), 5.20 (ddd, *J* = 10.4, 3.2, 1.2 Hz, 1H, H-3b_{All}), 4.86 (d, *J* = 11.2 Hz, 1H, C*H*HPh), 4.85 (d, *J*_{1,2} = 1.4 Hz, 1H, H-1), 4.61 (d, *J* = 11.1 Hz, 1H, CHHPh), 4.18 (ddt, *J* = 13.2, 5.1, 1.6 Hz, 1H, H-1a_{All}), 4.04 (dd, *J* = 9.0, 3.5 Hz, 1H, H-3), 4.00 (ddt, *J* = 13.2, 6.1, 1.4 Hz, 1H, H-1b_{All}), 3.84 (dd, *J* = 3.6, 1.5 Hz, 1H, H-2), 3.78-3.75 (m, 1H, H-5), 3.37 (t, *J* = 9.3 Hz, 1H, H-4), 2.64 (br s, 1H, OH), 1.29 (d, *J =* 6.4 Hz, 3H, H-6), 0.97 (s, 9H, C(C*H*₃)₃), 0.16 (s, 3H, C*H*₃Si), 0.15 (s, 3H, C*H*₃Si).

**Allyl 4-*O*-Benzyl-3-*O*-*tert*-butyldimethylsilyl-2-*O*-levulinoyl-*α*-L-rhamnopyranoside (3).** Alcohol 2 (3.0 g, 7.3 mmol, 1.0 equiv.) was dissolved in anhydrous DCM (152 mL). Levulinic acid (1.35 mL, 13.2 mmol, 1.8 equiv.), DCC (2.3 g, 11.0 mmol, 1.5 equiv.) and DMAP (1.8 g, 14.7 mmol, 2.0 equiv.) were added to the solution and the mixture was stirred at rt for 1 h under Ar. Then, the reaction mixture was filtered through a pad of Celite and the filtrate was concentrated under reduced pressure. The obtained residue was dissolved in DCM (100 mL), transferred into a separtory funnel and washed with 5% aq. NaHCO₃ (50 mL) and brine (50 mL). The combined organic phases were dried over anhydrous Na₂SO₄. The solution was evaporated under pressure. Then, the crude was dissolved in EtOAc (200 mL) and the mixture was stored in cold room (4 °C). After 15 h, the solution was filtered over a pad of Celite and the filtrate was concentrated to dryness under vacuo. The residue was purified by flash chromatography (Tol/EtOAc 9:1 to 75:25) to give compound **3** (3.5 g, 96 %) as a yellow oil.

**¹H NMR (400 MHz, CDCl₃) δ (ppm):** 7.38-7.30 (m, 5H, C*H*-Ar), 5.94 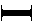 5.84 (m, 1H, H-2_{All}), 5.28 (ddd, *J=* 17.2, 3.7, 1.6 Hz, 1H, H-3a_{All}), 5.19 (ddd, *J =* 10.4, 3.3, 1.2 Hz, 1H, H-3b_{All}), 5.09 (dd, *J=* 3.7, 1.8 Hz, 1H, H-2), 4.91 (d, *J =* 11.2 Hz, 1H, C*H*HPh), 4.70 (d, *J*_{1,2} = 1.6 Hz, 1H, H-1), 4.62 (d, *J =* 11.1 Hz, 1H, CHHPh), 4.18 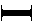 4.12 (m, 2H, H-1a_{All}, H-3), 3.96 (ddt, *J =* 13.1, 5.9, 1.4 Hz, 1H, H-1b_{All}), 3.78-3.72 (m, 1H, H-5), 3.36 (t, J= 9.2 Hz, 1H, H-4), 2.80-2.68 (m, 4H, 2 x (CH)_{2Lev}), 2.22 (s, 3H, (C*H*_{3Lev})), 1.29 (d, *J=* 6.5 Hz, 3H, H-6), 0.92 (s, 9H, C(C*H*₃)₃), 0.11 (s, 3H, C*H*₃Si), 0.12 (s, 3H, C*H*₃Si).

### 4-O-Benzyl-3-O-tert-butyldimethylsilyl-2-O-levulinoyl-α/β-L-rhamnopyranose (4).

To a solution of compound **3** (11 g, 21.6 mmol, 1.0 equiv.) in DCM/H₂O (3:1, 216 mL), stirred at rt, was added PdCl₂ (60%, 255.3 mg, 0.86 mmol, 0.04 equiv.). The mixture was stirred for 4 h at 50 °C. Then, a solution of I₂ (11 g, 43.2 mmol, 2.0 equiv.) and NaHCO₃ (14.5 g, 172.8 mmol, 8.0 equiv.) in THF/H₂O (5:1, 238 mL) was poured at 0 °C and the mixture was stirred for another 3 h at rt. The reaction mixture was quenched with 10% aq. Na₂S₂O₃ (20 mL), then filtered over a pad of Celite and cotton to remove the excess of Pd. THF was removed under reduced pressure and DCM (100 mL) was added. The aqueous layer was extracted with DCM (2 x 50 mL) and the combined organic phases were washed with satd aq. NaHCO₃ (80 mL) and brine (80 mL), dried over anhydrous Na₂SO₄ and concentrated to dryness under vacuo. The residue was quickly filtered through a pad of silica (Tol/EtOAc 9:1 to 7:3) to give the desired hemiacetal **4** (9.8 g, 97 %, α/β 10:3) as a yellow oil.

**¹H NMR (400 MHz, CDCl₃) δ (ppm):** 7.38-7.30 (m, 5H, C*H*-Ar), 5.94 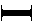 5.84 (m, 1H, H-2_{All}), 5.28 (ddd, *J =* 17.2, 3.7, 1.6 Hz, 1H, H-3a_{All}), 5.19 (ddd, *J =* 10.4, 3.3, 1.2 Hz, 1H, H-3b_{All}), 5.09 (dd, *J =* 3.7, 1.8 Hz, 1H, H-2), 4.91 (d, *J =* 11.2 Hz, 1H, C*H*HPh), 4.70 (d, *J*_{1,2} = 1.6 Hz, 1H, H-1), 4.62 (d, *J =* 11.1 Hz, 1H, CHHPh), 4.18-4.12 (m, 2H, H-1a_{All}, H-3), 3.96 (ddt, *J =* 13.1, 5.9, 1.4 Hz, 1H, H-1b_{All}), 3.78-3.72 (m, 1H, H-5), 3.36 (t, *J =* 9.2 Hz, 1H, H-4), 2.80-2.68 (m, 4H, 2 x C*H*_{2Lev}), 2.22 (s, 3H, C*H*_{3Lev}), 1.29 (d, *J =* 6.5 Hz, 3H, H-6), 0.92 (s, 9H, C(C*H*₃)₃), 0.12 (s, 3H, C*H*₃Si), 0.11 (s, 3H, C*H*₃Si).

### 4-O-Benzyl-3-O-tert-butyldimethylsilyl-2-O-levulinoyl-α/β-L-rhamnopyranosyl trichloroacetamidate [A (2-Lev)].

To a solution of hemiacetal (22.2 g, 47.8 mmol, 1.0 equiv.) in anhydrous DCE (248 mL), stirred at rt under Ar, were successively added Cl₃CCN (19.2 mL, 191.1 mmol, 4.0 equiv.) and K₂CO₃ (19.8 mg, 143.3 mmol, 3.0 equiv.). The mixture was stirred for 3 h at rt. Salts were filtered off over a pad of Celite and the filtrate was concentrated under reduced pressure. The residue was purified by flash chromatography (Tol/EtOAc 9:1 to 75:25 + 1% Et₃N) to give donor **A (2-Lev)** (27 g, 92 %, α/β 95:5) as a yellow solid.

**¹H NMR (400 MHz, CDCl₃) δ (ppm):** 8.66 (s, 1H, NH), 7.37-7.30 (m, 5H, C*H*-Ar), 6.14 (d, *J*_{1,2} = 1.9 Hz, 1H, H-1), 5.25 (dd, *J =* 3.4, 2.1 Hz, 1H, H-2), 4.93 (d, *J =* 11.0 Hz, 1H, *CH*HPh), 4.64 (d, *J =* 11.2 Hz, 1H, CH*H*Ph), 4.22 (dd, *J =* 9.0, 3.4 Hz, 1H, H-3), 3.97-3.90 (m, 1H, H-5), 3.47 (t, *J =* 9.4 Hz, 1H, H-4), 2.82-2.74 (m, 4H, 2 x C*H*_{2Lev}), 2.23 (s, 3H, C*H*_{3Lev}), 1.32 (d, *J =* 6.1 Hz, 3H, H-6), 0.92 (s, 9H, C(C*H*₃)₃), 0.13 (s, 3H, C*H*₃Si), 0.12 (s, 3H, C*H*₃Si).

### Example 2: Synthesis of disaccharide acceptor BC

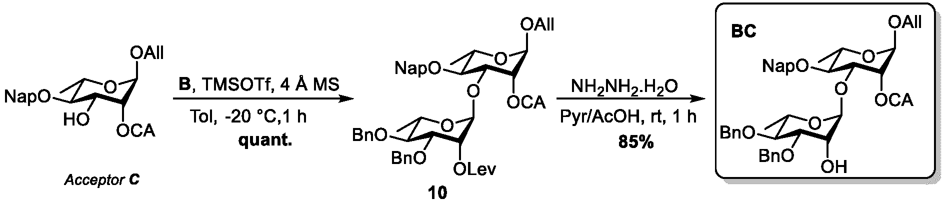

### Allyl 3,4-di-O-benzyl-2-O-levulinoyl-α-L-rhamnopyranosyl-(1→3)-2-O-chloroacetyl-4-O-(2-naphtylmethyl)-α-L-rhamnopyranoside (10).

To a solution of acceptor **C** (9.4 g, 22.4 mmol, 1.25 equiv.) in anhydrous Toluene (112 mL), stirred under Ar, were successively added donor **B** (10.4 g, 17.8 mmol, 1.0 equiv.) and activated 4Å MS (4.6 g). The suspension was stirred for 25 min at rt then cooled to - 20 °C and stirred for another 5 min. TMSOTf (966 µL, 5.3 mmol, 0.3 equiv.) was slowly added at this temperature. The mixture was then stirred for 1 h at - 20°C under Ar. After that time, the mixture was neutralized by slow addition of Et₃N (868 µL, 6.23 mmol, 0.35 equiv.). Then, the suspension was filtered over a pad of Celite. The filtrate was concentrated under reduced pressure and directly purified by flash chromatography (Tol/EtOAc 95:5 to 75:25) to give the desired disaccharide **10** (15 g, quant.) as a yellow oil.

**¹H NMR (400 MHz, CDCl₃) δ (ppm):** 7.89 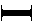 7.82 (m, 4H, C*H*-Ar), 7.52 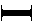 7.46 (m, 3H, *CH-*Ar), 7.387.25 (m, 10H, C*H*-Ar), 5.94 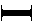 5.85 (m, 1H, H-2_{All}), 5.44 (dd, *J =* 3.4, 1.8 Hz, 1H, H-2B), 5.30 (ddd, *J* = 17.4, 3.7, 1.4 Hz, 1H, H-3a_{All}), 5.25 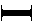 5.21 (m, 2H, H-2C, H-3b_{All}), 5.09 (d, *J*_{1,2}= 1.8 Hz, 1H, H-1B), 4.97 (d, *J* = 11.1 Hz, 1H, C*H*HPh), 4.92 (d, *J* = 11.1 Hz, 1H, CHHPh), 4.81 (d, *J*_{1,2} = 1.6 Hz, 1H, H-1C), 4.76 (d, *J =* 11.3 Hz, 1H, C*H*HPh), 4.66 (d, *J* = 11.3 Hz, 1H, C*H*HPh), 4.58 (d, *J =* 11.6 Hz, 1H, C*H*HPh), 4.46 (d, *J =* 11.5 Hz, 1H, C*H*HPh), 4.25 (dd, *J* = 9.5, 3.1 Hz, 1H, H-3C), 4.16 (ddt, *J =* 13.0, 5.8, 1.5 Hz, 1H, H-1a_{All}), 4.12 (s, 2H, C*H*_{2CA}), 4.00 (ddt, *J =* 13.0, 6.1, 1.1 Hz, 1H, H-1b_{All}), 3.89 (dd, *J=* 9.4, 3.3 Hz, 1H, H-3B), 3.85 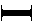 3.75 (m, 2H, H-5B,H-5C), 3.52 (t, *J* = 10.2 Hz, 1H, H-4C), 3.42 (t, *J=* 10.2 Hz, 1H, H-4B), 2.64 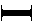 2.55 (m, 4H, 2 x (C*H*)_{2Lev}), 2.11 (s, 3H, (C*H*_{3Lev})), 1.33 (d, *J=* 6.3 Hz, 3H, H-6C), 1.30 (d, *J=* 6.2 Hz, 3H, H-6B).

### Allyl 3,4-di-O-benzyl-α-L-rhamnopyranosyl-(1→3)-2-O-chloroacetyl-4-O-(2-naphtylmethyl)-α-L-rhamnopyranoside (acceptor BC).

To a solution of compound 10 (15.9 g, 18.8 mmol, 1.0 equiv.) in a previously prepared mixture of anhydrous pyr (256 mL) and AcOH (188 mL), stirred at rt under Ar, was added hydrazine monohydrate (50-60%, 2.3 mL, 26.3 mmol, 1.4 equiv.). The suspension was stirred for 15 min at 0 °C then for 1 h at rt under Ar. Following addition of water (200 mL) and DCM (200 mL), the two layers were separated and the aq. one was extracted twice with DCM (50 mL). The combined organic phases were washed with 5% aq. citric acid (150 mL) and brine (150 mL), dried over Na₂SO₄ and concentrated to dryness. The crude was directly purified by flash chromatography (Tol/EtOAc 95:5 to 85:15) to give the desired acceptor **BC** (12 g, 85%) as a translucent oil.

**¹H NMR (400 MHz, CDCl₃) δ (ppm):** 7.87 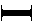 7.79 (m, 4H, C*H*-Ar), 7.53 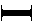 7.43 (m, 3H, *CH-*Ar), 7.377.18 (m, 10H, C*H*-Ar), 5.95-5.85 (m, 1H, H-2_{All}), 5.31 (ddd, *J =* 17.2, 3.6, 1.5 Hz, 1H, H-3a_{All}), 5.25-5.22 (m, 2H, H-2C, H-3b_{All} ), 5.13 (d, *J*_{1,2} = 1.5 Hz, 1H, H-1B), 4.89 (d, *J =* 11.2 Hz, 1H, C*H*HPh), 4.86 (d, *J =* 11.2 Hz, 1H, CHHPh), 4.82 (d, *J*_{1,2} = 1.8 Hz, 1H, H-1C), 4.78 (d, *J =* 11.3 Hz, 1H, C*H*HPh), 4.67 (d, *J =* 11.1 Hz, 1H, C*H*HPh), 4.58 (d, *J =* 11.6 Hz, 1H, C*H*HPh), 4.50 (d, *J =* 11.6 Hz, 1H, C*H*HPh), 4.22 (dd, *J =* 9.5, 3.4 Hz, 1H, H-3C), 4.18 (ddt, *J =* 12.5, 5.1, 1.3 Hz, 1H, H-1a_{All}), 4.14 (s, 2H, C*H*_{2CA}), 4.00 (ddt, *J =* 12.7, 6.0, 1.3 Hz, 1H, H-1b_{All}), 3.92 (dd, *J =* 3.2, 1.7 Hz, 1H, H-2B), 3.86-3.79 (m, 1H, H-5C), 3.78-3.79 (m, 2H, H-3B, H-5B), 3.51 (t, *J =* 9.6 Hz, 1H, H-4C), 3.46 (t, *J =* 9.3 Hz, 1H, H-4B), 1.34 (d, *J=* 6.2 Hz, 3H, H-6C), 1.29 (d, *J =* 6.1 Hz, 3H, H-6B).

### Example 3: Synthesis of trisaccharide donor (Lev)ABC

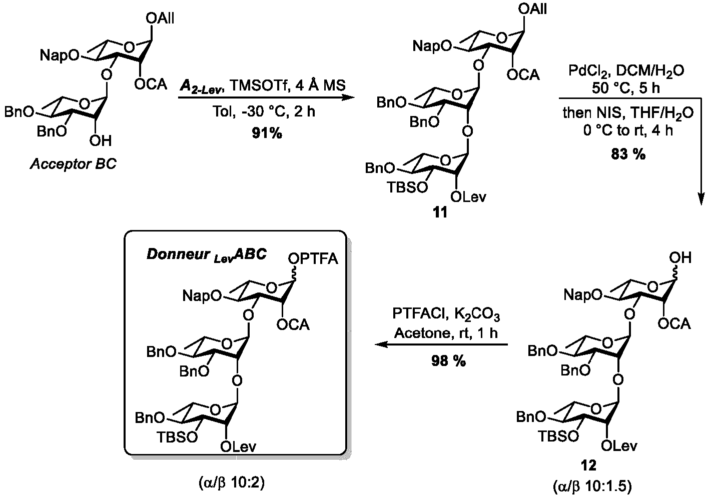

### Allyl 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranoside (11).

Acceptor **BC** (702 mg, 0.939 mmol, 1.0 equiv.) and donor **A (2-Lev)** (689 mg, 1.13 mmol, 1.2 equiv.) were dissolved in anhydrous Toluene (9.4 mL) and activated 4Å MS (1.4 g) was added. The suspension was stirred under Ar for 25 min at rt, then cooled to 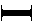 30 °C and stirred for another 5 min. TMSOTf (9 µL, 0.05 mmol, 0.05 equiv.) was slowly added at this temperature. The mixture was then stirred for 1 h at 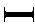 30 °C under Ar. After that time, the mixture was neutralized by slow addition of Et₃N (8 µL, 0.06 mmol, 0.05 equiv.) and the suspension was filtered over a pad of Celite. The filtrate was concentrated under reduced pressure and directly purified by flash chromatography (cHex/EtOAc 90:10 to 75:25) to give the desired trisaccharide **11** (1 g, 91%) as a cristalline solid.

**¹H NMR (400 MHz, CDCl₃) δ (ppm):** 7.86 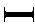 7.75 (m, 4H, C*H*-Ar), 7.52 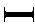 7.42 (m, 3H, *CH-*Ar), 7.38-7.18 (m, 15H, C*H*-Ar), 5.94-5.84 (m, 1H, H-2_{All}), 5.29 (ddd, *J =* 17.2, 3.7, 1.7 Hz, 1H, H-3a_{All}), 5.25 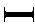 5.20 (m, 3H, H-2A, H-2C, H-3b_{All}), 5.06 (d, *J*_{1,2}= 1.8 Hz, 1H, H-1B), 4.90-4.87 (m, 4H, H-1A, C*H*HPh), 4.81 (d, *J =* 1.6 Hz, 1H, H-1C), 4.67 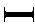 4.51 (m, 5H, H-1C, C*H*HPh), 4.19 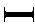 4.13 (m, 5H, H-3C, H-3A, H-1a_{All}, C*H*_{2CA}), 4.00 (ddt, *J =* 12.8, 6.0, 1.2 Hz, 1H, H-1b_{All}), 3.97 (t, *J* = 2.5 Hz, 1H, H-2B), 3.85 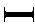 3.75 (m, 3H, H-5A, H-3B, H-5C), 3.70 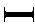 3.63 (m, 1H, H-5B), 3.47 (t, *J* = 9.4 Hz, 1H, H-4C), 3.46 (t, *J* = 9.4 Hz, 1H, H-4B), 3.34 (t, *J* = 9.4 Hz, 1H, H-4A), 2.77 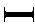 2.67 (m, 4H, 2 x C*H*_{2Lev}), 2.21 (s, 3H, C*H*_{3Lev}), 1.24 (d, *J* = 5.5 Hz, 6H, H-6C*, H-6B*), 1.16 (d, *J* = 6.2 Hz, 3H, H-6B), 0.92 (s, 9H, C(C*H*₃)₃), 0.14 (s, 3H, C*H*₃Si), 0.11 (s, 3H, C*H*₃Si).

### 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α/β-L-rhamnopyranose (12).

To a solution of compound **11** (7.0 g, 5.88 mmol, 1.0 equiv.) in DCM/H₂O (3:1, 58 mL) stirred at rt, was added PdCl₂ (60%, 122 mg, 0.41 mmol, 0.07 equiv.). The mixture was stirred for 5 h at 50 °C. Then, a solution of NIS (1.6 g, 7.06 mmol, 1.2 equiv.) in THF/H₂O (4:1, 39 mL) was poured at 0 °C and the mixture was stirred for another 3 h from 0 °C to rt. After that time, the reaction mixture was quenched with 10% aq. Na₂S₂O₃ (35 mL), then filtered over a pad of Celite and cotton to remove the excess of Pd. THF and DCM were removed under reduced pressure and EtOAc (100 mL) was added. The aqueous layer was extracted with EtOAc (2 x 20 mL) and the combined organic phases were washed with satd aq. NaHCO₃ (100 mL) and brine (100 mL), dried over anhydrous Na₂SO₄ and concentrated to dryness under vacuo. The residue was purified by flash chromatography (Tol/EtOAc 9:1 to 75:25) to give the desired hemiacetal **12** (5.6 g, 83 %, α/β 10:1.5) along with the unexpected propyl derivative **13** (290 mg, 4 %) both as brown oil.

**¹H NMR (400 MHz, CDCl₃) δ (ppm):** 7.86 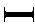 7.75 (m, 4H, C*H*-Ar), 7.51 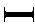 7.42 (m, 3H, *CH-*Ar), 7.38-7.15 (m, 15H, C*H*-Ar), 5.25 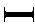 5.23 (m, 2H, H-2A, H-2C), 5.18 (d, *J*_{1,2}= 1.6 Hz, 1H, H-1C), 5.07 (d, *J*_{1,2}= 1.7 Hz, 1H, H-1B), 4.91-4.87 (m, 4H, H-1A, C*H*HPh), 4.67 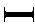 4.51 (m, 5H, C*H*HPh), 4.22 (dd, *J =* 9.4, 3.3 Hz, 1H, H-3C), 4.16 (dd, *J =* 9.1, 3.3 Hz, 1H, H-3A), 4.12 (s, 2H, C*H*_{2CA}), 4.03 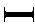 3.96 (m, 2H, H-5C, H-2B), 3.83 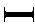 3.79 (m, 2H, H-3B, H-5A), 3.71 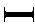 3.64 (m, 1H, H-5B), 3.47 (t, *J =* 9.4 Hz, 1H, H-4C), 3.46 (t, *J =* 9.4 Hz, 1H, H-4B), 3.34 (t, *J =* 9.4 Hz, 1H, H-4A), 2.77 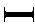 2.67 (m, 4H, 2 x C*H*_{2Lev}), 2.21 (s, 3H, C*H*_{3Lev}), 1.24 (d, *J =* 6.5 Hz, 3H, H-6B), 1.22 (d, *J =* 6.5 Hz, 3H, H-6C), 1.15 (d, *J =* 6.4 Hz, 3H, H-6A), 0.93 (s, 9H, C(C*H*₃)₃), 0.14 (s, 3H, C*H*₃Si), 0.11 (s, 3H, C*H*₃Si).

### 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α/β-L-rhamnopyranosyl (N-phenyl)trifluoroacetimidate (Donor _{Lev}ABC).

To a solution of hemiacetal **12** (5.6 g, 4.88 mmol, 1.0 equiv.) in anhydrous acetone (98 mL) stirred at rt under Ar, were successively added PTFACl (1.2 mL, 7.33 mmol, 1.5 equiv.) and K₂CO₃ (1.3 g, 9.77 mmol, 2.0 equiv.). The mixture was stirred for 1 h at rt. After that time, the reaction mixture was filtered over a pad of Celite to remove salts and the filtrate was concentrated to dryness under vacuo. The residue was quickly purified in a fritted funnel (Tol/EtOAc 9:1 to 7:3 + 1% TEA) to give the desired donor **_{Lev}ABC** (6.4 g, 98 %, α/β 10:2) as a yellow solid.

**¹H NMR (400 MHz, CDCl₃)** δ **(ppm):** 7.86-7.76 (m, 4H, C*H*-Ar), 7.52-7.43 (m, 3H, *CH-*Ar), 7.387.11 (m, 18H, C*H*-Ar), 6.85 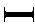 6.83 (m, 2H, C*H*-Ar), 6.17 (br s, 1H, H-1C), 5.39 (t, *J* = 2.3 Hz, 1H, H-2C), 5.27 (dd, *J =* 3.3, 2.0 Hz, 1H, H-2A), 5.11 (d, *J*_{1,2} = 1.4 Hz, 1H, H-1B), 4.93-4.88 (m, 4H, H-1A, C*H*HPh), 4.69-4.55 (m, 5H, C*H*HPh), 4.22 (dd, *J =* 9.6, 3.0 Hz, 1H, H-3C), 4.17 (dd, *J =* 9.3, 3.3 Hz, 1H, H-3A), 4.12 (s, 2H, C*H*_{2CA}), 3.98 (t, *J =* 2.4 Hz, 1H, H-2B), 3.94 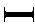 3.77 (m, 3H, H-5C, H-5A, H-2B), 3.72 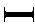 3.56 (m, 1H, H-5B), 3.56 (t, *J =* 9.6 Hz, 1H, H-4C), 3.48 (t, *J =* 9.3 Hz, 1H, H-4B), 3.35 (t, *J =* 9.3 Hz, 1H, H-4A), 2.78 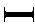 2.69 (m, 4H, 2 x C*H*_{2Lev}), 2.21 (s, 3H, C*H*_{3Lev}), 1.28 (d, *J =* 6.3 Hz, 3H, H-6B), 1.26 (d, *J =* 6.2 Hz, 3H, H-6C), 1.15 (d, *J =* 6.2 Hz, 3H, H-6A), 0.94 (s, 9H, C(C*H*₃)₃), 0.15 (s, 3H, C*H*₃Si), 0.12 (s, 3H, C*H*₃Si).

### Example 4: Synthesis of tetrasaccharide (Lev)ABCD

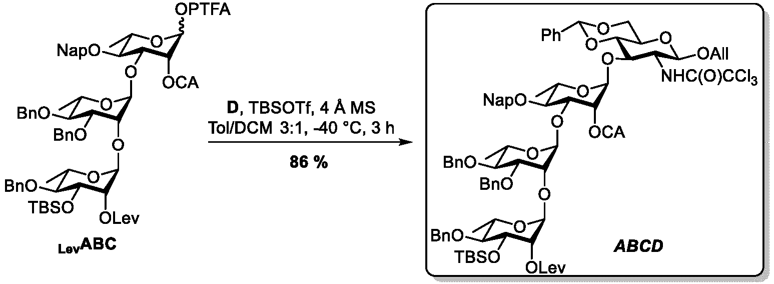

### Allyl 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranoside (_{Lev}ABCD).

To a solution of donor **_{Lev}ABC** (6.4 g, 4.8 mmol, 1.0 equiv.) in anhydrous toluene/DCM (3:1, 96 mL), stirred at rt under Ar, were successively added glucose acceptor **D** (2.6 g, 5.7 mmol, 1.2 equiv.) and activated 4Å MS (1.3 g). The suspension was stirred for 15 min at rt, then cooled to - 40 °C and stirred for another 10 min. TBSOTf(110 µL, 0.48 mmol, 0.1 equiv.) was slowly added at this temperature. The mixture was then stirred for 2 h from 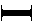 40 °C to 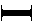 20 °C under Ar. After that time, the mixture was neutralized by slow addition of Et₃N (94 µL, 0.68 mmol, 0.14 equiv.) and stirred for 10 min at 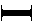 20 °C. Then, the suspension was filtered over a pad of Celite. The filtrate was concentrated under reduced pressure and directly purified by flash chromatography (Tol/EtOAc 95:5 to 8:2) to give the desired tetrasaccharide **_{Lev}ABCD** (6.6 g, 86 %) as a whitish cristalline solid.

**¹H NMR (400 MHz, CDCl₃) δ (ppm):** 7.85 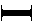 7.82 (m, 3H, C*H*-Ar), 7.68 (s, 1H, C*H*-Ar), 7.52 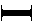 7.45 (m, 4H, C*H*-Ar), 7.39 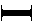 7.17 (m, 19H, C*H*-Ar), 7.03 (d, *J =* 7.4 Hz, 1H, N*H*_{TCA}), 5.94 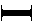 5.84 (m, 1H, H-2_{All}), 5.56 (s, 1H, H-7D), 5.30 (ddd, *J =* 17.2, 3.5, 1.5 Hz, 1H, H-3a_{All}), 5.25 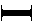 5.21 (m, 3H, H-2C, H-2A, H-3b_{All}), 5.09 (d, *J*_{1,2} = 8.3 Hz, 1H, H-1D), 5.05 (d, *J*_{1,2} = 1.7 Hz, 1H, H-1B), 4.93 (d, *J*_{1,2} = 1.9 Hz, 1H, H-1A), 4.90 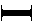 4.87 (m, 3H, C*H*HPh, H-1C), 4.81 (d, *J=* 11.5 Hz, 1H, C*H*HPh), 4.65 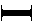 4.52 (m, 6H, H-3D, C*H*HPh), 4.41-4.34 (m, 2H, H-6aD, H-1a_{All}), 4.16 (dd, *J =* 9.2, 3.2 Hz, 1H, H-3A), 4.14 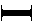 4.08 (m, 2H, H-3C, H-1b_{All}), 4.03 (s, 2H, C*H*_{2CA}), 4.01 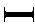 3.96 (m, 2H, H-5C, H-2B), 3.83 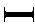 3.75 (m, 3H, H-5A, H-3B, H-6bD), 3.70 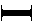 3.56 (m, 3H, H-5B, H-4D, H-5D), 3.50 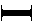 3.43 (m, 2H, H-2D, H-4B), 3.33 (td, *J =* 9.4, 1.8 Hz, 1H, H-4C*, H-4A*), 2.77 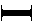 2.67 (m, 4H, 2 x C*H*_{2Lev}), 2.21 (s, 3H, C*H*_{3Lev}), 1.24 (d, *J =* 6.1 Hz, 3H, H-6B), 1.09 (d, *J =* 6.2 Hz, 3H, H-6A), 0.92 (s, 9H, C(C*H*₃)₃), 0.71 (d, *J =* 6.1 Hz, 3H, H-6C), 0.15 (s, 3H, C*H*₃Si), 0.12 (s, 3H, C*H*₃Si).

### Example 5: Synthesis of acceptor (OH)ABCD from (Lev)ABCD

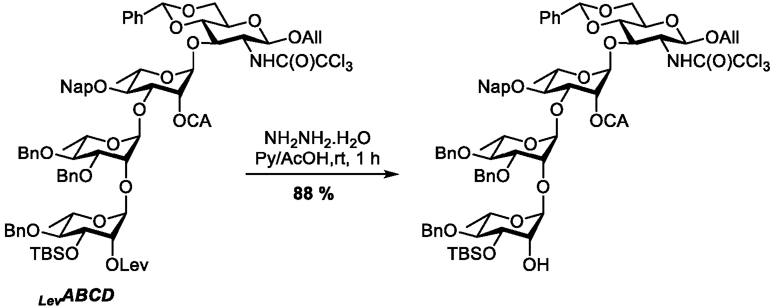

### Allyl 4-O-benzyl-3-O-tert-butyldimethylsilyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranoside (Acceptor _{Lev}ABCD).

To a solution of tetrasaccharide **_{Lev}ABCD** (0.4 g, 0.25 mmol, 1.0 equiv.) in a mixture of anhydrous pyridine (3 mL) and AcOH (3 mL) stirred at rt under Ar, was added hydrazine monohydrate (50-60%, 27 µL, 0.30 mmol, 1.2 equiv.). The suspension was stirred for 15 min at 0 °C then for 1 h at rt under Ar. Following addition of water (15 mL) and DCM (20 mL), the two layers were separated and the aq. one was extracted twice with DCM (8 mL). The combined organic phases were washed with 5% aq. citric acid (100 mL) and brine (100 mL), dried over Na₂SO₄ and concentrated to dryness. The crude was directly purified by flash chromatography (Tol/EtOAc 95:5 to 85:15) to give the desired acceptor **_{Lev}ABCD** (329 mg, 88%) as a whitish cristalline solid.

**¹H NMR (400 MHz, CDCl₃) δ (ppm):** 7.85 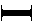 7.82 (m, 3H, C*H*-Ar), 7.68 (s, 1H, C*H*-Ar), 7.52 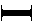 7.45 (m, 5H, C*H*-Ar), 7.38 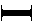 7.16 (m, 19H, C*H*-Ar), 7.02 (d, *J =* 7.4 Hz, 1H, N*H*_{TCA}), 5.93 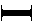 5.83 (m, 1H, H-2_{All}), 5.56 (s, 1H, H-7D), 5.30 (ddd, *J =* 17.2, 3.5, 1.3 Hz, 1H, H-3a_{All}), 5.24 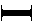 5.22 (m, 2H, H-2A, H-3b_{All}), 5.09 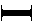 5.07 (m, 2H, H-1D, H-1B), 5.04 (d, *J*_{1,2} = 1.6 Hz, 1H, H-1A), 4.87 (br s, 1H, H-1C), 4.86 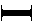 4.80 (m, 3H, C*H*HPh), 4.60 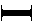 4.45 (m, 6H, H-3D, C*H*HPh), 4.41-4.33 (m, 2H, H-6aD, H-1a_{All}), 4.13-4.08 (m, 2H, H-3C, H-1b_{All}), 4.06-3.95 (m, 5H, H-3B, C*H*_{2CA}, H-2A, H-5C) 3.93 (dd, *J =* 3.4, 1.8 Hz, 1H, H-2B), 3.87 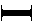 3.73 (m, 3H, H-6bD, H-3A, H-5B), 3.69 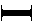 3.55 (m, 3H, H-5A, H-4D, H-5D), 3.51 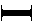 3.33 (m, 4H, H-2D, H-4A, H-4B, H-4C), 1.23 (d, *J =* 6.2 Hz, 3H, H-6A), 1.10 (d, *J =* 6.2 Hz, 3H, H-6B), 0.98 (s, 9H, C(C*H*₃)₃), 0.71 (d, *J =* 6.2 Hz, 3H, H-6C), 0.15 (s, 6H, C*H*₃Si).

### Example 6: Synthesis of donor (lev)ABCD

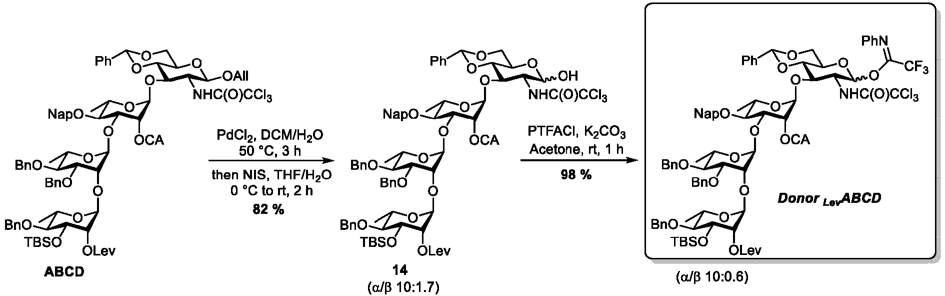

### 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-α/β-D-glucopyranose (14).

To a solution of tetrasaccharide **_{Lev}ABCD** (0.5 g, 0.31 mmol, 1.0 equiv.) in DCM/H₂O (3:1, 3 mL) stirred at rt, was added PdCl₂ (60%, 6.5 mg, 0.022 mmol, 0.07 equiv.). The mixture was stirred for 3 h at 50 °C. Then a solution of NIS (85 mg, 0.38 mmol, 1.2 equiv.) in THF/H₂O (4:1, 3 mL) was poured at 0 °C and the mixture was stirred for 2 h from 0 °C to rt. After that time, the reaction mixture was quenched with 10% aq. Na₂S₂O₃ (8 mL), then filtered over a pad of Celite and cotton to remove the excess of Pd. THF and DCM were removed under reduced pressure and EtOAc (50 mL) was added. The aqueous layer was extracted with EtOAc (2 x 100 mL) and the combined organic phases were washed with satd aq. NaHCO₃ (50 mL) and brine (50 mL), dried over anhydrous Na₂SO₄ and concentrated to dryness under vacuo. The residue was purified by flash chromatography (Tol/EtOAc 95:5 to 8:2) to give the desired compoun **14** (401 mg, 82 %, α/β 10:1.7) as a whitish cristalline solid.

**¹H NMR (400 MHz, CDCl₃)** δ **(ppm):** 7.85-7.80 (m, 3H, C*H*-Ar), 7.68 (s, 1H, C*H*-Ar), 7.51 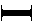 7.45 (m, 4H, C*H*-Ar), 7.37 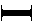 7.16 (m, 19H, C*H*-Ar), 6.90 (d, *J=* 9.5 Hz, 1H, N*H*_{TCA}), 5.56 (s, 1H, H-7D), 5.27 (t, *J* = 4.2 Hz, 1H, H-1D), 5.23 (dd, *J* = 3.3, 1.9 Hz, 1H, H-2A), 5.17 (dd,*J* = 3.3, 2.2 Hz, 1H, H-2C), 5.06 (d, *J*_{1,2} = 1.5 Hz, 1H, H-1B), 4.93 (d, *J*_{1,2} = 1.9 Hz, 1H, H-1C), 4.90 (d, *J*_{1,2} = 1.8 Hz, 1H, H-1A), 4.89 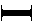 4.79 (m, 3H, C*H*HPh), 4.64 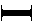 4.51 (m, 3H, CHHPh), 4.32 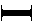 4.26 (m, 2H, H-6aD, H-2D), 4.18 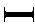 4.08 (m, 4H, H-3C, H-5D, H-3A, H-3D), 4.02 (s, 2H, C*H*_{2CA}), 3.99 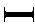 3.93 (m, 2H, H-5C, H-2B), 3.83 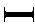 3.65 (m, 5H, H-6bD, H-5A, H-3B, H-5B, H-4D), 3.44 (t, *J =* 9.5 Hz, 1H, H-4B), 3.36 (t, *J =* 9.4 Hz, 1H, H-4C), 3.28 (t, *J =* 9.2 Hz, 1H, H-4A), 3.18 (dd, *J =* 3.8, 1.5 Hz, 1H, OH), 2.76 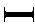 2.65 (m, 4H, 2 x C*H*_{2Lev}), 2.20 (s, 3H, C*H*_{3Lev}), 1.26 (d, *J =* 6.2 Hz, 3H, H-6B), 1.08 (d, *J =* 6.3 Hz, 3H, H-6A), 0.92 (s, 9H, C(C*H*₃)₃), 0.70 (d, *J =* 6.2 Hz, 3H, H-6C), 0.13 (s, 3H, C*H*₃Si), 0.10 (s, 3H, C*H*₃Si).

### 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-α/β-D-glucopyranosyl (N-phenyl)trifluoroacetimidate (Donor _{AZMB}ABCD).

To a solution of hemiacetal **14** (389 mg, 0.25 mmol, 1.0 equiv.) in anhydrous acetone (5 mL) stirred at rt under Ar, were successively added PTFACl (0.06 mL, 0.38 mmol, 1.5 equiv.) and K₂CO₃ (69 mg, 0.50 mmol, 2.0 equiv.). The mixture was stirred for 1 h at rt. After that time, the reaction mixture was filtered over a pad of Celite to remove salts and the filtrate was concentrated to dryness under vacuo. The residue was quickly purified in a fritted funnel (cHex/EtOAc 9:1 to 7:3 + 1% TEA) to give the desired donor **_{Lev}ABCD** (424 mg, 98 %, α/β 10:0.6) as a cristalline solid.

**¹H NMR (400 MHz, CDCl₃) δ (ppm):** 7.85 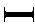 7.82 (m, 3H, C*H*-Ar), 7.69 (s, 1H, C*H*-Ar), 7.52 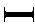 7.44 (m, 5H, C*H*-Ar), 7.38 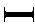 7.13 (m, 20H, C*H*-Ar), 6.80 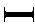 6.75 (m, 3H, C*H*-Ar), 6.89 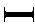 6.84 (m, 3H, C*H*-Ar), 6.39 (br s, 1H, H-1D), 5.60 (s, 1H, H-7D), 5.23 (dd, *J =* 3.5, 2.0 Hz, 1H, H-2A), 5.17 (dd, *J* = 3.2, 2.1 Hz, 1H, H-2C), 5.04 (d, *J*_{1,2} = 1.6 Hz, 1H, H-1B), 5.00 (d, *J*_{1,2} = 1.7 Hz, 1H, H-1C), 4.91 (d, *J*_{1,2} = 1.8 Hz, 1H, H-1A), 4.89 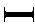 4.82 (m, 3H, C*H*HPh), 4.64 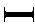 4.47 (m, 6H, H-2D, CHHPh), 4.37 (dd, *J =* 10.4, 4.8 Hz, 1H, H-6aD), 4.18 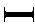 4.12 (m, 3H, H-3A, H-3D, H-3C), 4.04 (s, 2H, C*H*_{2CA}), 4.03-3.94 (m, 3H, H-5D, H-5C, H-2B), 3.84 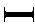 3.75 (m, 4H, H-4D, H-6bD, H-5A, H-3B), 3.71 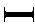 3.64 (m, 1H, H-5B), 3.45 (t, *J =* 9.3 Hz, 1H, H-4B), 3.36 (t, *J=* 9.3 Hz, 1H, H-4C), 3.32 (t, *J =* 9.3 Hz, 1H, H-4A), 2.77 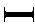 2.66 (m, 4H, 2 x C*H*_{2Lev}), 2.20 (s, 3H, C*H*_{3Lev}), 1.24 (d, *J =* 6.1 Hz, 3H, H-6B), 1.09 (d, *J =* 6.2 Hz, 3H, H-6A), 0.92 (s, 9H, C(C*H*₃)₃), 0.74 (d, *J =* 6.1 Hz, 3H, H-6C), 0.14 (s, 3H, C*H*₃Si), 0.11 (s, 3H, C*H*₃Si).

### Example 7: Synthesis of octasaccharide [(lev)ABCD]₂

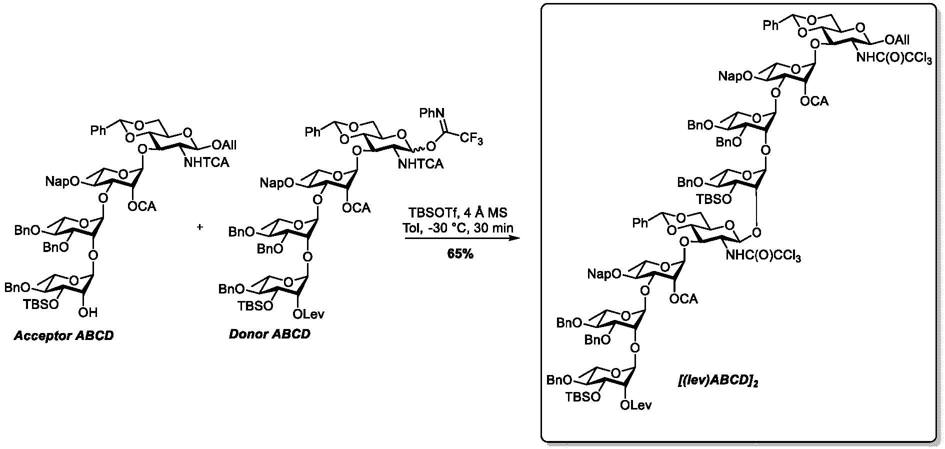

### Allyl 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranosyl-(1→2)-4-O-benzyl-3-O-tert-butyldimethylsilyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranoside [(_{Lev}ABCD)₂].

To a solution of acceptor **_{Lev}ABCD** (329 mg, 0.22mmol, 1.0 equiv.) in anhydrous toluene (6 mL) stirred at rt under Ar, were successively added donor **_{Lev}ABCD** (417 mg, 0.24 mmol, 1.1 equiv.) and activated 4Å MS (0.21 g). The suspension was stirred for 15 min at rt under Ar atm, then cooled to - 35 °C and stirred for another 10 min. TBSOTf (7 µL, 0.03 mmol, 0.13 equiv.) was slowly added at this temperature. The mixture was then stirred for 30 min from - 35 °C to - 30 °C under Ar. After that time, the mixture was neutralized by slow addition of Et₃N (4 µL, 0.031 mmol, 0.14 equiv.) and stirred for 10 min at - 30 °C. Then, the suspension was filtered over a pad of Celite. The filtrate was concentrated under reduced pressure and directly purified by flash chromatography (cHex/EtOAc 95:5 to 8:2) to give the desired octasaccharide **[(_{Lev}ABCD)₂]** (0.43 g, 65 %) as a whitish cristalline solid.

TLC: R*_{f}* 0.7 (Tol/EtOAc 8:2)

### Example 8: Bzl group removal

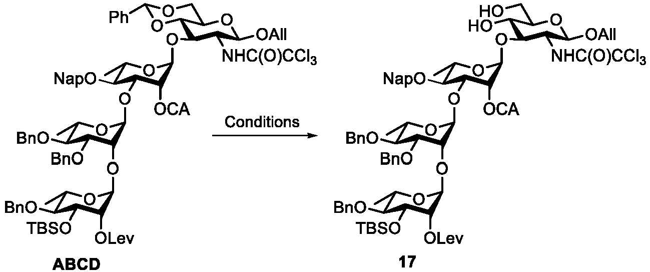

### Allyl 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-2-deoxy-2-trichloroacetamido-β-D-glucopyranoside (17).

To a solution of tetrasaccharide **_{Lev}ABCD** (4.0 g, 2.515 mmol, 1.0 equiv.) in anhydrous DCM (50 mL) stirred at rt under Ar, were successively added CSA (584 mg, 2.515 mmol, 1.0 equiv.) and 1.2-dithioethane (422 µL, 5.030 mmol, 2.0 equiv.). The suspension was stirred overnight at rt under Ar. Following addition of DCM (200 mL), the solution was poured into a separatory funnel, washed with saturated NaHCO₃ (2 x 70 mL), with water (70 mL) and brine (70 mL), dried over Na₂SO₄ and concentrated to dryness. The crude was directly purified by flash chromatography (Tol/EtOAc 95:5 to 8:2) to give compound 17 (3.21 g, 85 %), as a cristalline solid.

**¹H NMR (400 MHz, CDCl₃) δ (ppm):** 7.87 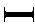 7.82 (m, 3H, C*H*-Ar), 7.73 (s, 1H, C*H*-Ar), 7.52 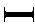 7.47 (m, 2H, C*H*-Ar), 7.42 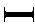 7.14 (m, 19H, C*H*-Ar), 6.93 (d, *J =* 7.6 Hz, 1H, N*H*_{TCA}), 5.93 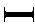 5.83 (m, 1H, H-2_{All}), 5.30 (ddd, *J =* 17.3, 3.6, 1.5 Hz, 1H, H-3a_{All}), 5.25 (dd, *J=* 3.4, 2.3 Hz, 1H, H-2C), 5.23 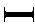 5.20 (m, 2H, H-2A, H-3b_{All}), 5.01 (d, *J*_{1,2} = 1.7 Hz, 1H, H-1B), 4.92 (d, *J*_{1,2} = 8.2 Hz, 1H, H-1D), 4.90 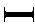 4.86 (m, 6H, H-1A, H-1C, C*H*HPh), 4.64 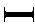 4.57 (m, 4H, C*H*HPh), 4.52 (d, *J=* 11.9 Hz, 1H, C*H*HPh), 4.35 (ddd, *J =* 13.0, 5.3, 1.5 Hz, 1H, H-1a_{All}), 4.16-4.06 (m, 6H, H-3A, H-3C, H-1b_{All}, C*H*_{2CA}, H-3D), 4.01 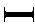 3.93 (m, 3H, H-5C, H-6aD, H-2B), 3.85 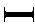 3.76 (m, 3H, H-6bD, H-5A, H-3B), 3.64 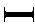 3.57 (m, 1H, H-5B), 3.54 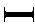 3.39 (m, 5H, H-2D, H-4D, H-4C, H-4B, H-5D), 3.35 (t, *J=* 9.3 Hz, 1H, H-4A), 2.77 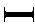 2.67 (m, 4H, 2 x C*H*_{2Lev}), 2.21 (s, 3H, C*H*_{3Lev}), 1.26 (d, *J=* 6.2 Hz, 3H, H-6C), 1.20 (d, *J=* 6.3 Hz, 3H, H-6A), 1.18 (d, *J* = 6.1 Hz, 3H, H-6B), 0.94 (s, 9H, C(C*H*₃)₃), 0.15 (s, 3H, C*H*₃Si), 0.12 (s, 3H, C*H*₃Si).

### Example 9: Nap group removal

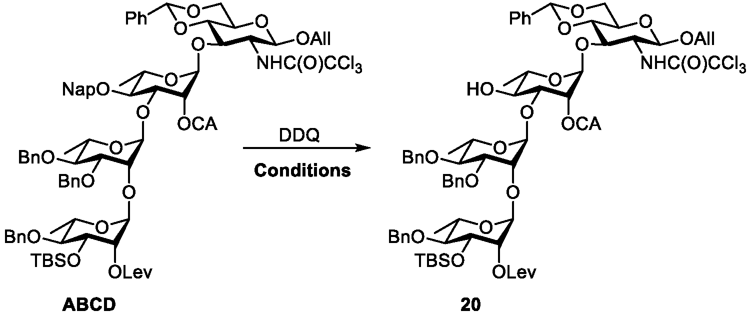

### Allyl 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-α-L-rhamnopyranosyl-(1→3)-4,6-O-benzylidene-2-deoxy-2-trichloroacetamido-β-D-glucopyranoside (20).

To a solution of tetrasaccharide **_{Lev}ABCD** (2.0 g, 1.257 mmol, 1.0 equiv.) in DCM/H₂O (20:1, 52.5 mL) stirred at rt under Ar, were successively added portionwise DDQ (856 mg, 3.772 mmol, 3.0 equiv.) and β-pinene (1.8 mL, 12.57 mmol, 10 equiv.). The suspension was stirred overnight at rt under Ar. After that time, solid NaHCO₃ was added and the reaction mixture was stirred for another 15 min. Following addition of DCM (200 mL), the solution was poured into a separatory funnel, washed with saturated NaHCO₃ (3 x 50 mL), with water (50 mL) and brine (50 mL), dried over Na₂SO₄ and concentrated to dryness. The crude was directly purified by flash chromatography (Tol/EtOAc 95:5 to 8:2) to give compound **20** (1.42 g, 78 %), as a cristalline yellow solid.

**¹H NMR (400 MHz, CDCl₃) δ (ppm):** 7.51 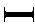 7.48 (m, 2H, C*H*-Ar), 7.38 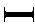 7.28 (m, 20H, C*H-*Ar), 7.04 (d, *J =* 7.8 Hz, 1H, N*H*_{TCA}), 5.92 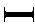 5.82 (m, 1H, H-2_{All}), 5.55 (s, 1H, H-7D), 5.29 (ddd, *J =* 17.1, 4.5, 1.6 Hz, 1H, H-3a_{All}), 5.24 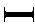 5.18 (m, 3H, H-2C, H-2A, H-3b_{All}), 5.08 (d, *J*_{1,2} = 8.1 Hz, 1H, H-1D), 5.03 (d, *J*_{1,2} = 3.4 Hz, 1H, H-1B), 4.91 (d, *J =* 11.0 Hz, 1H, C*H*HPh), 4.88 (d, *J*_{1,2}= 2.1 Hz, 1H, H-1A), 4.83 (d, *J*_{1,2}= 1.2 Hz, 1H, H-1C), 4.71 (d, *J=* 11.4 Hz, 1H, C*H*HPh), 4.65 (s, 2H, C*H*HPh), 4.59 (d, *J=* 11.9 Hz, 1H, C*H*HPh), 4.57 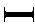 4.49 (m, 2H, H-3D, C*H*HPh), 4.41-4.33 (m, 2H, H-6aD, H-1a_{All}), 4.18 (dd, *J =* 9.1, 3.4 Hz, 1H, H-3A), 4.10 (ddd, *J =* 12.8, 6.3, 1.3 Hz, 1H, H-1b_{All}), 3.98 (s, 2H, C*H*_{2CA}), 3.91 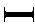 3.78 (m, 5H, H-5A, H-3C, H-2B, H-5C, H-6bD), 3.72 (dd, *J* = 7.0, 2.8 Hz, 1H, H-3B), 3.65 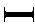 3.55 (m, 3H, H-5B, H-4D, H-5D), 3.49 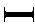 3.34 (m, 4H, H-2D, H-4C, H-4B H-4A), 2.80 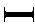 2.66 (m, 4H, 2 x C*H*_{2Lev}), 2.23 (s, 3H, C*H*_{3Lev}), 1.24 (d, *J =* 6.2 Hz, 3H, H-6A), 1.22 (d, *J =* 6.2 Hz, 3H, H-6B), 0.93 (s, 9H, C(C*H*₃)₃), 0.68 (d, *J =* 6.1 Hz, 3H, H-6C), 0.15 (s, 3H, C*H*₃Si), 0.13 (s, 3H, C*H*₃Si).

### Example 10: TBS group removal

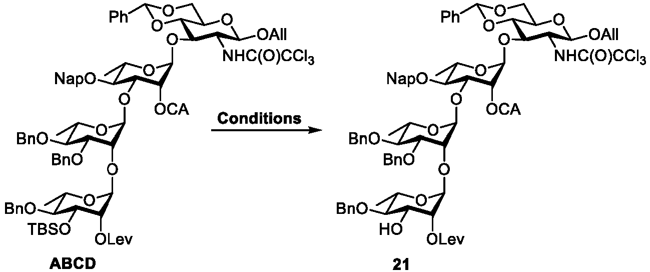

### Allyl 4-O-benzyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranoside (21).

To a solution of tetrasaccharide **_{Lev}ABCD** (2.0 g, 1.257 mmol, 1.0 equiv.) in anhydrous THF (82 mL) stirred at rt under Ar, was added TREAT.HF (12.3 mL, 75.446 mmol, 60.0 equiv.). The suspension was stirred for 2 days at 30 °C under Ar. Then the reaction mixture was gradually cooled to rt. Following addition of EtOAc (200 mL), the solution was poured into a separatory funnel, washed with a cooled saturated NaHCO₃ (3 x 70 mL) while checking the pH of the solution, with water (70 mL) and brine (70 mL), dried over Na₂SO₄ and concentrated to dryness. The crude was directly purified by flash chromatography (Tol/EtOAc 95:5 to 8:2) to give compound 21 (1.55 g, 84 %), as a cristalline solid.

**¹H NMR (400 MHz, CDCl₃) δ (ppm):** 7.87 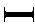 7.83 (m, 3H, C*H*-Ar), 7.70 (s, 1H, C*H*-Ar), 7.52 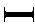 7.47 (m, 4H, C*H*-Ar), 7.39 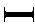 7.18 (m, 23H, C*H*-Ar), 7.10 (d, *J =* 7.0 Hz, 1H, N*H*_{TCA}), 5.94-5.84 (m, 1H, H-2_{All}), 5.58 (s, 1H, H-7D), 5.34 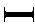 5.28 (m, 2H, H-2A, H-3a_{All}), 5.25 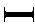 5.22 (m, 2H, H-3b_{All}, H-2C), 5.08 (d, *J*_{1,2} = 8.0 Hz, 1H, H-1D), 5.06 (d, *J*_{1,2} = 1.1 Hz, 1H, H-1B), 4.95 (d, *J*_{1,2} = 1.4 Hz, 1H, H-1A), 4.91 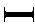 4.87 (m, 3H, C*H*HPh, H-1C), 4.83 (d, *J =* 11.5 Hz, 1H, C*H*HPh), 4.69 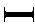 4.51 (m, 6H, H-3D, C*H*HPh), 4.42-4.34 (m, 2H, H-6aD, H-1a_{All}), 4.18-4.09 (m, 3H, H-3A, H-1b_{All}, H-3C), 4.04 (s, 2H, C*H*_{2CA}), 4.02 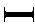 3.98 (m, 1H, H-5C), 3.96 (t, *J=* 2.4 Hz, H-2B), 3.83 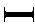 3.77 (m, 3H, H-5A, H-3B, H-6bD), 3.72 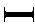 3.56 (m, 3H, H-5B, H-4D, H-5D), 3.54 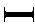 3.45 (m, 2H, H-2D, H-4B), 3.33 (td, *J* = 15.3, 9.6 Hz, 2H, H-4C, H-4A), 2.87 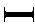 2.57 (m, 4H, 2 x C*H*_{2Lev}), 2.21 (s, 3H, C*H*_{3Lev}), 1.27 (d, *J* = 6.2 Hz, 3H, H-6B), 1.17 (d, *J=* 6.3 Hz, 3H, H-6A), 0.71 (d, *J* = 6.1 Hz, 3H, H-6C).

### Example 11: Selective benzylation at position 6_{D}

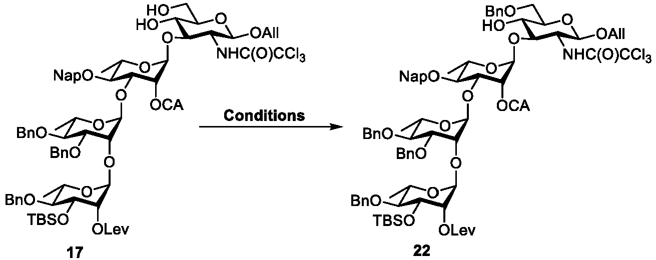

### Allyl 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-2-deoxy-2-trichloroacetamido-β-D-glucopyranoside (22).

To a solution of diol **17** (3.2 g, 2.140 mmol, 1.0 equiv.) in anhydrous ACN (214 mL) stirred at rt under Ar, were successively added Taylor's reagent (223 mg, 0.428 mmol, 0.2 equiv.), Ag₂O (992 mg, 4.280 mmol, 2.0 equiv.) and BnBr (1018 µL, 8.559 mmol, 4.0 equiv.). The suspension was stirred overnight at 60 °C under Ar. After that time, the reaction mixture was cooled to rt, and the grey suspension was filtered over Celite, rinsed with DCM (200 mL) and the solvents were evaporated under reduced pressure. The crude was directly purified by flash chromatography (Tol/EtOAc 95:5 to 75:25) to give compound **22** (2.41 g, 71 %), as a cristalline yellow solid.

**¹H NMR (400 MHz, CDCl₃) δ (ppm):** 7.88 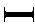 7.84 (m, 3H, C*H*-Ar), 7.76 (s, 1H, C*H*-Ar), 7.53 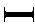 7.48 (m, 2H, C*H*-Ar), 7.45 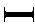 7.15 (m, 19H, C*H*-Ar), 6.94 (d, *J =* 7.7 Hz, 1H, N*H*_{TCA}), 5.94 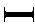 5.84 (m, 1H, H-2_{All}), 5.33 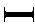 5.26 (m, 2H, H-3a_{All}, H-2C), 5.25 (dd, *J =* 3.3, 2.0 Hz, 1H, H-2A), 5.21 (ddd, *J =* 10.5, 1.6, 1.2 Hz, 1H, H-3b_{All}), 5.04 (d, *J*_{1,2} = 1.4 Hz, 1H, H-1B), 4.91 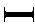 4.87 (m, 6H, H-1C, H-1A, H-1D, C*H*HPh), 4.68 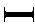 4.58 (m, 6H, C*H*HPh), 4.53 (d, *J =* 12.0 Hz, 1H, C*H*HPh), 4.38 (ddd, *J =* 12.9, 5.3, 1.5 Hz, 1H, H-1a_{All}), 4.17 (dd, *J =* 8.9, 3.3 Hz, 1H, H-3A), 4.14 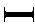 4.07 (m, 5H, H-3C, H-1b_{All}, C*H*_{2CA}, H-3D), 4.04 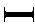 3.98 (m, 1H, H-5C), 3.96 (dd, *J* = 3.1, 2.0 Hz, 1H, H-2B), 3.88 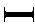 3.73 (m, 4H, H-6aD, H-5A, H-3B, H-6bD), 3.67 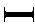 3.59 (m, 1H, H-5B), 3.58 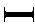 3.48 (m, 4H, H-2D, H-4D, H-5D, H-4C), 3.45 (t, *J=* 9.4 Hz, 1H, H-4B), 3.36 (t, *J=* 9.5 Hz, 1H, H-4A), 2.78 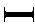 2.67 (m, 4H, 2 x C*H*_{2Lev}), 2.21 (s, 3H, C*H*_{3Lev}), 1.27 (d, *J* = 6.2 Hz, 3H, H-6C), 1.21 (d, *J=* 6.3 Hz, 3H, H-6A, H-B), 0.95 (s, 9H, C(C*H*₃)₃), 0.16 (s, 3H, C*H*₃Si), 0.14 (s, 3H, C*H*₃Si).

### Example 12: Synthesis of pentasaccharide _{Lev}ABC(E4)D 28 (1a/1b)

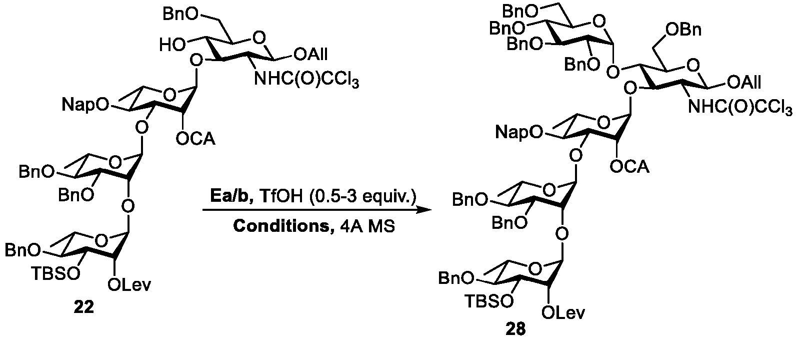

### Allyl 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-[2,3,4,6-tetra-O-benzyl-α-D-glucopyranosyl-(1→4)]-6-O-benzyl-2-deoxy-2-trichloroacetamido-β-D-glucopyranoside (28).

To a solution of acceptor **22** (2.4 g, 1.514 mmol, 1.0 equiv.) in anhydrous DCM/Et₂O (1:5, 35 mL), were added DMF (704 µL, 9.087 mmol, 6.0 equiv.) and activated ground molecular sieves (4Å, 5 g). The mixture was stirred at rt for 15 min under Ar. Then, the reaction flask was cooled to -60 °C. After 5 min, TfOH (335 µL, 3.786 mmol, 2.5 equiv.) was added. Then a solution of PTFA donor **Ea** (1.8 g, 2.575 mmol, 1.7 equiv.) in anhydrous DCM/Et₂O (1.5:4, 13 mL) was slowly added over 1 h from -60 to -50 °C. After that time, the reaction mixture was allowed to stirr under Ar overnight while being gradually warmed to rt.

The reaction was quenched with Et₃N, filtered over Celite, rinsed with DCM (50 mL) and concentrated under reduced pressure. The crude was directly purified by flash chromatography (cHex/EtOAc 95:5 to 7:3) to give compound **28** (2.48 g, 77 %), as a cristalline solid.

**¹H NMR (400 MHz, CDCl₃) δ (ppm):** 7.85-7.81 (m, 3H, C*H*-Ar), 7.74 (s, 1H, C*H*-Ar), 7.52 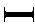 7.48 (m, 2H, C*H*-Ar), 7.42 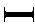 7.11 (m, 47H, C*H*-Ar), 5.98 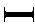 5.88 (m, 1H, H-2_{All}), 5.32 (ddd, *J =* 17.3, 3.6, 1.5 Hz, 1H, H-3a_{All}), 5.29 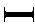 5.26 (m, 2H, H-2C, H-2A), 5.22 (ddd, *J =* 10.5, 3.5, 1.5 Hz, 1H, H-3b_{All}), 5.11 (d, *J*_{1,2} = 1.2 Hz, 1H, H-1B), 5.07 (d, *J*_{1,2} = 1.4 Hz, 1H, H-1C), 4.93 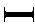 4.78 (m, 10H, H-1A, H-1E, H-1D, C*H*HPh), 4.68 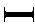 4.44 (m, 11H, C*H*HPh), 4.32 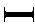 4.23 (m, 3H, H-1a_{All}, C*H*HPh, H-2D), 4.17 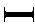 4.05 (m, 6H, H-3A, C*H*_{2CA}, H-3D, H-3C, H-1b_{All}), 4.03 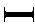 3.98 (m, 3H, H-6aD, H-6bD, H-4D), 3.96 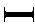 3.90 (m, 2H, H-2B, H-3E), 3.83 (dd, *J=* 9.4, 2.7 Hz, 1H, H-3B), 3.81 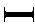 3.63 (m, 7H, H-5A, H-5E, H-5B, H-5D, H-5C, H-4E, H-6aE), 3.57 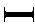 3.50 (m, 2H, H-2E, H-4C), 3.48 (t, *J=* 9.5 Hz, 1H, H-4B), 3.39 (dd, *J=* 10.9, 1.4 Hz, 1H, H-6bE), 3.31 (t, *J=* 9.3 Hz, 1H, H-4A), 2.76 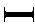 2.66 (m, 4H, 2 x C*H*_{2Lev}), 2.20 (s, 3H, C*H*_{3Lev}), 1.28 (d, *J=* 6.1 Hz, 3H, H-6B), 1.17 (d, *J=* 6.1 Hz, 3H, H-6C), 1.02 (d, *J=* 6.2 Hz, 3H, H-6A), 0.93 (s, 9H, C(C*H*₃)₃), 0.14 (s, 3H, C*H*₃Si), 0.10 (s, 3H, C*H*₃Si).

### Example 13: Synthesis of pentasaccharide _{Lev}AB(E)CD 31 (2a)

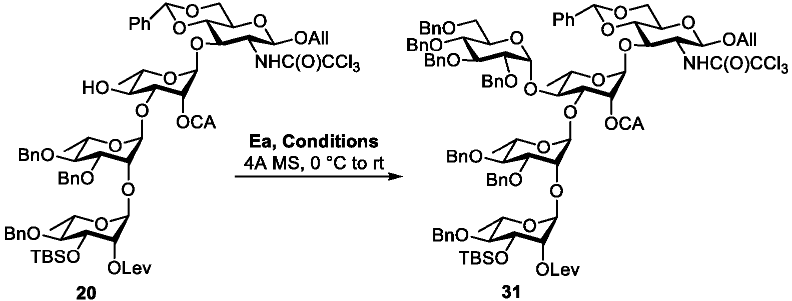

### Allyl 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-[2,3,4,6-tetra-O-benzyl-α-D-glucopyranosyl-(1→4)]-2-O-chloroacetyl-α-L-rhamnopyranosyl-(1→3)-4,6-O-benzylidene-2-deoxy-2-trichloroacetamido-β-D-glucopyranoside (31).

To a solution of acceptor **20** (3.6 g, 2.459 mmol, 1.0 equiv.) in anhydrous Toluene (74 mL), were added DMF (1333 µL, 17.220 mmol, 7.0 equiv.) and activated ground molecular sieves (4Å, 3.5 g). The mixture was stirred at rt for 15 min under Ar. Then, the reaction flask was cooled to -35 °C. After 5 min, TfOH (544 µL, 6.149 mmol, 2.5 equiv.) was added. Then a solution of PTFA donor **Ea** (3.5 g, 4.919 mmol, 2.0 equiv.) in anhydrous Toluene (49 mL) was slowly added over 1 h from -35 to -30 °C. After that time, the reaction mixture was allowed to stirr under Ar overnight while being gradually warmed to rt.

The reaction was quenched with Et₃N, filtered over Celite, rinsed with DCM (50 mL) and concentrated under reduced pressure. The crude was directly purified by flash chromatography (cHex/EtOAc 95:5 to 7:3) to give compound **31** (4.28 g, 88 %), as a cristalline yellow solid.

**¹H NMR (400 MHz, CDCl₃) δ (ppm):** 7.48 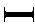 7.18 (m, 41H, C*H*-Ar), 7.04 (d, *J =* 5.8 Hz, 1H, N*H*_{TCA}), 5.94-5.84 (m, 1H, H-2_{All}), 5.56 (s, 1H, H-7D), 5.34-5.30 (m, 2H, H-3a_{All}, H-2A), 5.25 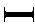 5.22 (m, 2H, H-3b_{All}, H-2C), 5.08 (d, *J*_{1D,2D} = 7.5 Hz, 1H, H-1D), 5.05 (s, 1H, H-1A), 4.96 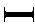 4.81 (m, 9H, H-1B, H-1E, H-1C, C*H*HPh), 4.66 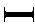 4.49 (m, 10H, C*H*HPh, H-3D), 4.41 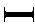 4.35 (m, 4H, H-6aD, C*H*HPh, H-3C, H-1a_{All}), 4.22 (dd, *J =* 9.2, 3.1 Hz, 1H, H-3A), 4.12 (ddd, *J=* 12.7, 1.8, 1.0 Hz, 1H, H-1b_{All}), 4.01 (s, 2H, C*H*_{2CA}), 3.97 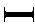 3.87 (m, 4H, H-5A, H-5C*, H-5E*, H-2B), 3.84 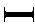 3.65 (m, 5H, H-3E, H-6bD, H-6aE, H-4E, H-3B), 3.63-3.51 (m, 3H, H-4D, H-5D, H-5B), 3.48 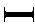 3.38 (m, 5H, H-2D, H-4C, H-4B, H-4A, H-2E), 2.73 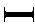 2.52 (m, 4H, 2 x C*H*_{2Lev}), 2.12 (s, 3H, C*H*_{3Lev}), 1.33 (d, *J=* 6.2 Hz, 3H, H-6A), 1.17 (d, *J=* 6.2 Hz, 3H, H-6B), 0.93 (s, 9H, C(C*H*₃)₃), 0.82 (d, *J=* 6.1 Hz, 3H, H-6C), 0.16 (s, 3H, C*H*₃Si), 0.13 (s, 3H, C*H*₃Si).

### Example 14: Synthesis of pentasaccharide _{Lev}(E)ABCD 32 (3a)

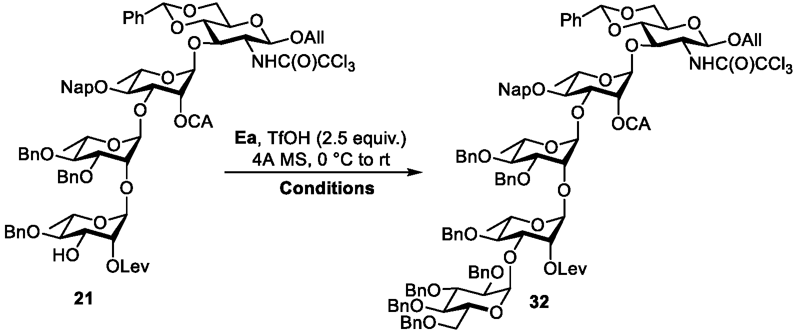

### Allyl [2,3,4,6-tetra-O-benzyl-α-D-glucopyranosyl-(1→3)]-4-O-benzyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranoside (32).

To a solution of acceptor **21** (4.7 g, 3.245 mmol, 1.0 equiv.) in anhydrous DCM/Et₂O (1:5, 68 mL), were added DMF (1759 µL, 22.712 mmol, 7.0 equiv.) and activated ground molecular sieves (4Å, 9.5 g). The mixture was stirred at rt for 15 min under Ar. Then, the reaction flask was cooled to -50 °C. After 5 min, TfOH (718 µL, 8.112 mmol, 2.5 equiv.) was added. Then a solution of PTFA donor **Ea** (3.3 g, 4.802 mmol, 1.5 equiv.) in anhydrous DCM/Et₂O (1:3, 19 mL) was slowly added over 1 h from -50 to -40 °C. After that time, the reaction mixture was allowed to stirr under Ar overnight while being gradually warmed to rt.

The reaction was quenched with Et₃N, filtered over Celite, rinsed with DCM (100 mL) and concentrated under reduced pressure. The crude was directly purified by flash chromatography (Tol/EtOAc 95:5 to 8:2) to give compound **32** (6.39 g, 98 %), as a cristalline solid.

**¹H NMR (400 MHz, CDCl₃) δ (ppm):** 7.87 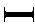 7.83 (m, 3H, C*H*-Ar), 7.69 (s, 1H, C*H*-Ar), 7.53 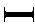 7.47 (m, 4H, C*H*-Ar), 7.41 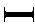 7.08 (m, 37H, C*H*-Ar), 7.05 (d, *J =* 7.2 Hz, 1H, N*H*_{TCA}), 5.94-5.84 (m, 1H, H-2_{All}), 5.57 (s, 1H, H-7D), 5.54 (t, *J=* 2.3 Hz, H-2A), 5.31 (ddd, *J=* 17.3, 3.6, 1.4 Hz, 1H, H-3a_{All}), 5.26 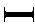 5.23 (m, 3H, H-1E, H-2C, H-3b_{All}), 5.11 (d, J = 8.2 Hz, 1H, H-1D), 5.04 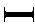 5.01 (m, 2H, H-1B, C*H*HPh), 4.98 (br s, 1H, H-1A), 4.95-4.77 (m, 7H, H-1C, C*H*HPh), 4.69-4.49 (m, 10H, H-3D, C*H*HPh), 4.43-4.33 (m, 3H, H-6aD, C*H*HPh, H-1a_{All}), 4.24 (dd, *J=* 9.5, 3.0 Hz, 1H, H-3A), 4.17 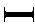 3.99 (m, 7H, H-1b_{All}, H-3E, H-3C, C*H*_{2CA}, H-5E, H-5C), 3.97 (t, *J=* 2.4 Hz, H-2B), 3.87 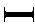 3.79 (m, 4H, H-5A, H-6bD, H-4E, H-3B), 3.70 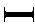 3.44 (m, 9H, H-5B, H-6aE, H-4D, H-2E, H-5D, H-6bD, H-4A, H-4B, H-2D), 3.34 (t, *J=* 9.5 Hz, 1H, H-4A), 2.58 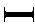 2.36 (m, 4H, 2 x C*H*_{2Lev}), 2.07 (s, 3H, C*H*_{3Lev}), 1.27 (d, *J=* 6.2 Hz, 3H, H-6B), 1.21 (d, *J=* 6.2 Hz, 3H, H-6A), 0.71 (d, *J=* 6.2 Hz, 3H, H-6C).

### Example 15: Synthesis of homooligosaccharides

### Synthesis of pentasaccharide donors (1b, 2a and 3a)

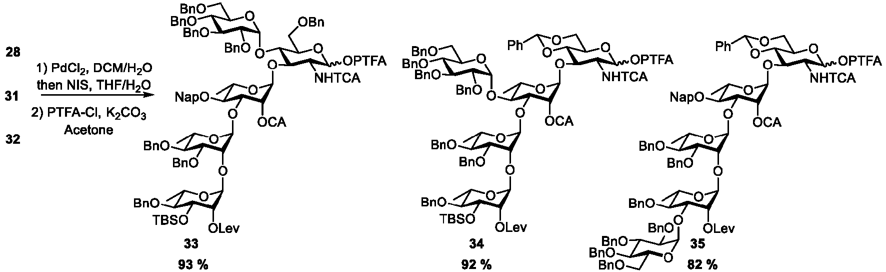

### General procedure for PTFA activation from pentasaccharides 28, 31 and 32.

To a solution of pentasaccharide (1.0 equiv.) in DCM/H₂O (3:1, 10 mL.mmol) stirred at rt, was added PdCl₂ (0.07 equiv.). The mixture was stirred for 3 h at 50 °C. Then, a solution of NIS (1.2 equiv.) in THF/H₂O (4:1, 7 mL.mmol) was poured into the previous solution at 0°C and the obtained suspension was stirred for 3h at rt. The reaction mixture was then filtered over a pad of Celite and cotton to remove excess of Pd and rinsed with DCM. The filtrate was transferred into a separatory funnel and quenched with 10 % aqueous Na₂S₂O₃, then the organic phase was washed with a saturated NaHCO₃ and brine, dried over Na₂SO₄ and concentrated to dryness. Then, to a solution of the obtained crude hemiacetal (1.0 equiv.) in anhydrous acetone (20 mL.mmol) stirred at rt under Ar, were successively added PTFACl (1.5 equiv.) and K₂CO₃ (2.0 equiv.). The suspension was stirred for 1 h at rt. After that time, the mixture was filtered over a pad of Celite to remove salts and the filtrate was concentrated under reduced pressure and purified by flash chromatography.

### 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-[2,3,4,6-tetra-O-benzyl-α-D-glucopyranosyl-(1→4)]-6-O-benzyl-2-deoxy-2-trichloroacetamido-α/β-D-glucopyranosyl (N-phenyl)trifluoroacetimidate (33).

The title compound was synthesized from pentasaccharide **28** (250 mg, 0.118 mmol, 1.0 equiv.) according to the general procedure for PTFA activation. Purification by flash chromatography (cHex/EtOAc 95:5 to 7:3) gave compound **33** (200 mg, 93 %, over 2 steps) as a cristalline solid along with traces of its corresponding oxazoline **39a.**

**¹H NMR (400 MHz, CDCl₃) δ (ppm):** 7.84-7.79 (m, 4H, C*H*-Ar), 7.71 (s, 1H, C*H*-Ar), 7.63 (d, *J =* 8.7 Hz, 1H, N*H*_{TCA}), 7.51-7.47 (m, 2H, C*H*-Ar), 7.39-7.07 (m, 47H, C*H*-Ar), 6.84 (d, *J =* 7.6 Hz, 2H, C*H*-Ar), 6.40 (br s, 1H, H-1D), 5.25 (dd, *J =* 3.3, 2.0 Hz, 1H, H-2A), 5.20 (t, *J* = 3.4 Hz, 1H, H-2C), 5.09 (s, 1H, H-1B), 4.99 (s, 1H, H-1C), 4.94-4.78 (m, 11H, H-1A, H-1E, C*H*HPh), 4.65-4.45 (m, 14H, H-2D, C*H*HPh), 4.31 (d, *J =* 12.1 Hz, 1H, C*H*HPh), 4.21-4.07 (m, 3H, H-5D, H-3A, H-3C), 4.05 (d, *J =* 1.5 Hz, 2H, C*H*_{2CA}), 4.02 (dd, *J =* 5.1, 2.9 Hz, 1H, H-3D), 3.94 (t, *J =* 3.3, 1H, H-2B), 3.89 (t, *J =* 9.2, 1H, H-3E), 3.81-3.74 (m, 7H, H-5A, H-4D, H-3B, H-5C, H-6aD, H-6bD, H-5E), 3.73 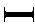 3.54 (m, 4H, H-5B, H-4E, H-6aE, H-2E), 3.49-3.40 (m, 3H, H-4C, H-4B, H-6bE), 3.30 (t, *J =* 9.3 Hz, 1H, H-4A), 2.76 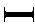 2.66 (m, 4H, 2 x C*H*_{2Lev}), 2.20 (s, 3H, C*H*_{3Lev}), 1.19 (d, *J =* 6.0 Hz, 3H, H-6B), 1.12 (d, *J =* 6.2 Hz, 3H, H-6C), 1.02 (d, *J =* 6.2 Hz, 3H, H-6A), 0.92 (s, 9H, C(C*H*₃)₃), 0.13 (s, 3H, C*H*₃Si), 0.08 (s, 3H, C*H*₃Si).

### 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-[2,3,4,6-tetra-O-benzyl-α-D-glucopyranosyl-(1→4)]-2-O-chloroacetyl-α-L-rhamnopyranosyl-(1→3)-4,6-O-benzylidene-2-deoxy-2-trichloroacetamido-α/β-D-glucopyranosyl (N-phenyl)trifluoroacetimidate (34).

The title compound was synthesized from pentasaccharide **31** (250 mg, 0.127 mmol, 1.0 equiv.) according to the general procedure for PTFA activation. Purification by flash chromatography (cHex/EtOAc 95:5 to 7:3) gave compound **34** (195 mg, 93 %, over 2 steps) as a cristalline solid.

**¹H NMR (400 MHz, CDCl₃) δ (ppm):** 7.51 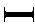 7.49 (m, 2H, C*H*-Ar), 7.40 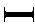 7.14 (m, 47H, CH-Ar), 6.83 (d, *J=* 7.6 Hz, 1H, N*H*'_{TCA}), 6.81 (d, *J =* 7.1 Hz, 1H, N*H*_{TCA}), 6.42 (br s, 1H, H-1D), 5.61 (s, 1H, H-7D), 5.30 (br s, 1H, H-2A), 5.18 (t, *J =* 2.8 Hz, 1H, H-2C), 5.04 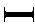 5.03 (m, 2H, H-1C, H-1A), 4.95 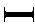 4.90 (m, 5H, H-1B, H-1E, C*H*HPh), 4.84 (d, *J =* 10.9 Hz, 1H, C*H*HPh), 4.83 (d, *J =* 10.9 Hz, 1H, C*H*HPh), 4.67-4.50 (m, 10H, H-2D, C*H*HPh), 4.41-4.36 (m, 3H, H-6aD, H-3C, C*H*HPh), 4.05 (dd, *J =* 9.3, 3.2 Hz, 1H, H-3A), 4.16-4.12 (m, 1H, H-3D), 4.03-3.73 (m, 10H, C*H*_{2CA}, H-5D, H-5A, H-2B, H-5C, H-3E, H-5E, H-6bD, H-4D, H-6aE, H-4E), 3.68 (dd, *J =* 9.3, 2.5 Hz, 1H, H-3B), 3.60-3.53 (m, 1H, H-5B), 3.48-3.37 (m, 4H, H-2E, H-4C, H-4B, H-4A), 2.70-2.53 (m, 4H, 2 x C*H*_{2Lev}), 2.13 (s, 3H, C*H*_{3Lev}), 1.31 (d, *J =* 6.0 Hz, 3H, H-6A), 1.18 (d, *J =* 6.2 Hz, 3H, H-6B), 0.93 (s, 9H, C(C*H*₃)₃), 0.91 (d, *J =* 6.2 Hz, 3H, H-6C), 0.17 (s, 3H, C*H*₃Si), 0.13 (s, 3H, C*H*₃Si).

### [2,3,4,6-tetra-O-benzyl-α-D-glucopyranosyl-(1→3)]-4-O-benzyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranosyl (N-phenyl)trifluoroacetimidate (35).

The title compound was synthesized from pentasaccharide **32** (500 mg, 0.520 mmol, 1.0 equiv.) according to the general procedure for PTFA activation. Purification by flash chromatography (cHex/EtOAc 95:5 to 7:3) gave compound **35** (452 mg, 92 %, over 2 steps) as a cristalline solid along with traces of its corresponding oxazoline **43.**

**¹H NMR (400 MHz, CDCl₃)** δ **(ppm):** 7.88 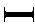 7.84 (m, 3H, C*H*-Ar), 7.71 (s, 1H, C*H*-Ar), 7.54 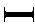 7.48 (m, 4H, C*H*-Ar), 7.40 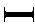 7.07 (m, 47H, C*H*-Ar), 6.82 (d, *J =* 7.9 Hz, 1H, N*H*_{TCA}), 6.78 (d, *J =* 9.0 Hz, 1H, N*H*'_{TCA}), 6.41 (br s, 1H, H-1D), 5.60 (s, 1H, H-7D), 5.54 (dd, *J =* 3.5, 2.1 Hz, 1H, H-2A), 5.23 (d, *J =* 3.4, 1H, H-1E), 5.18 (t, *J =* 3.5 Hz, 1H, H-2C), 5.02 (br s, 2H, H-1B, H-1C), 4.99 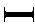 4.82 (m, 7H, H-1A, C*H*HPh), 4.76 (d, *J =* 12.0 Hz, 1H, C*H*HPh), 4.69 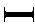 4.47 (m, 10H, H-2D, C*H*HPh), 4.42-4.33 (m, 2H, H-6aD, C*H*HPh), 4.23 (dd, *J =* 9.8, 3.1 Hz, 1H, H-3A), 4.19 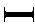 3.95 (m, 7H, H-3D, H-3C, H-3E, C*H*_{2CA}, H-5D, H-5C), 3.94 (t, *J =* 3.4 Hz, 1H, H-2B), 3.89 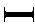 3.77 (m, 3H, H-5A, H-6bD, H-3B), 3.71-3.61 (m, 3H, H-5B, H-6aE, H-2E), 3.57-3.48 (m, 3H, H-6bE, H-4A, H-4B), 3.66 (t, *J =* 9.3 Hz, 1H, H-4C), 2.59 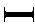 2.35 (m, 4H, 2 x C*H*_{2Lev}), 2.06 (s, 3H, C*H*_{3Lev}), 1.28 (d, *J =* 6.0 Hz, 3H, H-6B), 1.22 (d, *J =* 6.2 Hz, 3H, H-6A), 0.76 (d, *J =* 6.2 Hz, 3H, H-6C).

### Synthesis of pentasaccharide acceptors (1b, 2a and 3a)

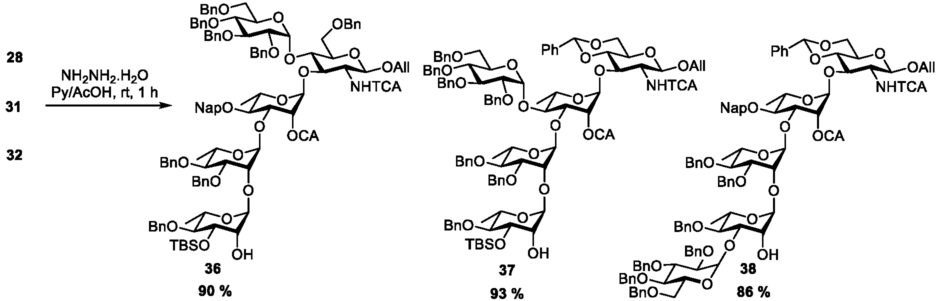

**General** procedure for the preparation of acceptors from pentasaccharides 28, 31, 32, 51, 52, 53 and 54.

To a solution of pentasaccharide (1.0 equiv.) in a mixture of Pyr./AcOH (1:1, 27 mL.mmol) stirred at 0 °C under Ar, was added hydrazine monohydrate (50-60 %, 1.2 or 2.0 equiv.). The mixture was stirred for 1 h while being gradually warmed to rt. Following addition of DCM, the organic phase was washed several times with a solution of CuSO₄. Then, the combined organic phases were washed with water, brine and dried over Na₂SO₄. The filtrate was concentrated under reduced pressure and purified by flash chromatography.

### Allyl 4-O-benzyl-3-O-tert-butyldimethylsilyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-[2,3,4,6-tetra-O-benzyl-α-D-glucopyranosyl-(1→4)]-6-O-benzyl-2-deoxy-2-trichloroacetamido-β-D-glucopyranoside (36).

The title compound was synthesized from pentasaccharide **28** (450 mg, 0.213 mmol, 1.0 equiv.) according to the general procedure for the preparation of acceptors. Purification by flash chromatography (cHex/EtOAc 95:5 to 7:3) gave compound **36** (386 mg, 90 %) as a cristalline solid.

**¹H NMR (400 MHz, CDCl₃) δ (ppm):** 7.92-7.88 (m, 4H, C*H*-Ar), 7.82 (s, 1H, C*H*-Ar), 7.57-7.54 (m, 4H, C*H*-Ar), 7.51-7.18 (m, 45H, C*H*-Ar), 6.06-6.96 (m, 1H, H-2_{All}), 5.43 (ddd, *J=* 18.8, 3.7, 1.4 Hz, 1H, H-3a_{All}), 5.37 (t, *J =* 3.2 Hz, 1H, H-2C), 5.29 (ddd, *J =* 10.5, 3.4, 1.2 Hz, 1H, H-3b_{All}), 5.18 (s, 1H, H-1B), 5.15 (br s, 2H, H-1C, H-1A), 5.02-4.86 (m, 10H, H-1E, H-1D, C*H*HPh), 4.74 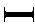 4.51 (m, 11H, C*H*HPh), 4.39 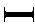 4.30 (m, 3H, H-1a_{All}, C*H*HPh, H-2D), 4.26 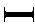 4.05 (m, 9H, H-4D, H-3C, C*H*_{2CA}, H-1b_{All}, H-3A, H-6aD, H-6bD, H-3D, H-2B, H-2A), 4.01 (t, *J =* 9.4 Hz, 1H, H-3E), 3.93 (dd, *J =* 9.4, 2.6 Hz, 1H, H-3B), 3.88-3.76 (m, 6H, H-5A, H-5B, H-5E, H-5C, H-5D, H-4E), 3.73 (dd, *J =* 10.9, 2.5 Hz, 1H, H-6aE), 3.63 (dd, *J =* 9.7, 3.8 Hz, 1H, H-2E), 3.61 (t *J =* 9.3 Hz, 1H, H-4C), 3.54 (*t J =* 9.4 Hz, 1H, H-4B), 3.47-3.44 (m, 1H, H-6bE), 3.42 (t, *J =* 9.2 Hz, 1H, H-4A), 1.36 (d, *J =* 5.8 Hz, 3H, H-6A), 1.26 (d, *J =* 6.2 Hz, 3H, H-6B), 1.11 (d, *J=* 6.2 Hz, 3H, H-6C), 1.05 (s, 9H, C(C*H*₃)₃), 0.22 (s, 3H, C*H*₃Si), 0.20 (s, 3H, C*H*₃Si).

### Allyl 4-O-benzyl-3-O-tert-butyldimethylsilyl-α-L-rhamnopyranosyl-(1→2)-3,4-di-O-benzyl-α-L-rhamnopyranosyl-(1→3)-[2,3,4,6-tetra-O-benzyl-α-D-glucopyranosyl-(1→4)]-2-O-chloroacetyl-α-L-rhamnopyranosyl-(1→3)-4,6-O-benzylidene-2-deoxy-2-trichloroacetamido-β-D-glucopyranoside (37).

To title compound was synthesized from pentasaccharide **31** (2000 mg, 1.014 mmol, 1.0 equiv.) according to the general procedure for the preparation of acceptors. Purification by flash chromatography (Tol/EtOAc 95:5 to 8:2) gave compound **37** (1.76 g, 93 %) as a cristalline solid.

**¹H NMR (400 MHz, CDCl₃) δ (ppm):** 7.47 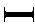 7.45 (m, 2H, C*H*-Ar), 7.39 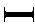 7.18 (m, 44H, *CH-*Ar), 7.01 (d, *J =* 5.4 Hz, 1H, N*H*_{TCA}), 5.94-5.84 (m, 1H, H-2_{All}), 5.55 (s, 1H, H-7D), 5.31 (ddd, *J =* 17.2, 3.5, 1.4 Hz, 1H, H-3a_{All}), 5.25-5.21 (m, 2H, H-3b_{All}, H-2C), 5.13 (s, 1H, H-1A), 5.08 (d, *J*_{1D,2D} = 7.5 Hz, 1H, H-1D), 4.96 (d, *J*_{1B,2B} = 1.5 Hz, 1H, H-1B), 4.91-4.86 (m, 6H, H-1E, H-1C, C*H*HP), 4.83 (d, *J =* 10.9 Hz, 1H, C*H*HPh), 4.64-4.34 (m, 14H, H-3D, H-3C, H-6aD, C*H*HPh, H-1a_{All}), 4.14-4.07 (m, 2H, H-1b_{All}, H-3A), 3.99 (s, 2H, C*H*_{2CA}), 3.97 (dd, *J =* 3.3, 1.5 Hz, 1H, H-2A), 3.95-3.88 (m, 4H, H-5A, H-5C, H-5E, H-2B), 3.85-3.75 (m, 4H, H-3E, H-6bD, H-6aE, H-6bE), 3.72 (t, *J =* 9.4 Hz, 1H, H-4E), 3.66 (dd, *J =* 9.6, 2.6 Hz, 1H, H-3B), 3.63-3.50 (m, 3H, H-4D, H-5D, H-5B), 3.48-3.35 (m, 5H, H-2D, H-4C, H-4A, H-2E, H-4B), 1.33 (d, *J=* 6.2 Hz, 3H, H-6A), 1.18 (d, *J=* 6.2 Hz, 3H, H-6B), 0.98 (s, 9H, C(C*H*₃)₃), 0.82 (d, *J=* 6.1 Hz, 3H, H-6C), 0.16 (s, 3H, C*H*₃Si), 0.15 (s, 3H, C*H*₃Si).

### Allyl [2,3,4,6-tetra-O-benzyl-α-D-glucopyranosyl-(1→3)]-4-O-benzyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranoside (38).

The title compound was synthesized from pentasaccharide **32** (224 mg, 0.112 mmol, 1.0 equiv.) according to the general procedure for the preparation of acceptors. Purification by flash chromatography (Tol/EtOAc 95:5 to 8:2) gave compound **38** (184 mg, 86 %) as a cristalline solid.

**¹H NMR (400 MHz, CDCl₃) δ (ppm):** 7.86 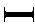 7.83 (m, 3H, C*H*-Ar), 7.71 (s, 1H, C*H*-Ar), 7.52 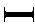 7.46 (m, 4H, C*H*-Ar), 7.39 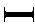 7.14 (m, 47H, C*H*-Ar), 7.03 (d, *J =* 6.9 Hz, 1H, N*H*_{TCA}), 5.93-5.83 (m, 1H, H-2_{All}), 5.57 (s, 1H, H-7D), 5.30 (ddd, *J=* 17.1, 3.8, 1.4 Hz, 1H, H-3a_{All}), 5.25 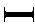 5.22 (m, 2H, H-2C, H-3b_{All}), 5.16 (br s, 1H, H-1A), 5.09 (d, *J*_{1D,2D} = 8.2 Hz, 1H, H-1D), 5.03 (br s, 1H, H-1B), 4.97 (d, *J =* 10.9 Hz, 1H, C*H*HPh), 4.92 (d, *J =* 11.0 Hz, 1H, C*H*HPh), 4.91 (d, *J* = 11.5 Hz, 1H, C*H*HPh), 4.88 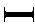 4.83 (m, 5H, H-1E, H-1C, C*H*HPh), 4.73 (d, *J* = 10.5 Hz, 1H, C*H*HPh), 4.68 (d, *J =* 11.0 Hz, 1H, C*H*HPh), 4.66 (d, *J =* 10.5 Hz, 1H, C*H*HPh), 4.60 (d, *J =* 10.5 Hz, 1H, C*H*HPh), 4.57 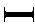 4.54 (m, 5H, H-3D, C*H*HPh), 4.49 (d, *J =* 10.9 Hz, 1H, C*H*HPh), 4.41-4.33 (m, 2H, H-6aD, H-1a_{All}), 4.31 (d, *J =* 12.0 Hz, 1H, C*H*HPh), 4.13-3.93 (m, 10H, H-1b_{All}, H-3C, H-3E, H-2B, C*H*_{2CA}, H-2A, H-3A, H-5C, H-5E), 3.85 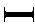 3.78 (m, 3H, H-5A, H-6bD, H-3B), 3.75 (t, *J =* 9.7 Hz, H-4E), 3.71 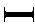 3.56 (m, 4H, H-5B, H-4D, H-2E, H-5D), 3.52 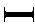 3.38 (m, 5H, H-4A, H-6aE, H-2D, H-4B, H-6bE), 3.34 (t, *J =* 9.4 Hz, 1H, H-4C), 1.28 (d, *J =* 6.1 Hz, 3H, H-6B), 1.19 (d, *J =* 6.2 Hz, 3H, H-6A), 0.71 (d, *J =* 6.1 Hz, 3H, H-6C).

### Synthesis of decasaccharides (1b, 2a and 3a)

### Synthesis of decasaccharide [_{Lev}ABC(E4)D]₂ 39 (1b)

The title compound was obtained as follows:

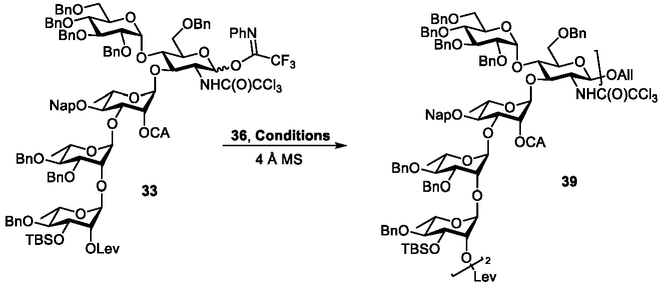

### Synthesis of decasaccharide [_{Lev}AB(E)CD]₂ 40 (2a)

The title compound was obtained as follows:

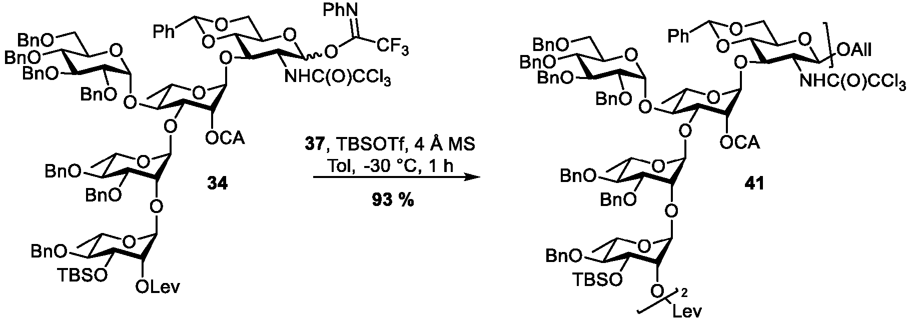

### Synthesis of decasaccharide [_{Lev}(E)ABCD]₂ 42 (3a)

The title compound was obtained as follows:

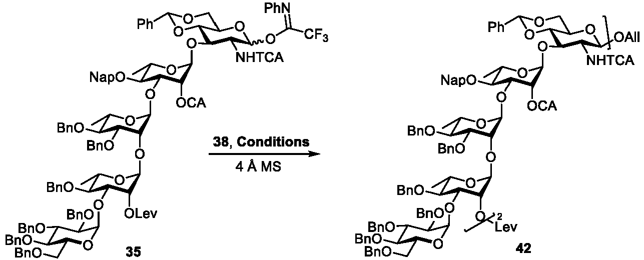

### Synthesis of decasaccharide donors and acceptors (1b, 2a and 3a)

The title compound was obtained as follows:

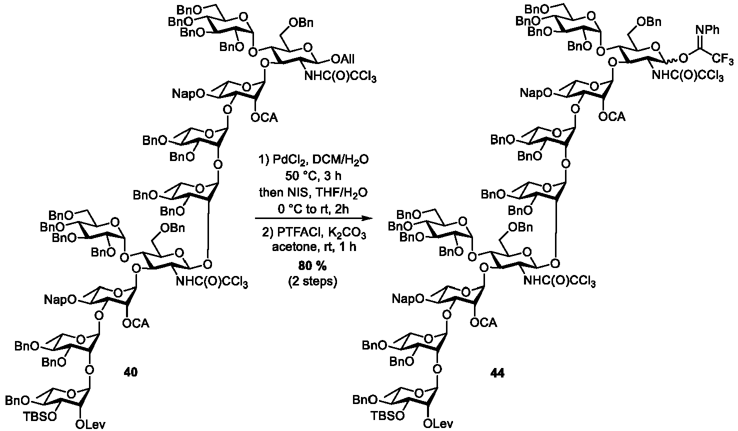

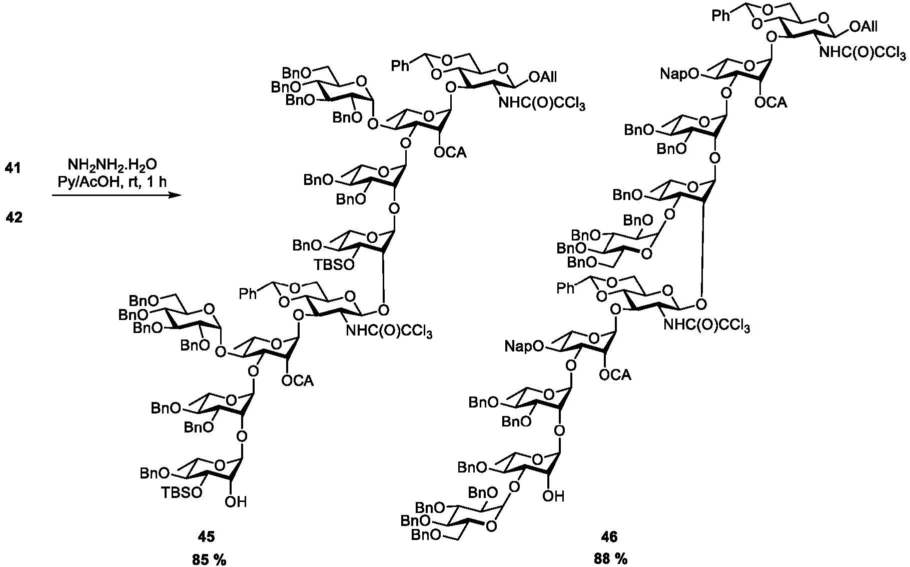

### Synthesis of pentadecasaccharides (1b, 2a and 3a)

### Synthesis of pentadecasaccharide [_{Lev}ABC(E)D]₃ 47 (1a/1b)

The title compound was obtained as follows:

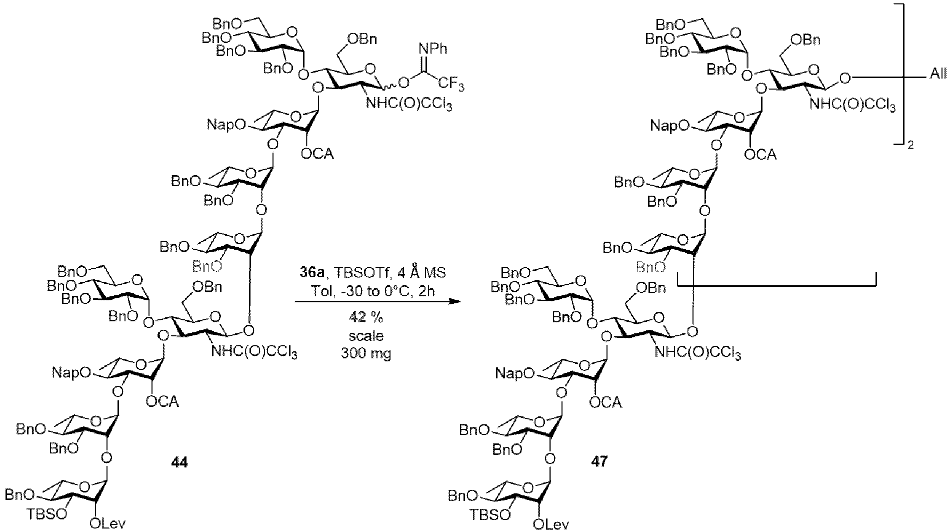

### Synthesis of pentadecasaccharide [_{Lev}AB(E)CD]₃ 48 (2a)

The title compound was obtained as follows:

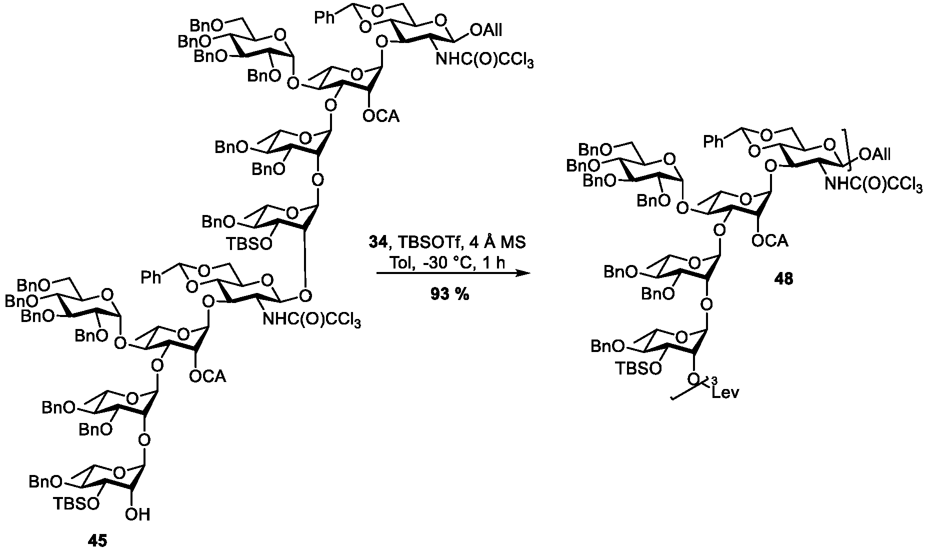

### Synthesis of pentadecasaccharide [_{Lev}(E)ABCD]₃ 49 (3a)

The title compound was obtained as follows:

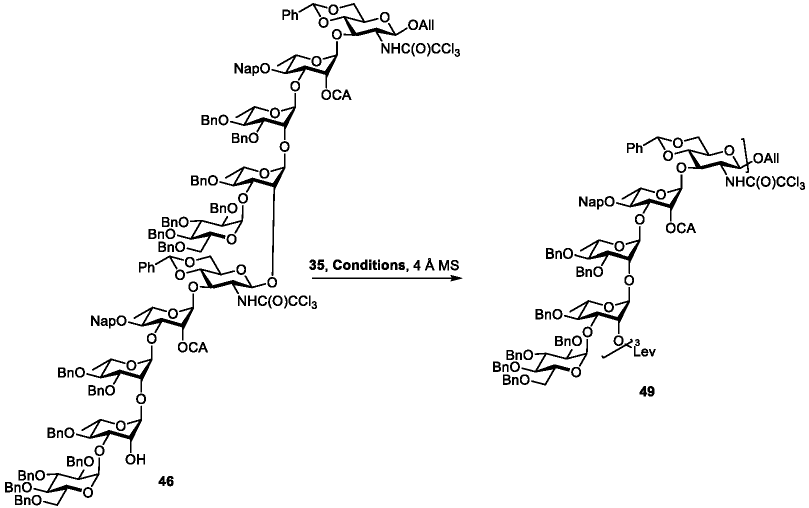

### Synthesis of pentadecasaccharides with their corresponding linker

### Synthesis of azidoethyl- or azidopropyl-equipped pentasaccharides

The title compounds were obtained as follows:

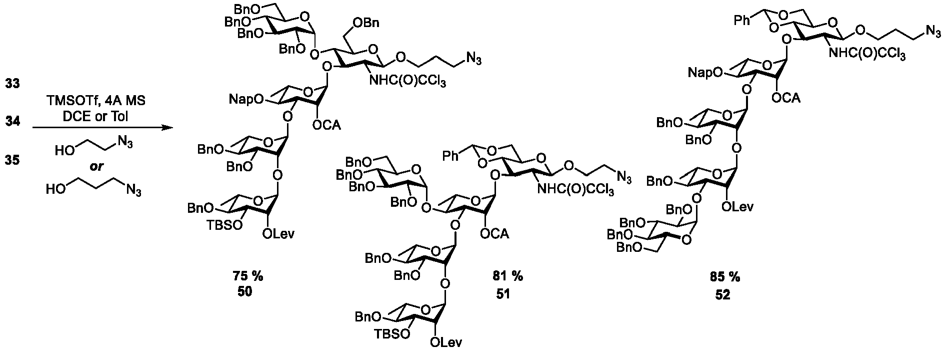

### (3-Azido-1-propyl) 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-[2,3,4,6-tetra-O-benzyl-α-D-glucopyranosyl-(1→4)]-6-O-benzyl-2-deoxy-2-trichloroacetamido-β-D-glucopyranoside (50).

To a solution of donor 33 (273 mg, 0.122 mmol, 1.0 equiv.) in anhydrous Toluene ( 2.4 mL), was added activated ground molecular sieves (4Å, 547 mg). The mixture was stirred at rt for 15 min under Ar. Then, the reaction flask was cooled to -65 °C. After 5 min, TMSOTf (4 µL, 0.024 mmol, 0.2 equiv.) was added. After 10 min, acceptor azidopropanol (17 µL, 0.183 mmol, 1.5 equiv. dissolved in 0.6 mL of anhydrous Toluene) was added to the suspension and the reaction mixture was allowed to stirr under Ar for 1 h from -65 to 0 °C.

The reaction was quenched with Et₃N, filtered over Celite, rinsed with DCM (50 mL) and concentrated under reduced pressure. The crude was directly purified by flash chromatography (cHex/EtOAc 95:5 to 8:2) to give compound **50** (197 mg, 75 %), as a cristalline solid.

**¹H NMR (400 MHz, CDCl₃) δ (ppm):** 7.85 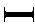 7.81 (m, 3H, C*H*-Ar), 7.76 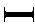 7.74 (m, 2H, N*H*_{TCA}, C*H*-Ar), 7.51 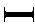 7.48 (m, 2H, C*H*-Ar), 7.41-7.10 (m, 40H, C*H*-Ar), 5.26 (dd, *J =* 3.4, 2.0 Hz, 1H, H-2A), 5.22 (dd, *J =* 3.5, 1.9 Hz, 1H, H-2C), 5.09 (d, *J*_{1B,2B} = 1.3 Hz, 1H, H-1B), 5.03 (d, *J*_{1C,2C} = 1.4 Hz, 1H, H-1C), 4.91 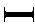 4.77 (m, 9H, H-1A, H-1E, C*H*HPh), 4.72 (d, *J*_{1D,2D} = 2.3 Hz, 1H, H-1D), 4.67 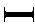 4.48 (m, 9H, C*H*HPh), 4.44 (d, *J =* 10.5 Hz, 1H, C*H*HPh), 4.26 (d, *J =* 12.0 Hz, 1H, C*H*HPh), 4.20 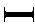 4.06 (m, 6H, H-2D, H-3A, C*H*_{2CA}, H-5D, H-3C), 3.99 (br s, 2H, H-3D, H-4D), 3.94 (t, *J* = 2.9 Hz, 1H, H-2B), 3.93 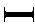 3.84 (m, 3H, H-6aD, H-3E, C*H*_{Link}), 3.82 (dd, *J* = 9.4, 2.6 Hz, 1H, H-3B), 3.79-3.66 (m, 6H, H-5A, H-5E, H-5B, H-5C, H-6bD, H-4E), 3.64 (dd, *J* = 10.8, 2.7 Hz, 1H, H-6aE), 3.58-3.36 (m, 7H, C*H*_{Link}, H-2E, H-4C, H-4B, C*H*_{2Link}, H-6bE), 3.31 (t, *J* = 9.2 Hz, 1H, H-4A), 2.76 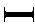 2.65 (m, 4H, 2 × C*H*_{2Lev}), 2.20 (s, 3H, C*H*_{3Lev}), 1.91 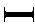 1.79 (m, 2H, C*H*_{*2*Link}), 1.27 (d, *J =* 6.1 Hz, 3H, H-6B), 1.17 (d, *J =* 6.2 Hz, 3H, H-6C), 1.02 (d, *J =* 6.2 Hz, 3H, H-6A), 0.92 (s, 9H, C(C*H*₃)₃), 0.14 (s, 3H, C*H*₃Si), 0.09 (s, 3H, C*H*₃Si).

### (2-Azido-1-ethyl) 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-[2,3,4,6-tetra-O-benzyl-α-D-glucopyranosyl-(1→4)]-2-O-chloroacetyl-α-L-rhamnopyranosyl-(1→3)-4,6-O-benzylidene-2-deoxy-2-trichloroacetamido-β-D-glucopyranoside (51).

To a solution of PTFA donor **34** (220 mg, 0.105 mmol, 1.0 equiv.) in anhydrous DCE ( 2.4 mL), was added activated ground molecular sieves (4Å, 440 mg). The mixture was stirred at rt for 15 min under Ar. Then, the reaction flask was cooled to -30 °C. After 5 min, TMSOTf (2 µL, 0.010 mmol, 0.1 equiv.) was added. After 10 min, acceptor azidoethanol (12 µL, 0.157 mmol, 1.5 equiv. dissolved in 1.3 mL of anhydrous DCE) was added to the suspension and the reaction mixture was allowed to stirr under Ar for 2 h 30 from -15 °C to rt.

The reaction was quenched with Et₃N, filtered over Celite, rinsed with DCM (50 mL) and concentrated under reduced pressure. The crude was directly purified by flash chromatography (Tol/EtOAc 95:5 to 85:15) to give compound **51** (170 mg, 81 %), as a cristalline solid.

**¹H NMR (400 MHz, CDCl₃) δ (ppm):** 7.47 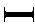 7.45 (m, 2H, C*H*-Ar), 7.40 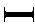 7.39 (m, 4H, *CH-*Ar), 7.367.18 (m, 43H, C*H*-Ar), 5.55 (s, 1H, H-7D), 5.29 (dd, *J =* 3.4, 2.0 Hz, 1H, H-2A), 5.22 (t, *J =* 3.4 Hz, 1H, H-2C), 5.14 (d, *J*_{1D,2D} = 7.8 Hz, 1H, H-1D), 5.05 (s, 1H, H-1A), 4.95 (s, 1H, H-1B), 4.94 (d, *J =* 10.9 Hz, 1H, C*H*HPh), 4.93 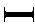 4.84 (m, 6H, H-1E, H-1C, C*H*HPh), 4.81 (d, *J=* 10.9 Hz, 1H, CHHPh), 4.65 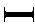 4.48 (m, 11H, C*H*HPh, H-3D), 4.40 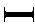 4.36 (m, 3H, H-6aD, C*H*HPh), 4.21 (dd, *J =* 9.2, 3.1 Hz, 1H, H-3A), 4.05-3.87 (m, 8H, H-3C, C*H*_{Link}, C*H*_{2CA}, H-5A, H-5C, H-5E, H-2B), 3.84 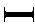 3.64 (m, 7H, H-3E, H-6bD, H-6aE, H-6bE, C*H*_{Link}, H-3B, H-4E), 3.62 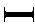 3.36 (m, 10H, H-4D, H-5D, H-5B, C*H*_{2Link}, H-2D, H-4C, H-4B, H-4A, H-2E), 2.71 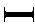 2.54 (m, 4H, 2 × C*H*_{2Lev}), 2.13 (s, 3H, C*H*_{3Lev}), 1.33 (d, *J =* 6.2 Hz, 3H, H-6A), 1.18 (d, *J* = 6.2 Hz, 3H, H-6B), 0.94 (s, 9H, C(C*H*₃)₃), 0.83 (d, *J =* 6.1 Hz, 3H, H-6C), 0.16 (s, 3H, C*H*₃Si), 0.13 (s, 3H, C*H*₃Si).

### (3-Azido-1-propyl) [2,3,4,6-tetra-O-benzyl-α-D-glucopyranosyl-(1→3)]-4-O-benzyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranoside (52).

To a solution of PTFA donor **35** (100 mg, 0.047 mmol, 1.0 equiv.) in anhydrous Toluene (1 mL) was added activated ground molecular sieves (4Å, 200 mg). The mixture was stirred at rt for 15 min under Ar. Then, the reaction flask was cooled to -60 °C and TMSOTf (1.0 µL, 0.006 mmol, 0.125 equiv.) was added. After 10 min, acceptor azidopropanol (7 µL, 0.07 mmol, 1.5 equiv. dissolved in 1 mL of anhydrous Toluene) was added to the previous solution and the mixture was stirred from -60 to -40°C for 2 h.

The reaction was quenched with Et₃N, filtered over Celite, rinsed with DCM (20 mL) and concentrated under reduced pressure. The crude was directly purified by flash chromatography (cHex/EtOAc 95:5 to 6:4) to give compound **52** (82 mg, 85 %), as a cristalline solid.

**¹H NMR (400 MHz, CDCl₃) δ (ppm):** 7.87 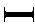 7.84 (m, 3H, C*H*-Ar), 7.71 (s, 1H, C*H*-Ar), 7.53 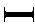 7.48 (m, 4H, C*H*-Ar), 7.42 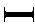 7.09 (m, 37H, C*H*-Ar), 7.05 (d, *J =* 7.2 Hz, 1H, N*H*_{TCA}), 5.94 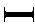 5.84 (m, 1H, H-2_{Al1}), 5.57 (s, 1H, H-7D), 5.56 (t, *J =* 3.3 Hz, H-2A), 5.26 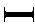 5.23 (m, 2H, H-1E, H-2C), 5.06 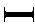 5.01 (m, 3H, H-1D, H-1B, C*H*HPh), 4.99 (d, *J*_{1A,2A} = 1.3 Hz, 1H, H-1A), 4.96 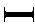 4.87 (m, 5H, H-1C, C*H*HPh), 4.82 (d, *J =* 11.4 Hz, 1H, C*H*HPh), 4.78 (d, *J =* 11.9 Hz, 1H, C*H*HPh), 4.7869 (d, *J =* 11.9 Hz, 1H, C*H*HPh), 4.78 (d, *J =* 11.1 Hz, 1H, C*H*HPh), 4.63 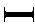 4.49 (m, 8H, H-3D, C*H*HPh), 4.41 (dd, *J =* 10.4, 4.4 Hz, 1H, H-6aD), 4.36 (d, *J =* 12.1 Hz, 1H, C*H*HPh), 4.25 (dd, *J =* 9.7, 3.1 Hz, 1H, H-3A), 4.14 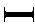 3.95 (m, 8H, H-3E, H-3C, H-5E, C*H*_{2CA}, H-5C, C*H*_{Link}, H-2B), 3.88 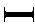 3.79 (m, 4H, H-5A, H-6bD, H-4E, H-3B), 4.25 (dd, *J =* 9.7, 3.1 Hz, 1H, H-3A), 3.70 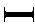 3.49 (m, 10H, H-5B, H-6aE, C*H*_{Link}, H-4D, H-2E, H-5D, H-6bE, H-4A, H-2D, H-4B), 3.42 (td, *J* = 6.7, 1.1 Hz, 2H, C*H*_{*2*Link}), 3.33 (t, *J* = 9.5 Hz, H-4C), 2.60 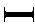 2.34 (m, 4H, 2 × C*H*_{2Lev}), 2.05 (s, 3H, C*H*_{3Lev}), 1.89 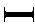 1.83 (m, 2H, C*H*_{*2*Link}), 1.26 (d, *J =* 6.2 Hz, 3H, H-6B), 1.20 (d, *J =* 6.2 Hz, 3H, H-6A), 0.71 (d, *J =* 6.1 Hz, 3H, H-6C).

### Synthesis of azidopropyl- or azidoethyl-equipped decasaccharides

### Starting from pentasaccharides

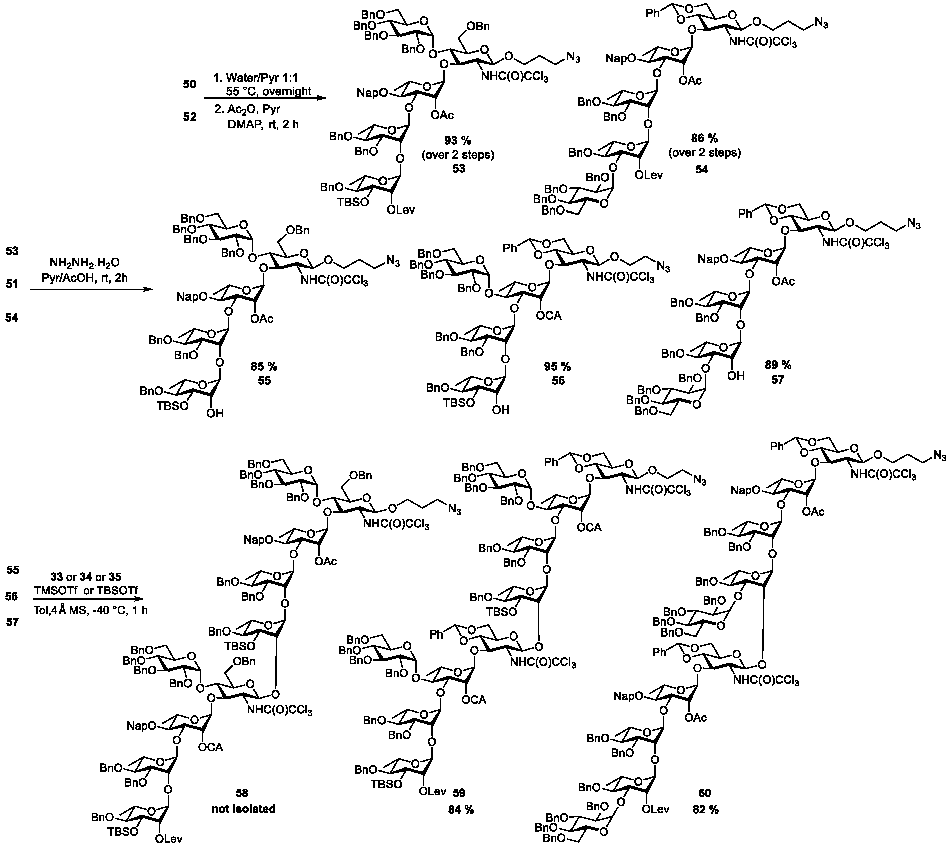

### (3-Azido-1-propyl) 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-2-O-acetyl-4-O-(2-naphtylmethyl)-α-L-rhamnopyranosyl-(1→3)-[2,3,4,6-tetra-O-benzyl-α-D-glucopyranosyl-(1→4)]-6-O-benzyl-2-deoxy-2-trichloroacetamido-β-D-glucopyranoside (53).

The compound 50 (197 mg, 0.091 mmol, 1.0 equiv.) was dissolved in a mixture of water/Pyr (1:1.3, 6.4 mL). The suspension was stirred at 55 °C overnight. After evaporation and coevaporation three times with toluene under reduced pressure, the obtained crude was dissolved in a mixture of Ac₂O/Pyr (1:1, 5.5 mL). DMAP (2 mg, 0.02 mmol, 0.2 equiv.) was added and the suspension was allowed to run at overnight at rt under Ar.

Thhe solvents were concentrated under reduced pressure and co-evaporated with toluene (3 × 2 mL). The crude was purified by flash chromatography (Tol/EtOAc 95:5 to 75:15) to give compound **54** (180 mg, 93 %, over 2 steps), as a cristalline yellow solid.

**¹H NMR (400 MHz, CDCl₃) δ (ppm):** 7.71 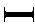 7.64 (m, 4H, C*H*-Ar), 7.61 (s, 1H, C*H*-Ar), 7.37 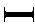 7.33 (m, 2H, C*H*-Ar), 7.27 (dd, *J =* 8.3, 1.5 Hz, 1H, *CH*-Ar), 7.24 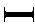 6.97 (m, 46H, CH-Ar), 5.12 (dd, *J =* 3.4, 2.0 Hz, 1H, H-2A), 5.04 (dd, *J =* 3.6, 1.8 Hz, 1H, H-2C), 4.94 (d, *J*_{1B,2B} = 1.3 Hz, 1H, H-1B), 4.88 (d, *J*_{1C,2C} = 1.3 Hz, 1H, H-1C), 4.78 (d, *J =* 11.0 Hz, 1H, CHHPh), 4.77 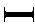 4.70 (m, 5H, **H-1A,** C*H*HPh), 4.67 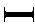 4.63 (m, 3H, H-1E, C*H*HPh), 4.58 (d, *J*_{1D,2D} = 2.2 Hz, 1H, H-1D), 4.53 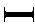 4.29 (m, 10H, C*H*HPh), 4.11 (d, *J =* 12.0 Hz, 1H, C*H*HPh), 4.08 (d, *J =* 8.0 Hz, 1H, H-2D), 4.01 (dd, *J =* 9.0, 3.2 Hz, 1H, H-3A), 3.99 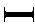 3.96 (m, 1H, H-5D), 3.92 (dd, *J =* 9.3, 3.3 Hz, 1H, H-3C), 3.85 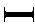 3.72 (m, 6H, H-3D, H-4D, H-2B, H-6aD, H-3E, C*H*_{Link}), 3.69 (dd, *J =* 9.3, 2.7 Hz, 1H, H-3B), 3.67 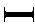 3.53 (m, 6H, H-5A, H-5E, H-5B, H-6bD, H-5C, H-4E), 3.50 (dd, *J =* 10.9, 2.7 Hz, 1H, H-6aE), 3.43 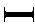 3.27 (m, 6H, H-2E, C*H*_{Link}, H-4C, H-4B, C*H*_{*2*Link}), 3.23 (dd, *J =* 10.9, 1.4 Hz, 1H, H-6bE), 3.17 (t, *J =* 9.3 Hz, 1H, H-4A), 2.62 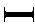 2.51 (m, 4H, 2 x C*H*_{2Lev}), 2.06 (s, 3H, C*H*_{3Lev}), 2.03 (s, 3H, C*H*_{3Ac}), 1.78-1.68 (m, 2H, C*H*_{*2*Link}), 1.14 (d, *J =* 6.2 Hz, 3H, H-6B), 1.07 (d, *J =* 6.2 Hz, 3H, H-6C), 0.89 (d, *J =* 6.2 Hz, 3H, H-6A), 0.79 (s, 9H, C(C*H*₃)₃), 0.00 (s, 3H, C*H*₃Si), -0.04 (s, 3H, C*H*₃Si).

### (3-Azido-1-propyl) [2,3,4,6-tetra-O-benzyl-α-D-glucopyranosyl-(1→3)]-4-O-benzyl-2-O-levulinoyl-α-L-rhamnopyranosyl-(1→2)-3,4-di-O-benzyl-α-L-rhamnopyranosyl-(1→3)-2-O-acetyl-4-O-(2-naphtylmethyl)-α-L-rhamnopyranosyl-(1→3)-4,6-O-benzylidene-2-deoxy-2-trichloroacetamido-β-D-glucopyranoside (54).

The compound **52** (840 mg, 0.411 mmol, 1.0 equiv.) was dissolved in a mixture of water/Pyr (1:1, 24 mL). The suspension was stirred at 55 °C overnight. After evaporation and coevaporation three times with toluene under reduced pressure, the obtained crude was dissolved in a mixture of Ac₂O/Pyr (1:1, 38 mL). DMAP (23 mg, 0.206 mmol, 0.5 equiv.) was added and the suspension was allowed to run at rt for 4 h under Ar.

Following addition of DCM (100 mL), the solution was poured into a separatory funnel, washed with aqueous solution of CuSO₄ (3 × 50 mL), with water (50 mL) and brine (50 mL), dried over Na₂SO₄ and concentrated to dryness. The crude was directly purified by flash chromatography (Tol/EtOAc 95:5 to 7:3) to give compound **54** (713 mg, 86 %, over 2 steps), as a cristalline yellow solid.

**¹H NMR (400 MHz, CDCl₃) δ (ppm):** 7.87 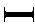 7.83 (m, 3H, C*H*-Ar), 7.70 (s, 1H, C*H*-Ar), 7.53 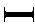 7.46 (m, 5H, C*H*-Ar), 7.41 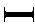 7.11 (m, 47H, C*H*-Ar), 7.08 (d, *J=* 6.7 Hz, 1H, N*H*_{TCA}), 5.57 (s, 1H, H-7D), 5.53 (t, *J =* 3.2 Hz, H-2A), 5.24 (d, *J*_{1E,2E} = 3.2 Hz, 1H, H-1E), 5.17 (dd, *J =* 3.5, 1.8 Hz, 1H, H-2C), 5.08 (d, *J*_{1D,2D} = 8.2 Hz, 1H, H-1D), 5.04 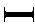 4.99 (m, 2H, H-1B, C*H*HPh), 4.97 (d, *J*_{1A,2A}= 1.5 Hz, 1H, H-1A), 4.94 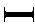 4.79 (m, 7H, H-1C, C*H*HPh), 4.77 (d, *J =* 12.1 Hz, 1H, C*H*HPh), 4.68 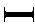 4.48 (m, 11H, H-3D, C*H*HPh), 4.39 (dd, *J =* 10.6, 4.5 Hz, 1H, H-6aD), 4.34 (d, *J=* 12.1 Hz, 1H, C*H*HPh), 4.23 (dd, *J* = 9.7, 3.0 Hz, 1H, H-3A), 4.10 (t, *J* = 9.4 Hz, 1H, H-3E), 4.07-3.94 (m, 7H, H-5E, H-3C, H-5C, C*H*_{2CA}, H-2B, C*H*_{Link}), 3.87 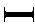 3.77 (m, 4H, H-5A, H-3B, H-4E, H-6bD), 3.73 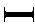 3.43 (m, 10H, H-5B, H-6aE, C*H*_{Link}, H-4D, H-2E, H-5D, H-6bE, H-4A, H-4B, H-2D), 3.39 (td, *J=* 6.9, 1.4 Hz, 2H, C*H*_{*2*Link}), 3.34 (t, *J=* 9.5 Hz, H-4C), 2.59 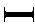 2.33 (m, 4H, 2 × C*H*_{2Lev}), 2.07 (s, 3H, C*H*_{3Ac}), 2.06 (s, 3H, C*H*_{3Lev}), 1.89-1.83 (m, 2H, C*H*_{*2*Link}), 1.26 (d, *J=* 6.2 Hz, 3H, H-6B), 1.20 (d, *J=* 6.2 Hz, 3H, H-6A), 0.75 (d, *J=* 6.1 Hz, 3H, H-6C).

### (3-Azido-1-propyl) 4-O-benzyl-3-O-tert-butyldimethylsilyl-α-L-rhamnopyranosyl-(1→2)-3,4-di-O-benzyl-α-L-rhamnopyranosyl-(1→3)-2-O-acetyl-4-O-(2-naphtylmethyl)-α-L-rhamnopyranosyl-(1→3)-[2,3,4,6-tetra-O-benzyl-α-D-glucopyranosyl-(1→4)]-6-O-benzyl-2-deoxy-2-trichloroacetamido-β-D-glucopyranoside (55).

The title compound was synthesized from pentasaccharide **53** (108 mg, 0.051 mmol, 1.0 equiv.) according to the general procedure for the preparation of acceptors. Purification by flash chromatography (Tol/EtOAc 95:5 to 85:15) gave compound **55** (88 mg, 85 %) as a cristalline solid.

**¹H NMR (400 MHz, CDCl₃) δ (ppm):** 7.85 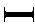 7.74 (m, 5H, N*H*_{TCA}, CH-Ar), 7.527.47 (m, 2H, CH-Ar), 7.42 (dd, *J =* 8.8, 1.6 Hz, 1H, CH-Ar), 7.387.11 (m, 39H, CH-Ar), 5.18 (dd, *J =* 3.5, 1.8 Hz, 1H, H-2C), 5.08 (d, *J*_{1B,2B} = 1.4 Hz, 1H, H-1B), 5.06 (d, *J*_{1A,2A} = 1.3 Hz, 1H, H-1A), 5.03 (d, *J*_{1C,2C} = 1.5 Hz, 1H, H-1C), 4.94 (d, *J =* 11.4 Hz, 1H, C*H*HPh), 4.89 (d, *J =* 10.8 Hz, 1H, C*H*HPh), 4.87 (d, *J =* 11.1 Hz, 1H, C*H*HPh), 4.84 (d, *J =* 10.8 Hz, 1H, C*H*HPh), 4.81 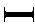 4.77 (m, 4H, H-1E, C*H*HPh), 4.73 (d, *J*_{1D,2D} = 2.1 Hz, 1H, H-1D), 4.67 (d, *J* = 11.9 Hz, 1H, C*H*HPh), 4.64 (d, *J =* 11.4 Hz, 1H, C*H*HPh), 4.60 (d, *J =* 12.7 Hz, 1H, C*H*HPh), 4.54 (d, *J =* 11.3 Hz, 1H, C*H*HPh), 4.53 (d, *J* = 11.7 Hz, 1H, C*H*HPh), 4.52-4.46 (m, 5H, C*H*HPh), 4.45 (d, *J* = 10.7 Hz, 1H, C*H*HPh), 4.26 (d, *J =* 12.1 Hz, 1H, C*H*HPh), 4.23-4.21 (m, 1H, H-2D), 4.14-4.04 (m, 4H, H-5D, H-3C, H-2B), 3.99 (br s, 2H, H-3D, H-4D), 3.96 (t, *J =* 3.7 Hz, 1H, H-2A), 3.94 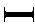 3.67 (m, 10H, H-6aD, H-3E, C*H*_{Link}, H-3B, H-5A, H-5E, H-5B, H-6bD, H-5C, H-4E), 3.64 (dd, *J =* 10.8, 2.7 Hz, 1H, H-6aE), 3.57 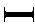 3.36 (m, 7H, H-2E, C*H*_{Link}, H-4C, H-4B, C*H*_{2Link}, H-6bE), 3.34 (t, *J =* 9.2 Hz, 1H, H-4A), 2.17 (s, 3H, C*H*_{3Ac}), 1.90 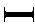 1.83 (m, 2H, C*H*_{*2*Link}), 1.28 (d, *J =* 6.2 Hz, 3H, H-6B), 1.21 (d, *J =* 6.2 Hz, 3H, H-6C), 1.06 (d, *J =* 6.3 Hz, 3H, H-6A), 0.97 (s, 9H, C(C*H*₃)₃), 0.15 (s, 3H, C*H*₃Si), 0.13 (s, 3H, C*H*₃Si).

### (2-Azido-1-ethyl) 4-O-benzyl-3-O-tert-butyldimethylsilyl-α-L-rhamnopyranosyl-(1→2)-3,4-di-O-benzyl-α-L-rhamnopyranosyl-(1→3)-[2,3,4,6-tetra-O-benzyl-α-D-glucopyranosyl-(1→4)]-2-O-chloroacetyl-α-L-rhamnopyranosyl-(1→3)-4,6-O-benzylidene-2-deoxy-2-trichloroacetamido-β-D-glucopyranoside (56).

The title compound was synthesized from pentasaccharide **51** (133 mg, 0.066 mmol, 1.0 equiv.) according to the general procedure for the preparation of acceptors. Purification by flash chromatography (Tol/EtOAc 95:5 to 75:15) gave compound **56** (120 mg, 95 %) as a yellow solid.

**TLC:** R*_{f}* 0.6 (Tol/EtOAc 8:2)

### (3-Azido-1-propyl) [2,3,4,6-tetra-O-benzyl-α-D-glucopyranosyl-(1→3)]-4-O-benzyl-α-L-rhamnopyranosyl-(1→2)-3,4-di-O-benzyl-α-L-rhamnopyranosyl-(1→3)-2-O-acetyl-4-O-(2-naphtylmethyl)-α-L-rhamnopyranosyl-(1→3)-4,6-O-benzylidene-2-deoxy-2-trichloroacetamido-β-D-glucopyranoside (57).

The title compound was synthesized from pentasaccharide **54** (37 mg, 0.018 mmol, 1.0 equiv.) according to the general procedure for the preparation of acceptors. Purification by flash chromatography (Tol/EtOAc 95:5 to 8:2) gave compound **57** (30 mg, 87 %) as a cristalline yellow solid.

**¹H NMR (400 MHz, CDCl₃) δ (ppm):** 7.87-7.84 (m, 3H, C*H*-Ar), 7.72 (s, 1H, C*H*-Ar), 7.53-7.47 (m, 4H, C*H*-Ar), 7.40-7.14 (m, 41H, C*H*-Ar), 7.09 (d, *J =* 7.3 Hz, 1H, N*H*_{TCA}), 5.57 (s, 1H, H-7D), 5.18 (dd, *J =* 3.6, 1.8 Hz, H-2C), 5.16 (s, 1H, H-1A), 5.06 (d, *J*_{1D,2D} = 8.2 Hz, H-1D), 5.02 (s, 1H, H-1B), 4.97 (d, *J =* 11.0 Hz, 1H, C*H*HPh), 4.94-4.81 (m, 7H, H-1E, H-1C, C*H*HPh), 4.76-4.45 (m, 11H, H-3D, C*H*HPh), 4.39 (dd, *J =* 10.4, 4.6 Hz, 1H, H-6aD), 4.32 (d, *J =* 12.2 Hz, 1H, C*H*HPh), 4.08-3.94 (m, 8H, H-2B, H-3C, H-3E, H-2A, H-3A, H-5C, C*H*_{Link}, H-5E), 3.87-3.55 (m, 9H, H-5A, H-3B, H-6bD, H-4E, H-5B, H-4D, C*H*_{Link}, H-2E, H-5D), 3.53-3.33 (m, 8H, H-6aE, H-4A, H-2D, H-4B, H-6bE, C*H*_{*2*Link}, H-4C), 2.07 (s, 3H, C*H*_{3Ac}), 1.89-1.83 (m, 2H, C*H*_{*2*Link}), 1.28 (d, *J =* 6.3 Hz, 3H, H-6B), 1.21 (d, *J =* 6.2 Hz, 3H, H-6A), 0.75 (d, *J =* 6.1 Hz, 3H, H-6C).

### Starting from decasaccharides

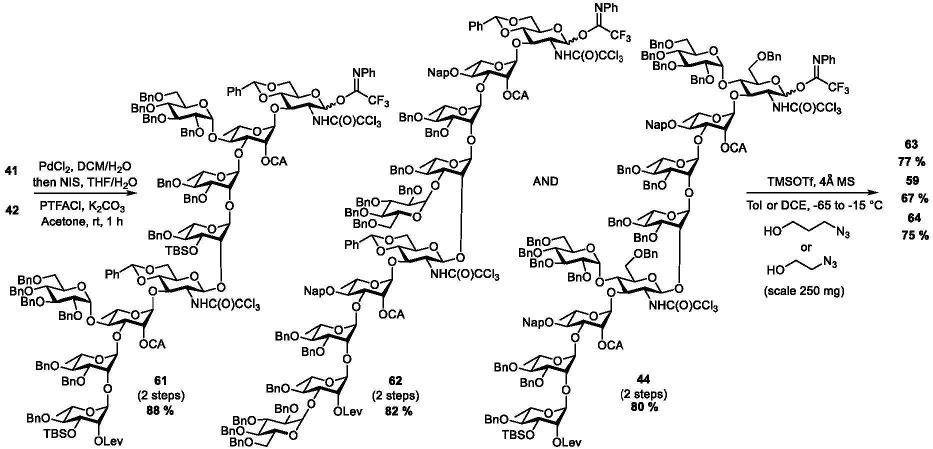

### Synthesis of azidopropyl-equipped pentadecasaccharide 67 (1a/1b)

### Starting from pentadecasaccharide 47 (1b)

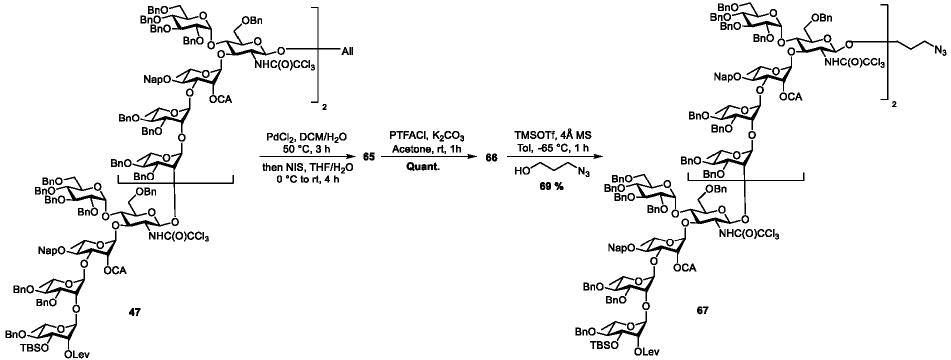

### Synthesis of azidoethyl-equipped pentadecasaccharide 70 (2a)

### Starting from azidoethyl-equipped pentasaccharide 51 (2a)

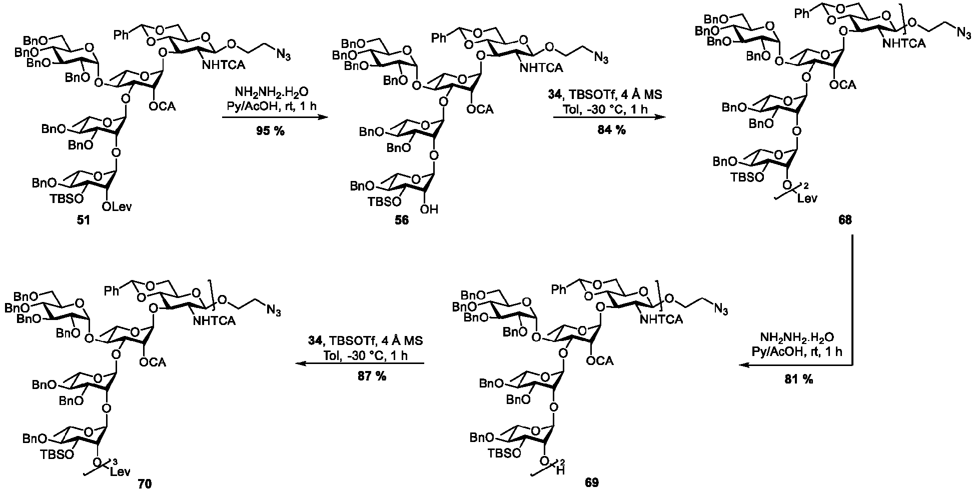

### Starting from decasaccharide 40 (2a)

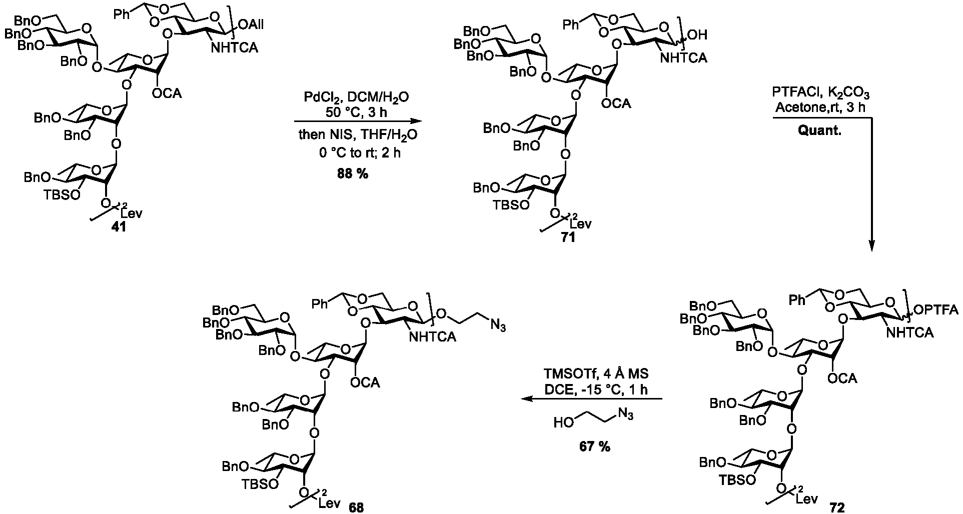

### Starting from pentadecasaccharide 48 (2a)

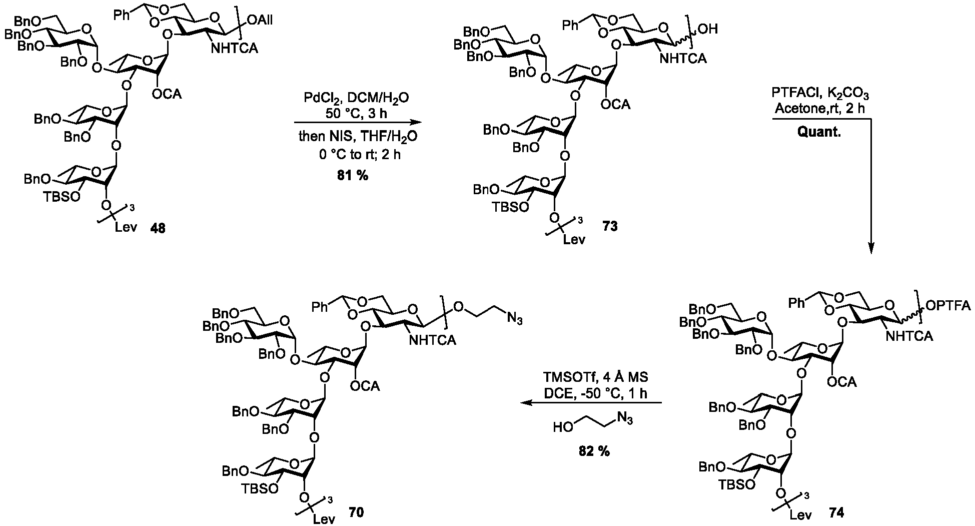

### Synthesis of azidopropyl-equipped pentadecasaccharide 79 (3a)

### Starting from pentasaccharide 52 (3a)

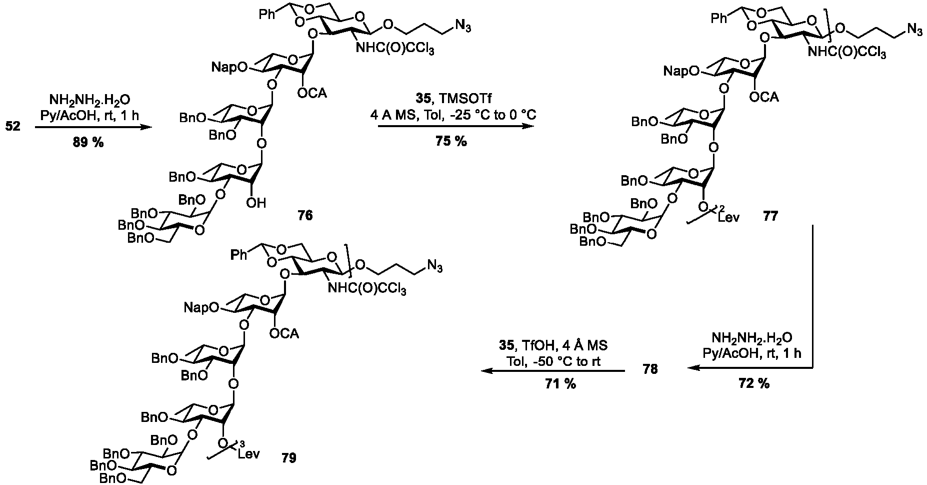

### (3-Azido-1-propyl) [2,3,4,6-tetra-O-benzyl-α-D-glucopyranosyl-(1→3)]-4-O-benzyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranoside (76).

The title compound was synthesized from pentasaccharide **52** (100 mg, 0.049 mmol, 1.0 equiv.) according to the general procedure for the preparation of acceptors. Purification by flash chromatography (Tol/EtOAc 95:5 to 8:2) gave compound **76** (84.5 mg, 89 %) as a cristalline solid.

**¹H NMR (400 MHz, CDCl₃) δ (ppm):** 7.86-7.83 (m, 3H, C*H*-Ar), 7.71 (s, 1H, C*H*-Ar), 7.53-7.46 (m, 4H, C*H*-Ar), 7.39-7.13 (m, 37H, C*H*-Ar), 7.01 (d, *J =* 7.5 Hz, 1H, N*H*_{TCA}), 5.56 (s, 1H, H-7D), 5.23 (dd, *J* = 3.3, 1.8 Hz, H-2C), 5.16 (s, 1H, H-1A), 5.04 (d, *J*_{1D,2D} = 4.8 Hz, H-1D), 5.03 (d, *J =* 1.5 Hz, H-1B), 4.96 (d, *J =* 11.1 Hz, 1H, C*H*HPh), 4.92 (d, *J* = 11.1 Hz, 1H, C*H*HPh), 4.89 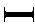 4.83 (m, 6H, H-1E, H-1C, C*H*HPh), 4.74 (d, *J =* 10.7 Hz, 1H, C*H*HPh), 4.69 (d, *J =* 11.2 Hz, 1H, C*H*HPh), 4.66 (d, *J =* 10.7 Hz, 1H, C*H*HPh), 4.61 (d, *J =* 10.6 Hz, 1H, C*H*HPh), 4.58 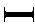 4.48 (m, 6H, H-3D, C*H*HPh), 4.39 (dd, *J =* 10.6, 4.8 Hz, 1H, H-6aD), 4.32 (d, *J =* 11.9 Hz, 1H, C*H*HPh), 4.09 (dd, *J =* 9.5, 3.1 Hz, 1H, H-3C), 4.05 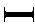 3.93 (m, 9H, H-3E, H-2B, C*H*_{2CA}, H-2A, H-3A, H-5C, C*H*_{Link}, H-5E), 3.84-3.77 (m, 3H, H-5A, H-3B, H-6bD), 3.75 (t, *J =* 9.7 Hz, 1H, H-4E), 3.69 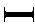 3.55 (m, 5H, H-5B, H-4D, C*H*_{Link}, H-2E, H-5D), 3.52 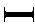 3.43 (m, 4H, H-4A, H-6aE, H-2D, H-4B), 3.42 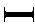 3.38 (m, 3H, H-6bE, C*H*_{*2*Link}), 3.35 (t, *J =* 9.5 Hz, H-4C), 1.90 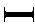 1.83 (m, 2H, C*H*_{*2*Link}), 1.28 (d, *J =* 6.3 Hz, 3H, H-6B), 1.19 (d, *J* = 6.2 Hz, 3H, H-6A), 0.72 (d, *J =* 6.2 Hz, 3H, H-6C).

### Starting from decasaccharide 42 (3a)

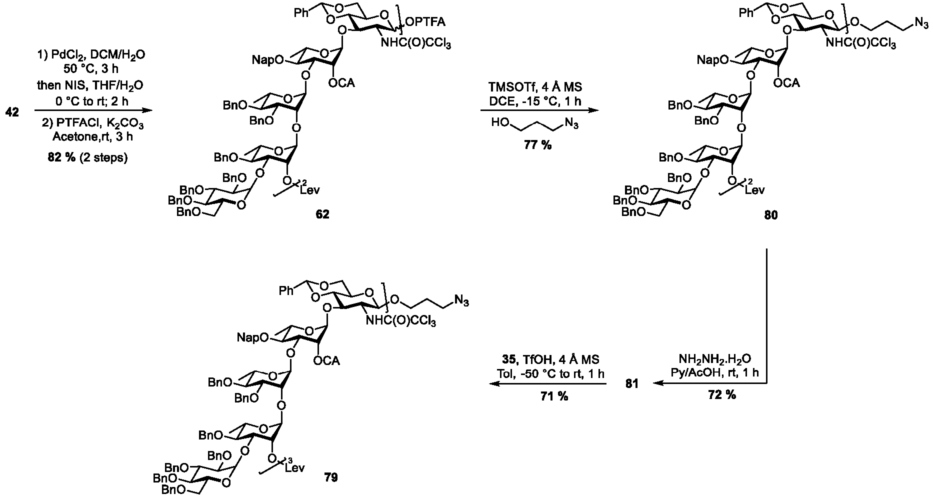

### Starting from pentadecasaccharide 49 (3a)

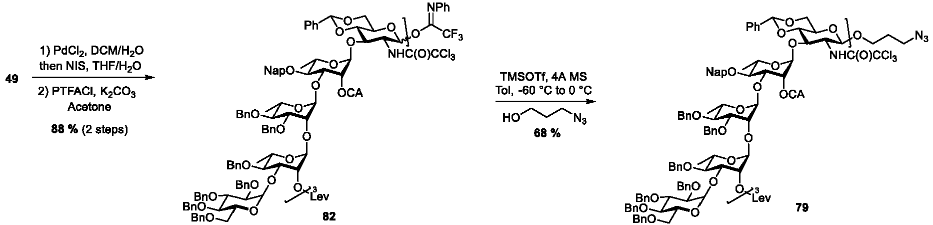

### Example 16: Synthesis of heterooligosaccharides (or chimeric oligosaccharides)

### Chloroacetyl conversion into acetyl of pentasaccharides 1b and 3a

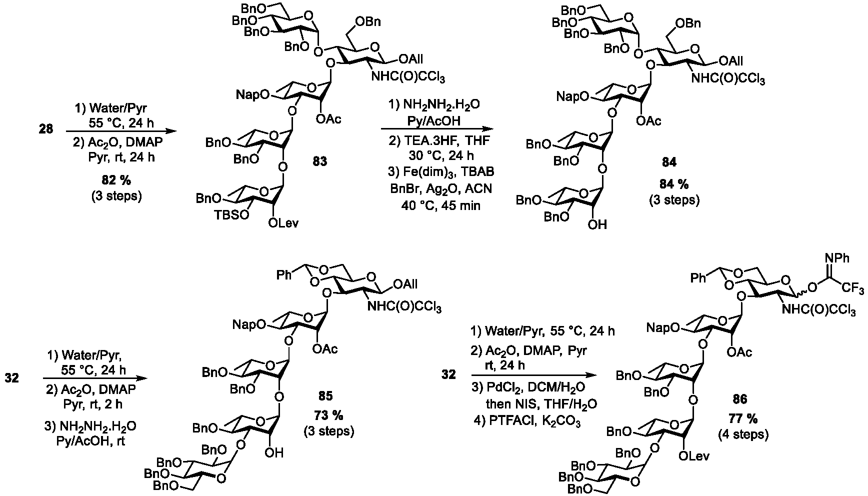

### Allyl 3,4-di-O-benzyl-α-L-rhamnopyranosyl-(1→2)-3,4-di-O-benzyl-α-L-rhamnopyranosyl-(1→3)-2-O-acetyl-4-O-(2-naphtylmethyl)-α-L-rhamnopyranosyl-(1→3)-[2,3,4,6-tetra-O-benzyl-α-D-glucopyranosyl-(1→4)]-6-O-benzyl-2-deoxy-2-trichloroacetamido-β-D-glucopyranoside (84).

To a solution of compound 83 (1000 mg, 0.481 mmol, 1.0 equiv.) in a mixture of Pyr./AcOH (1:1, 14 mL) stirred at 0 °C under Ar, was added hydrazine monohydrate (50-60 %, 51 µL, 0.577 mmol, 1.2 equiv.). The mixture was stirred for 2 h while being gradually warmed to rt. Following addition of EtOAc (100 mL), the organic phase was washed with a solution of CuSO₄ (3 × 20 mL). Then, the combined organic phases were washed with water (40 mL), brine (40 mL) and dried over Na₂SO₄. The filtrate was concentrated under reduced pressure. The crude residue was dissolved in anhydrous THF (31 mL) and stirred at rt under Ar. TEA.3HF (4.7 mL, 28.836 mmol, 60 equiv.). was added and the reaction mixture was stirred at 30 °C overnight. The suspension was cooled to rt, diluted with AcOEt (100 mL) and transferred into a separatory funnel. The organicphase was washed with saturated NaHCO₃ (30 mL), with water (30 mL) and brine (30 mL), dried over Na₂SO₄ and concentrated to dryness. The obtained residue was solubilized in anhydrous ACN (18 mL) and the mixture was stirred at rt under Ar. Iron-dibm complex (93.0 mg, 0.179 mmol, 0.4 equiv.), TBAB (43 mg, 0.134 mmol, 0.3 equiv.), Ag₂O (104 mg, 0.448 mmol, 1.0 equiv.) and BnBr (107 µL, 0.896 mmol, 2.0 equiv.) were added to the suspension and the reaction was allowed to stirr at 40 °C for 45 min.

The resulting mixture was cooled down to rt and diluted with DCM, filtered through Celite and concentrated to dryness. The crude was finally purified by flash chromatography (Tol/EtOAc 95:5 to 85:15) to give compound **36a** (791 mg, 90 % over 3 steps), as a yellow solid.

**¹H NMR (400 MHz, CDCl₃) δ (ppm):** 7.85-7.81 (m, 4H, C*H*-Ar), 7.76 (s, 1H, C*H*-Ar), 7.52 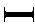 7.47 (m, 2H, C*H*-Ar), 7.42 (dd, *J =* 8.4, 1.5 Hz, 1H, C*H*-Ar), 7.39 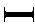 7.21 (m, 35H, *CH-*Ar), 7.197.11 (m, 5H, C*H*-Ar), 5.99 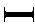 5.89 (m, 1H, H-2_{All}), 5.34 (ddd, *J =* 17.3, 3.8, 1.6 Hz, 1H, H-3a_{All}), 5.24 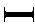 5.20 (m, 2H, H-2C, H-3b_{All}), 5.09 (d, *J*_{1B,2B} = 1.6 Hz, 1H, H-1B), 5.05 (br s, 2H, H-1A, H-1C), 4.96 (d, *J* = 11.6 Hz, 1H, C*H*HPh), 4.91 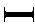 4.78 (m, 8H, H-1E, H-1D, C*H*HPh), 4.69-4.44 (m, 12H, C*H*HPh), 4.29 (ddt, *J* = 12.9, 5.0, 1.5 Hz, 1H, H-1a_{All}), 4.27 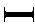 4.23 (m, 2H, H-2D, C*H*HPh), 4.18 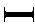 4.14 (m, 1H, H-5D), 4.11 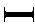 4.01 (m, 6H, H-2A, H-3C, H-1b_{All}, H-6aD, H-2B, H-3D), 3.93 (t, *J =* 9.3 Hz, 1H, H-3E), 3.85 (dd, *J* = 8.8, 2.8 Hz, 1H, H-3B), 3.82 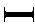 3.68 (m, 7H, H-3A, H-5A, H-5B, H-5E, H-5C, H-6bD, H-4E), 3.66 (dd, *J=* 10.7, 2.7 Hz, 1H, H-6aE), 3.56 (dd, *J =* 10.5, 3.7 Hz, 1H, H-2E), 3.53 (t, *J =* 9.3 Hz, 1H, H-4C), 3.44 (t, *J =* 9.2 Hz, 1H, H-4B) , 3.43 (t, *J =* 9.3 Hz, 1H, H-4A), 3.38 (dd, *J =* 10.8, 1.6 Hz, 1H, H-6bE), 2.39 (d, *J =* 1.7 Hz, 1H, O*H*), 2.18 (s, 3H, C*H*_{3Ac}), 1.31 (d, *J =* 6.2 Hz, 3H, H-6B*), 1.21 (d, *J =* 6.1 Hz, 3H, H-6C), 1.08 (d, *J =* 6.2 Hz, 3H, H-6A*).

### Allyl [2,3,4,6-tetra-O-benzyl-α-D-glucopyranosyl-(1→3)]-4-O-benzyl-α-L-rhamnopyranosyl-(1→2)-3,4-di-O-benzyl-α-L-rhamnopyranosyl-(1→3)-2-O-acetyl-4-O-(2-naphtylmethyl)-α-L-rhamnopyranosyl-(1→3)-4,6-O-benzylidene-2-deoxy-2-trichloroacetamido-β-D-glucopyranoside (85).

The title compound was synthesized from pentasaccharide **32** (224 mg, 0.112 mmol, 1.0 equiv.) according to the general procedure for the preparation of acceptors.

### [2,3,4,6-tetra-O-benzyl-α-D-glucopyranosyl-(1→3)]-4-O-benzyl-2-O-levulinoyl-α-L-rhamnopyranosyl-(1→2)-3,4-di-O-benzyl-α-L-rhamnopyranosyl-(1→3)-2-O-acetyl-4-O-(2-naphtylmethyl)-α-L-rhamnopyranosyl-(1→3)-4,6-O-benzylidene-2-deoxy-2-trichloroacetamido-β-D-glucopyranosyl (N-phenyl)trifluoroacetimidate (86).

The title compound was synthesized from pentasaccharide **32** (500 mg, 0.520 mmol, 1.0 equiv.) according to the general procedure for PTFA activation.

### Heterocoupling of donor 2a/acceptor 1b

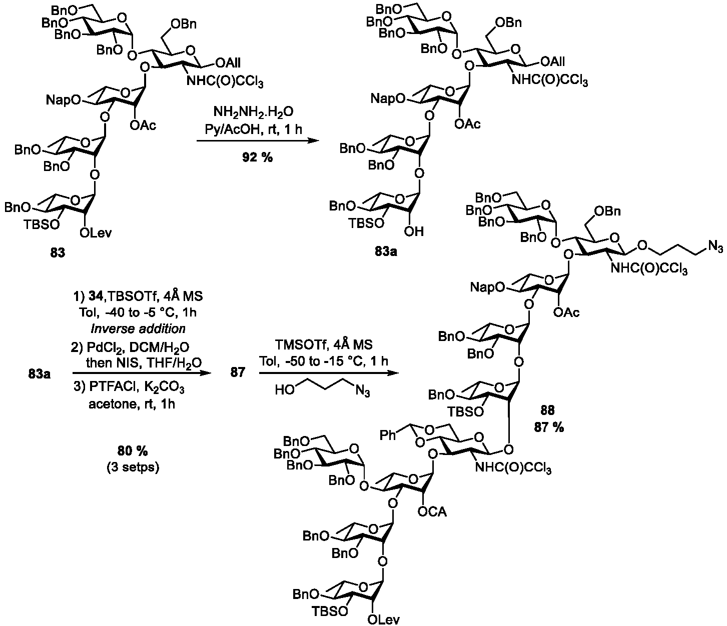

### Allyl 4-O-benzyl-3-O-tert-butyldimethylsilyl-α-L-rhamnopyranosyl-(1→2)-3,4-di-O-benzyl-α-L-rhamnopyranosyl-(1→3)-2-O-acetyl-4-O-(2-naphtylmethyl)-α-L-rhamnopyranosyl-(1→3)-[2,3,4,6-tetra-O-benzyl-α-D-glucopyranosyl-(1→4)]-6-O-benzyl-2-deoxy-2-trichloroacetamido-β-D-glucopyranoside (83a).

The title compound was synthesized from pentasaccharide **28** (450 mg, 0.213 mmol, 1.0 equiv.) according to the general procedure for the preparation of acceptors.

**(3-Azido-1-propyl) 4-*O*-benzyl-3-*O*-*tert*-butyldimethylsilyl-2-*O*-levulinoyl-*α*-L-rhamnopyranosyl-(1→2)-3,4-di-*O*-benzyl-*α*-L-rhamnopyranosyl-(1→3)-[2,3,4,6-tetra-*O-*benzyl-α-D-glucopyranosyl-(1→4)]-2-*O*-chloroacetyl-*α*-L-rhamnopyranosyl-(1→3)-4,6-*O*-benzylidene-2-deoxy-2-trichloroacetamido-*β*-D-glucopyranosyl-(1→2)-4-*O*-benzyl-3-*O*-*tert*-butyldimethylsilyl-*α*-L-rhamnopyranosyl-(1→2)-3,4-di-*O*-benzyl-*α*-L-rhamnopyranosyl-(1→3)-2-*O*-acetyl-4-*O*-(2-naphtylmethyl)-*α*-L-rhamnopyranosyl-(1→3)-[2,3,4,6-tetra-*O*-benzyl-α-D-glucopyranosyl-(1→4)]-6-*O*-benzyl-2-deoxy-2-trichloroacetamido-*β*-D-glucopyranoside (88).**

The title compound was synthesized from pentasaccharide **32** (224 mg, 0.112 mmol, 1.0 equiv.) according to the general procedure for the preparation of acceptors.

### Heterocoupling of donor 3a/acceptor 1b

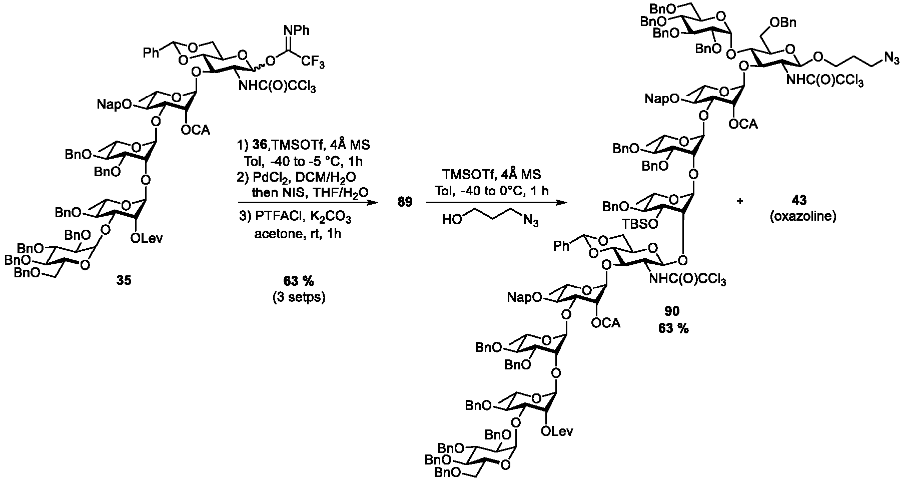

### (3-Azido-1-propyl) [2,3,4,6-tetra-O-benzyl-α-D-glucopyranosyl-(1→3)]-4-O-benzyl-2-O-levulinoyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-4,6-O-benzylidene-2-deoxy-2-trichloroacetamido-β-D-glucopyranosyl-(1→2)-4-O-benzyl-3-O-tert-butyldimethylsilyl-α-L-rhamnopyranosyl-(1→2)-3,4-di-O-benzyl-α-L-rhamnopyranosyl-(1→3)-2-O-chloroacetyl-4-O-(2-naphtylmethyl)-α-L-rhamnopyranosyl-(1→3)-[2,3,4,6-tetra-O-benzyl-α-D-glucopyranosyl-(1→4)]-6-O-benzyl-2-deoxy-2-trichloroacetamido-β-D-glucopyranoside (90).

The title compound was synthesized from pentasaccharide **32** (224 mg, 0.112 mmol, 1.0 equiv.) according to the general procedure for the preparation of acceptors.

### Heterocoupling of donor 2a/acceptor 3a

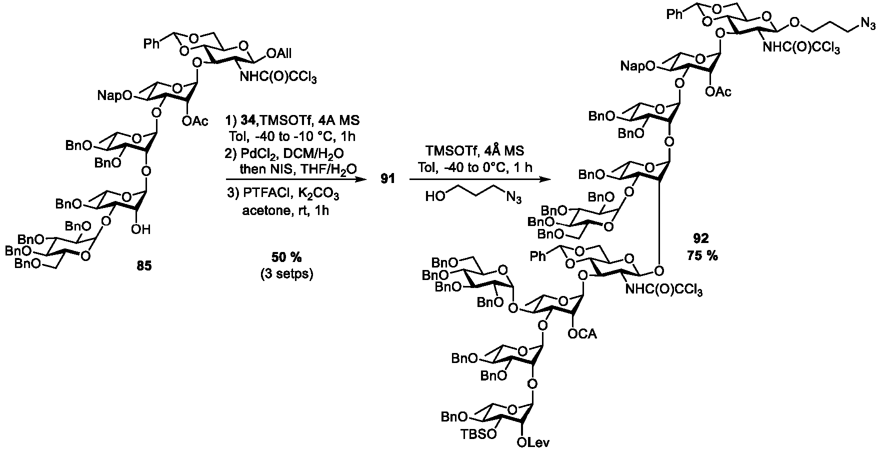

### (3-Azido-1-propyl) 4-O-benzyl-3-O-tert-butyldimethylsilyl-2-O-levulinoyl-α-L-rhamnopyranosyl-(1→2)-3,4-di-O-benzyl-α-L-rhamnopyranosyl-(1→3)-[2,3,4,6-tetra-O-benzyl-α-D-glucopyranosyl-(1→4)]-2-O-chloroacetyl-α-L-rhamnopyranosyl-(1→3)-4,6-O-benzylidene-2-deoxy-2-trichloroacetamido-β-D-glucopyranosyl-(1→2)-[2,3,4,6-tetra-O-benzyl-α-D-glucopyranosyl-(1→3)]-4-O-benzyl-α-L-rhamnopyranosyl-(1→2)-3,4-di-O-benzyl-α-L-rhamnopyranosyl-(1→3)-2-O-acetyl-4-O-(2-naphtylmethyl)-α-L-rhamnopyranosyl-(1→3)-4,6-O-benzylidene-2-deoxy-2-trichloroacetamido-β-D-glucopyranoside (92).

The title compound was synthesized from pentasaccharide **32** (224 mg, 0.112 mmol, 1.0 equiv.) according to the general procedure for the preparation of acceptors.

### Heterocoupling of donor 3a/acceptor 2a

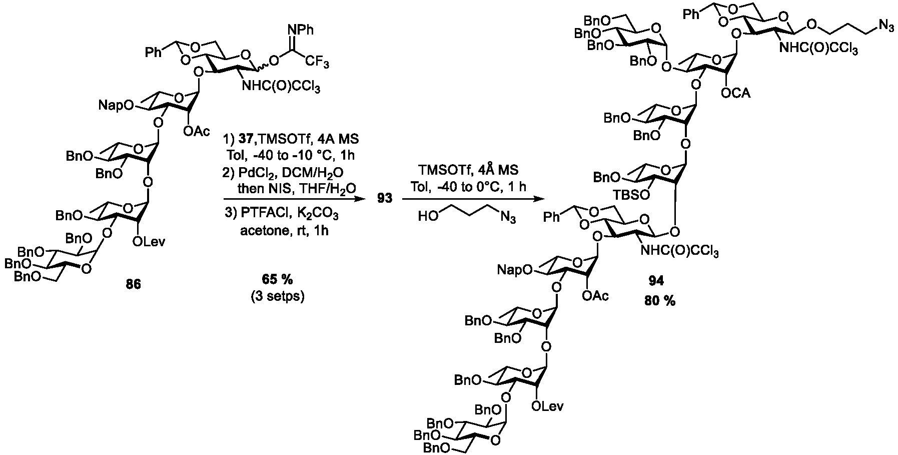

### Heterocoupling of donor 2a2a/acceptor 1b

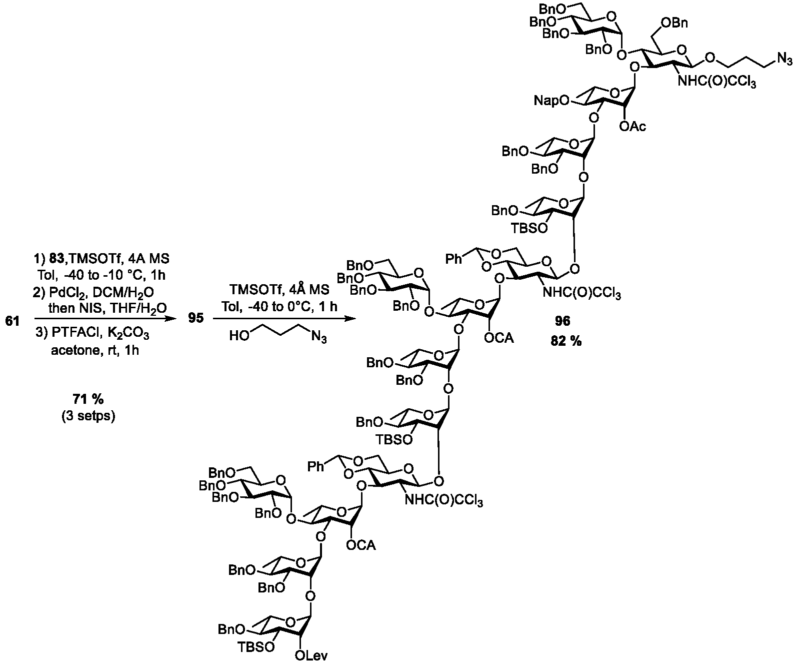

### Heterocoupling of donor 3a3a/acceptor 1b

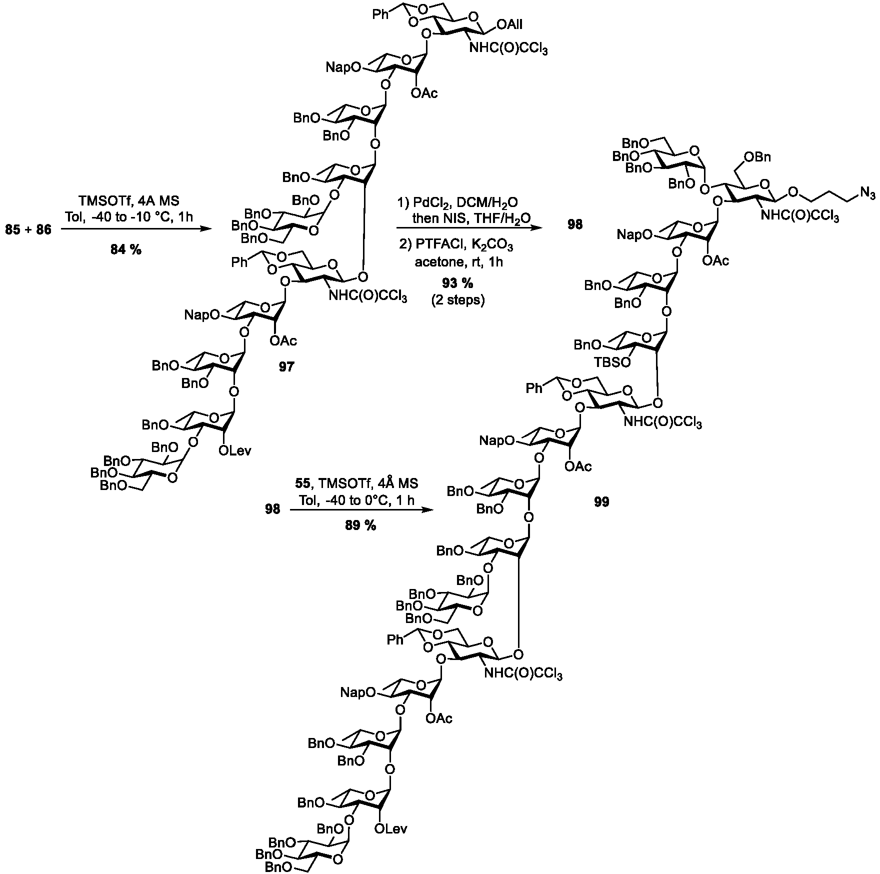

### Heterocoupling of donor 2a2a/acceptor 3a

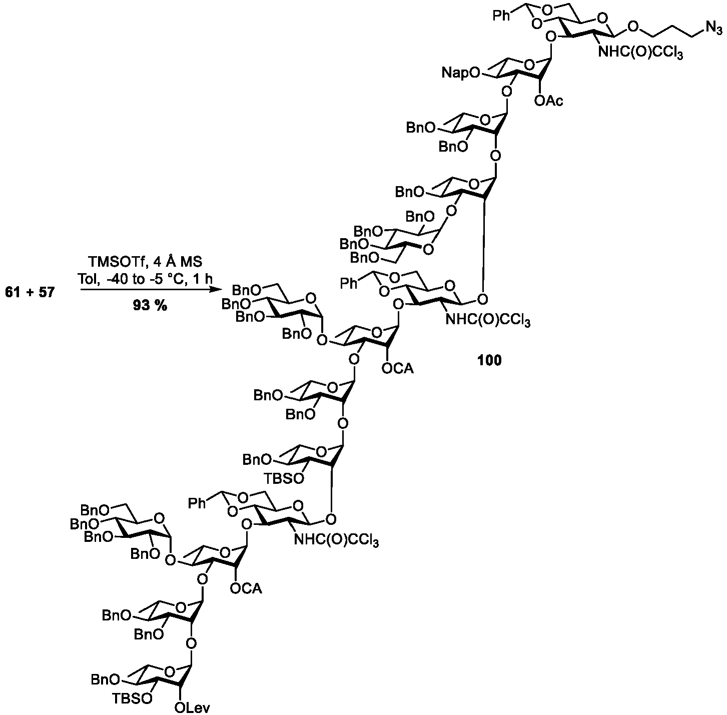

### Heterocoupling of donor 3a/acceptor 2a2a

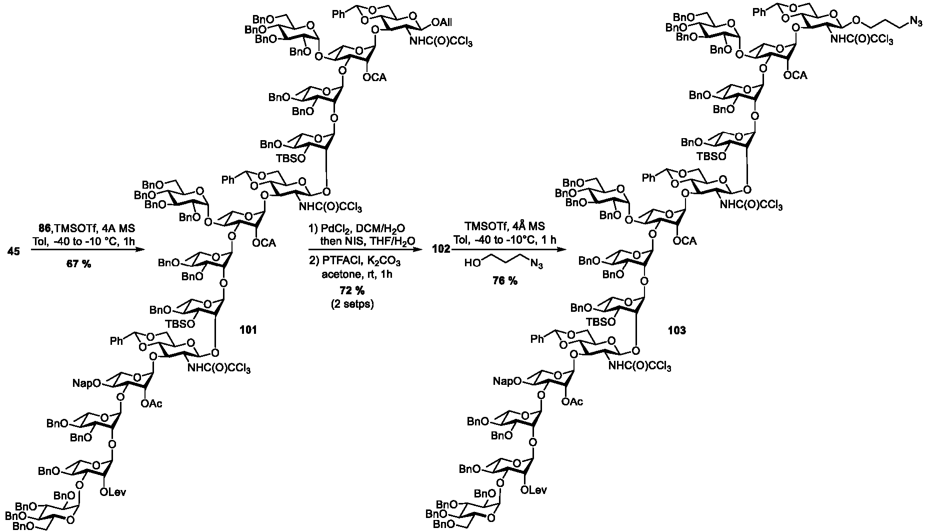

### Heterocoupling of donor 2a2a/acceptor 1b1b

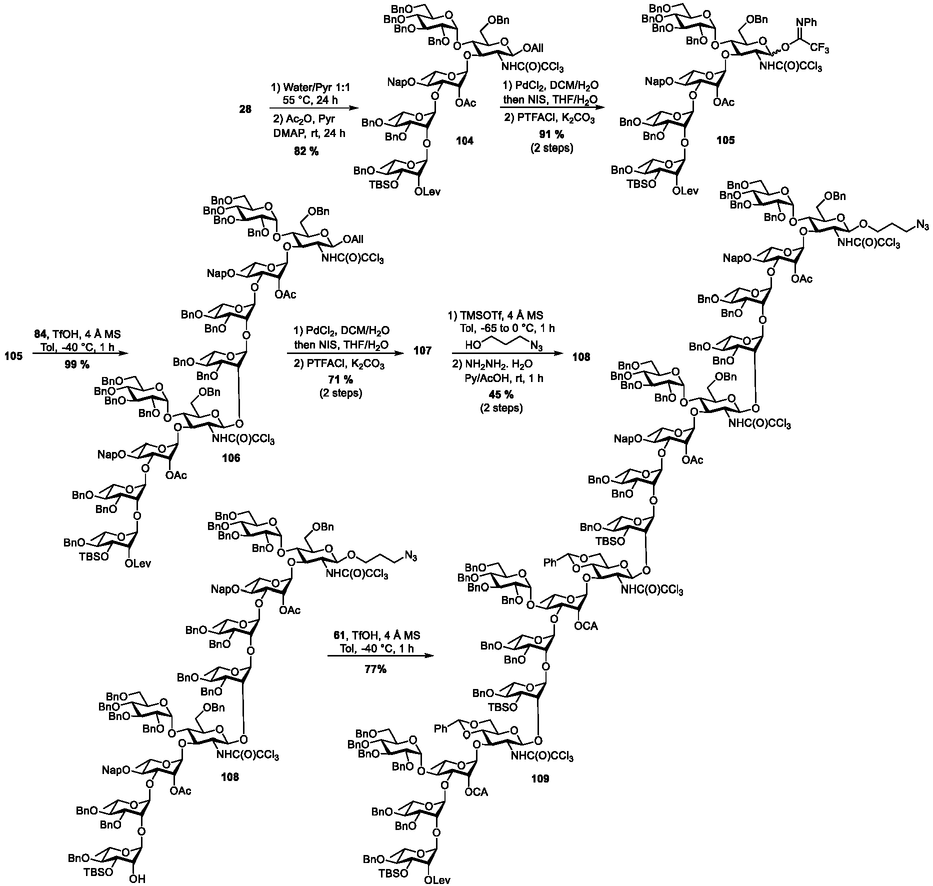

### Heterocoupling of donor 2a2a/acceptor 3a3a

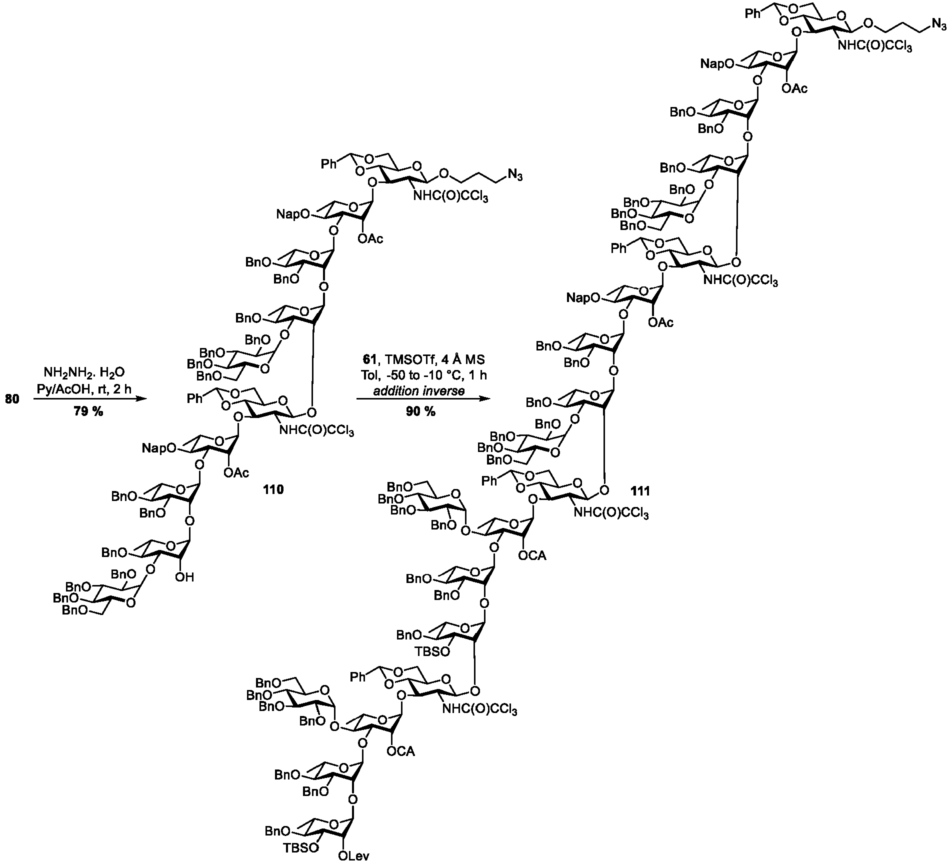

### Heterocoupling of donor 3a3a/acceptor 2a2a

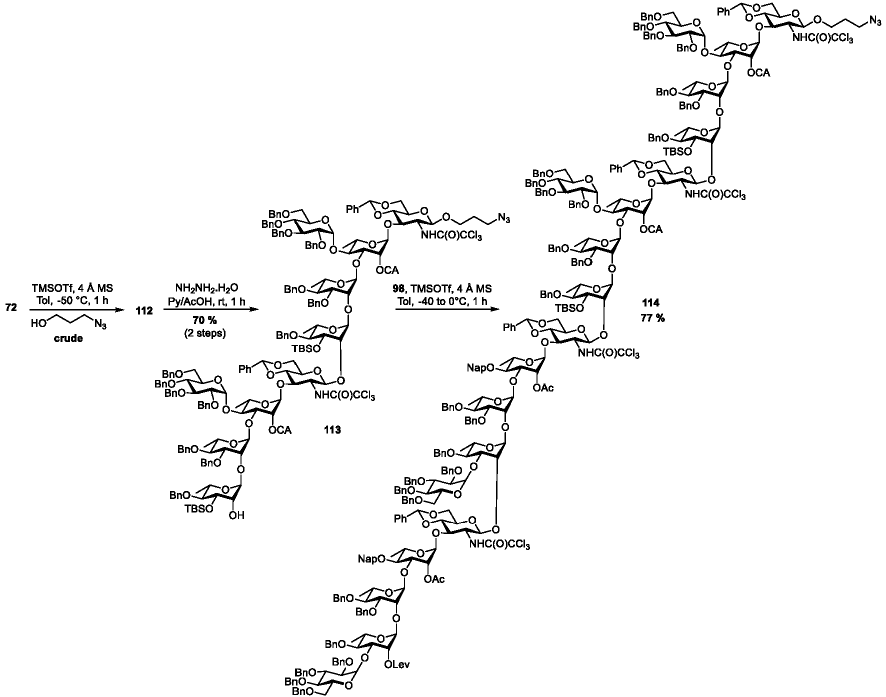

### Heterocoupling of donor 3a/acceptor 2alb

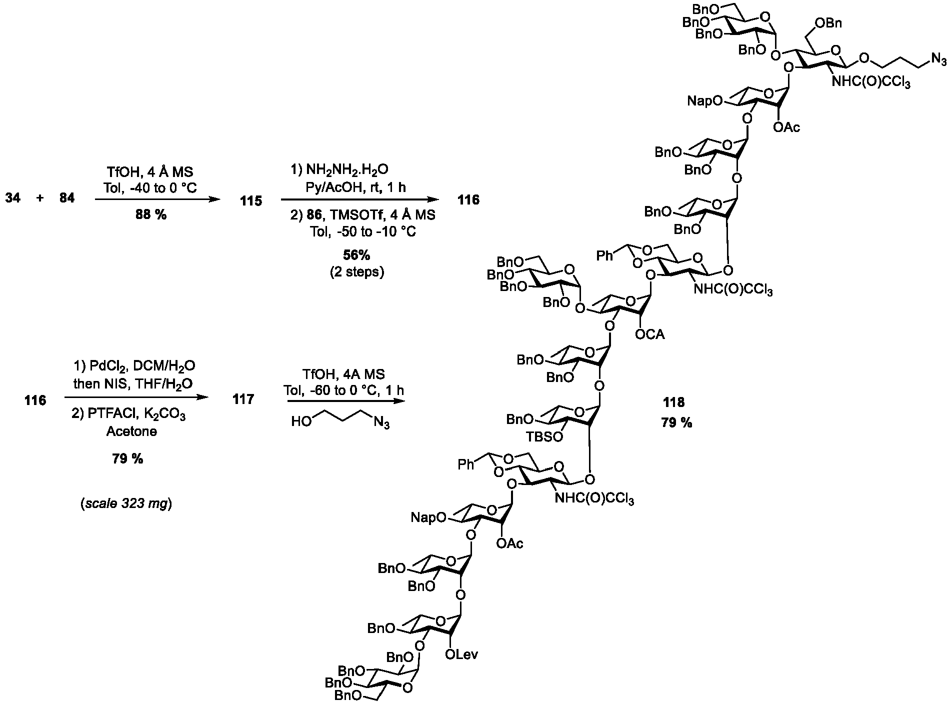

### Heterocoupling of donor 2aY/acceptor 3a

Y, when protected, corresponds to ABCD' as defined above, and to ABCD once deprotected.

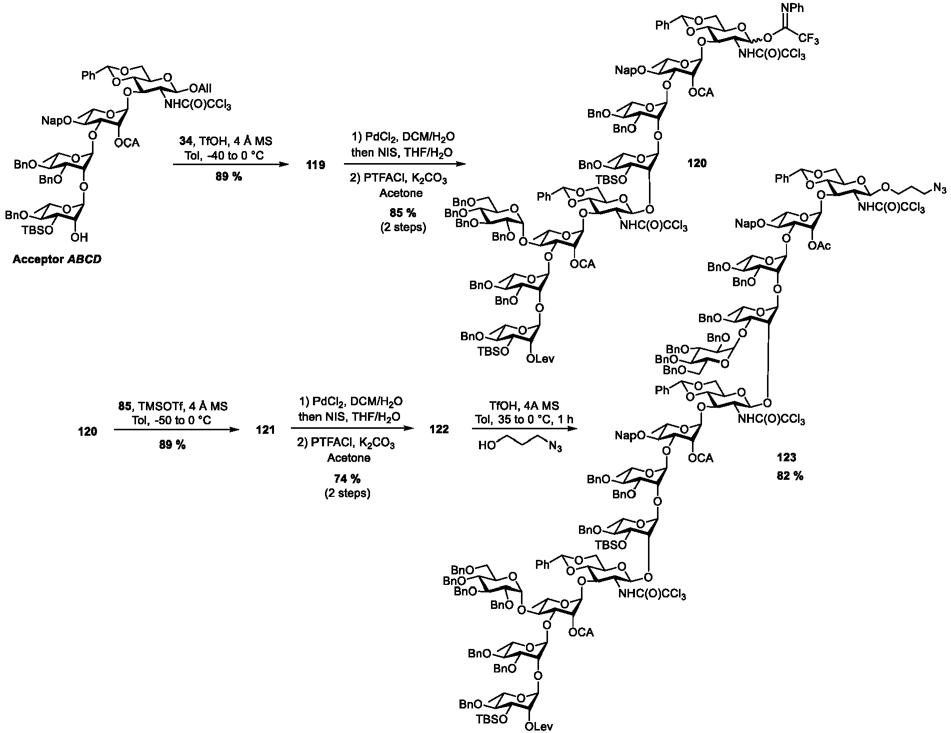

### Heterocoupling of donor 2aY/acceptor 3alb

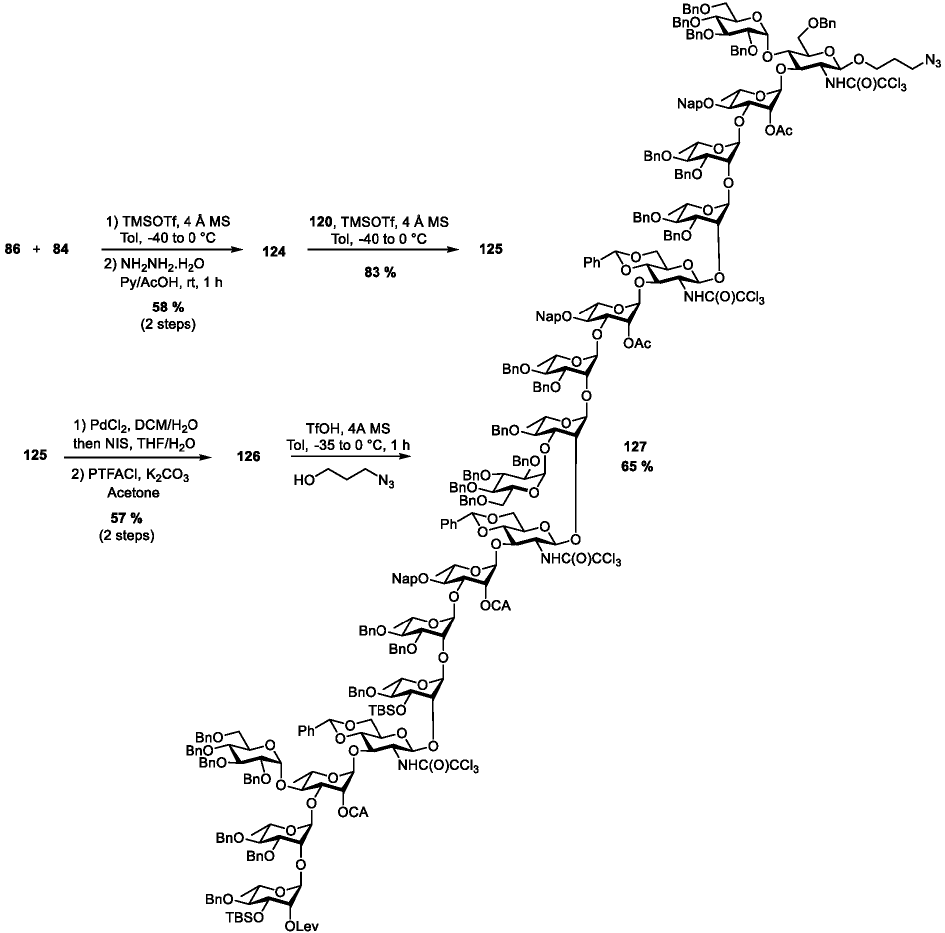

### Heterocoupling of donor 3a/acceptor 2a2alb

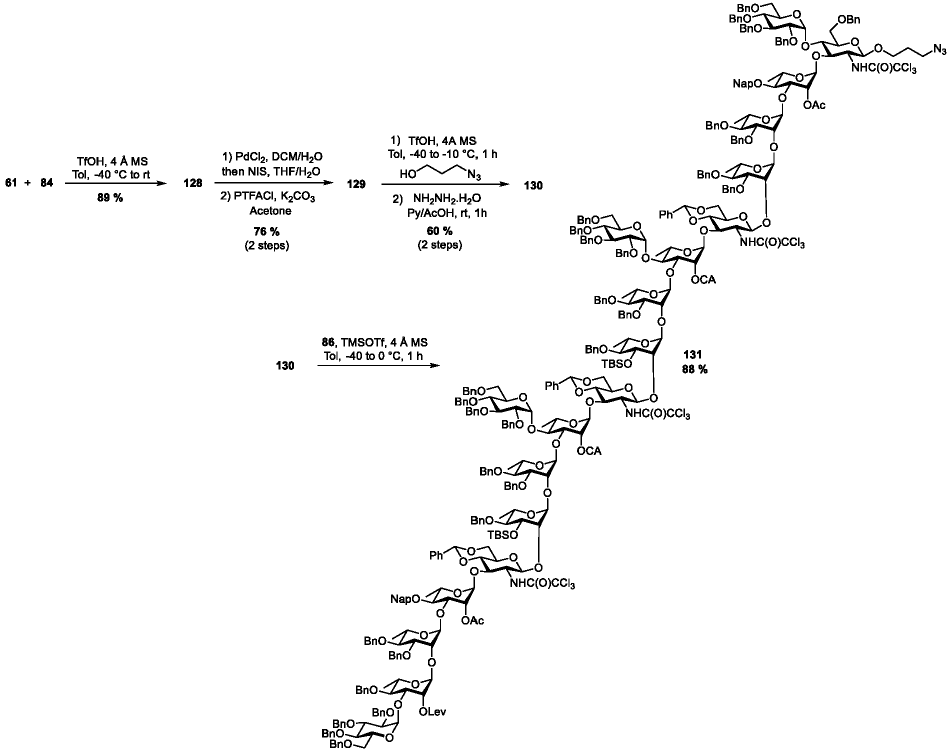

### Heterocoupling of donor 2a2a/acceptor 3a3alb

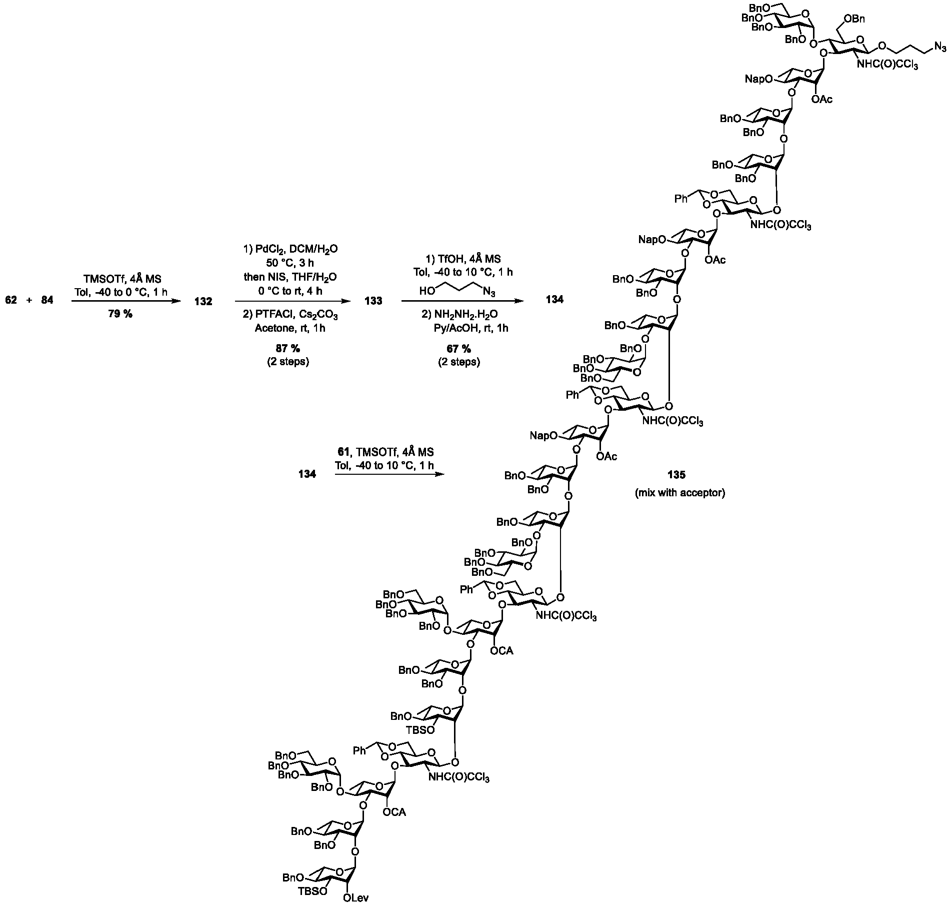

### Example 17: Partial deprotection of homo- and heterooligosaccharides

The partial deprotection of oligomers followed a three-step sequence involved (1) the removal of the levulinoyl group, (2) the cleavage of the chloroacetyl moiety to reveal the hydroxyl group at position 2C if appropriate, and finally, (3) cleavage of the silyl ether. However, the one- or two-step procedure could be applied for oligomers lacking respectively two or one of these three protecting groups in their midst.

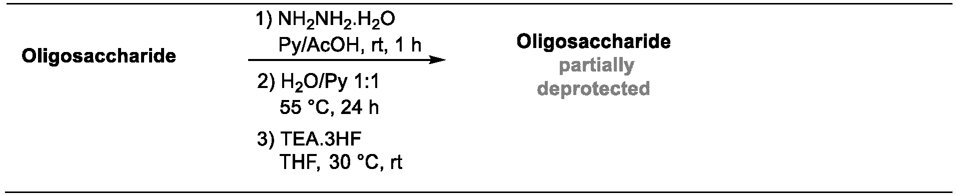

### Example 18: Hydrogenolysis/Hydrogenation

Global deprotection of partially deprotected oligomers was then implemented in order to prepare SF LPS OAg-like oligosaccharides. The strategy consists of a one-step hydrogenolysis/Hydrogenation/hydrodechlorination procedure involving the reduction of azide into the corresponding amine, the conversion of the NHC(O)CCl₃ moiety into the required NHAc, that of the CA if present at position 2c into the corresponding Ac, and the cleavage of the remaining permanent protecting groups (Bn, Nap and Bzl).

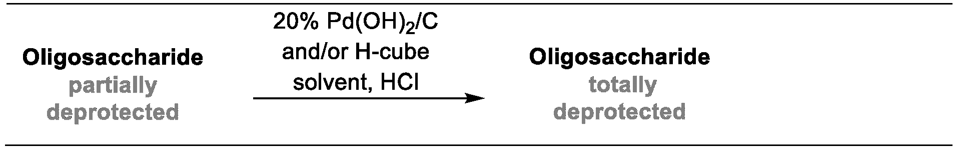

Importantly, Pd black together with HCl were needed for complete deprotection. Also, the presence of azidospacer required the use of HCl in order to protonate the amine formed upon its reduction, as it is known that primary amines poison transition metal catalysts. In some cases, microfluidic continuous flow hydrogenation reactor system (H-Cube) was envisioned. The modulation of some parameters such as solvent systems (DCM₅₀/EtOH₅₀, EtOH₈₀/H₂O₂₀, DCM, MeTHF, Isopropanolso/H₂O₂₀), range of HCl equivalents (0.5 equiv. to 1.0 equiv.) and reaction time may be necessary to induce a complete deprotection by avoiding important side reactions.

### Example 19: Glycoconjugate synthesis

Representative protocol exemplified for the synthesis of the SF1b-TT15 conjugate from the aminopropyl precursor SF1b 15mer-NH₂.

*Conversion of the aminolinker-equipped oligosaccharide into ready-for conjugation oligosaccharide featuring a thiol masked in the form of a pyridyldisulfide*

N-succinimidyl-3-(2-pyridyldithio)propionate (SPDP, Thermofisher, 339 µg, 3.6 µL, 1.5 equiv.) from a stock solution (2.8 mg in 30 µL DMSO) was added to a solution of the SF1b 15mer-NH₂ precursor (1.89 mg, 723 nmol) in phospahte buffer 0.1 M pH 7.4 (400 µL). After stirring for 1 h at rt in the dark, A RP-HPLC and MS monitoring indicated full conversion of the starting material into a less polar product. RP-HPLC purification provided the expected material (1.09 mg, 54%), which had Rₜ (215 nm, conditions A): 10.84 min, HRMS (ESI⁺) *m*/*z* [M+H+Na]²⁺ 1416.50, [M+H+NH₄]²⁺ 1414.02.

### TT modification (Batch for use in different preparations)

SEC purified tetanus toxoid (TT, 150 kDa, 1.0 mL, 6.9 mg, 46 µmol) from the stock solution (6.9 mg/mL, 46 µM) was buffer exchanged with 0.1 M HEPES pH 7.5 four times by passing through a 30 kD Amicon filter and concentrated to give buffer exchanged TT (final concentration 12.9 mg/mL, 520 µL). A solution of (N-[0-maleimidobutyryloxy]succinimide ester (GMBS, 2.06 mg, 160 equiv.) in DMSO (29 µL) was added. Modification was performed at rt for 1 h, stirring gently in the dark. After this time, the entire volume of TT_{Mal} intermediate was buffer exchanged with 0.1 M Phosphate buffer pH 6.3 containing 5 mM EDTA and finally concentrated to 600 µL to reach a final concentration of TT_{Mal} of 11.44 mg/mL (measured by UV).

### Thiol unmasking

TCEP·HCl (83 µg, 1.9 µL, 1.02 equiv.) from a stock solution in DMSO (150 mM, 30 µL) was added over 4 h in three portions to a solution of SF1b 15mer-PDT (800 µg, 0.28 µmol) in 0.1 M Phosphate buffer pH 6.3 containing 5 mM EDTA (200 µL) stirred at rt in the dark. Monitoring by analytical RP-HPLC indicated full conversion of the starting material (Rt (215 nm): 10.96 min) into a more polar product. The corresponding thiol had Rt (215 nm, conditions A): 9.34 min.
Conditions A for analytical RP-HPLC: Kromasil C18 3.5 µm 100 Å, 3x150 mm column eluting at 0.4 mL/min with 0.08% aq. TFA-MeCN 0-40% over 20 min.

### Conjugation step

The remaining solution of the thiol intermediate (0.285 µmol, 201 µL, 32 equiv.) was added to the above solution of TT_{Mal} in 0.1 M Phosphate buffer pH 6.3 containing 5 mM EDTA (1.31 mg, 8.7 nmol, 115 µL). Conjugation was performed at rt for 3.5 h. Finally, cysteamine·HCl was added to cap any residual reactive maleimide groups. For this, cysteamine·HCl (0.38 mg, 3.0 µmol) in desionised water (20 µL, 19 mg/mL) was added. After stirring at rt for 30 min, the mixture was left overnight at 4 °C. The entire volume of conjugate was buffer exchanged with PBS 1X pH 6.0 by passing through a 30 kDa centrifugal filter five times, and finally harvested. The final volume was adjusted to 990 µL. The obtained conjugate was analyzed for conjugation yield and carbohydrate:protein molar ratio, respectively. Data for **SF1b-TT15** were as follows: [protein]: 1.42/1.34 mg/mL (BCA, UV), [carbohydrate]: 497 µg/mL (Anthrone assay), and a glycan:TT molar loading of 21/22 (BCA/Anthrone, UV/anthrone).

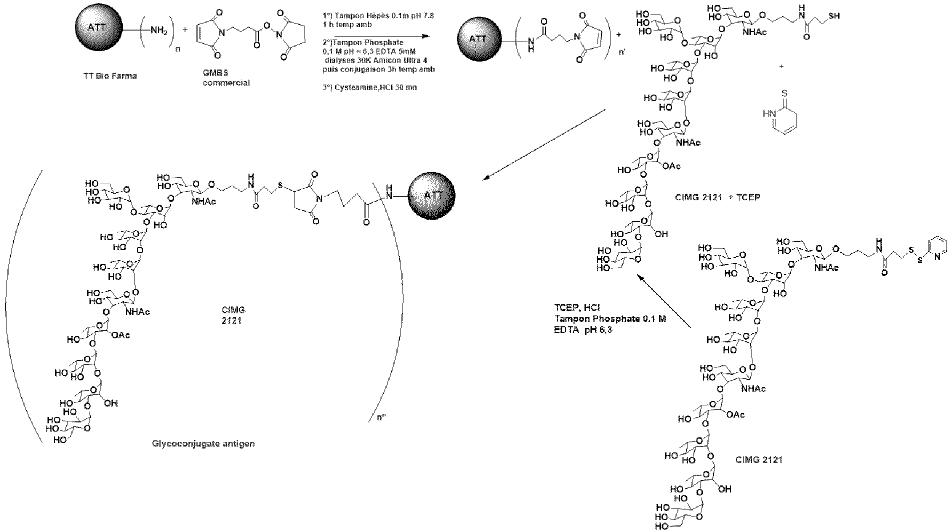

### Example 20: Immunization Assays

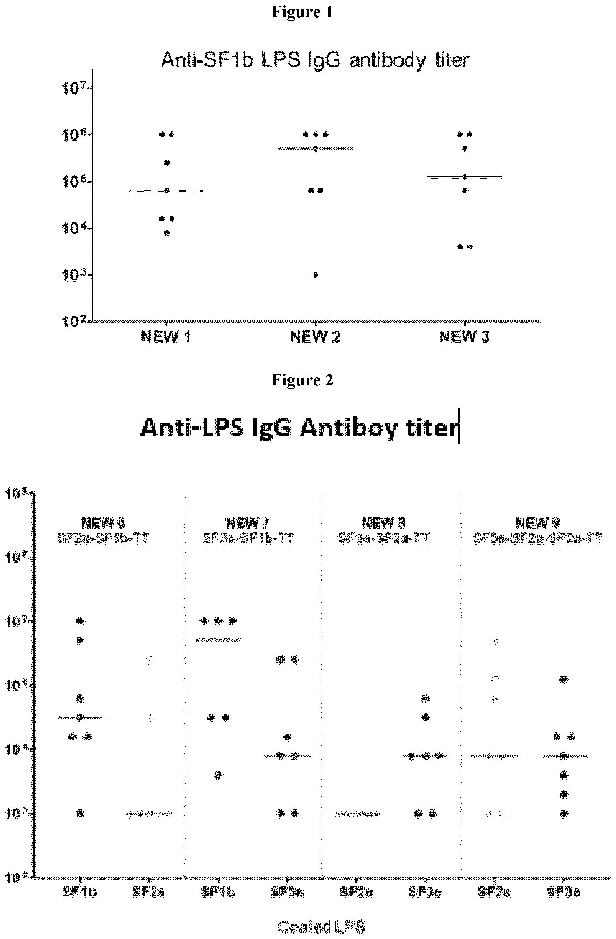

Immunization assays have been performed as follows:
Injection of a total amount of conjugate corresponding to 2µg of SF1b oligosaccharide, adjuvanted with aluminium phosphate (AIP), 3 times at 3 week-interval per mouse. Data are shown after the 3rd immunization (figure 1).

Injection of a total amount of conjugate corresponding to 2µg of oligosaccharide, adjuvanted with aluminium phosphate (AIP), 3 times at 3 week-interval per mouse. Mice were bled 3 weeks after each immunization. Data are shown after the 3rd immunization (figure 2).

### More specifically:

### Mice immunization

For each of the conjugates, seven week-old Balb/c female mice (Janvier Labs, France) were immunized intramuscularly (i.m.) with amounts of conjugates corresponding to 2.0 µg equivalent of total oligosaccharide per dose, adjuvanted with aluminium phosphate (Adju-Phos, InvivoGen, Toulouse, France). Adju-Phos was used at a concentration of 2.8 mg/mL in Tris 20 mM, NaCl 150 mM, pH 7.5 and mixed v/v with a solution of conjugates, resulting in a dose of 140 µg Adju-Phos per mouse/per injection under genltle stirring. After 5 min incubation at rt, 100 µL of the adjuvanted glycoconjugates were injected at two sites (50 µL at each site). Three immunizations were performed at 3 week-interval. Blood samples were recovered three weeks after the third injection. Seven mice were used per group.

### Measurement of the anti-S. flexneri LPS IgG response

The glycoconjugate-induced anti-LPS IgG response specific for each one of the *S. flexneri* 1b (SF1b), *S. flexneri* 2a (SF2a, strain 454), and *S. flexneri* 3a (SF3a) serotypes was measured by ELISA using the homologous *S. flexneri* LPS purified as previously described (Westphal, O., and J. Jann. 1965. Bacterial lipopolysaccharides:extraction with phenol-water and further application of the procedures. Meth. Carbohydr. Chem. 5: 83-91). Briefly, 2.5 µg of purified SF1b, SF2a and SF3a LPS was coated per ELISA plate well in PBS and incubated at 4 °C overnight. After washing the wells with PBS-Tween 20 0.01%, saturation was performed by incubating the plate for 30 min at 37 °C with PBS-BSA 1%. Then, serial dilutions of mouse sera in PBS-BSA 1% were incubated for 1 h at 37 °C. After washing with PBS-Tween 20 0.01%, anti-mouse IgG peroxidase-labeled conjugate (Sigma-Aldrich) was used as secondary antibody at a dilution of 1/5,000. The IgG titer was defined as the last dilution of serum giving rise to twice the OD value obtained with similarly diluted pre-immune serum.

## Claims

1. A tetrasaccharide compound of following formula (O) : 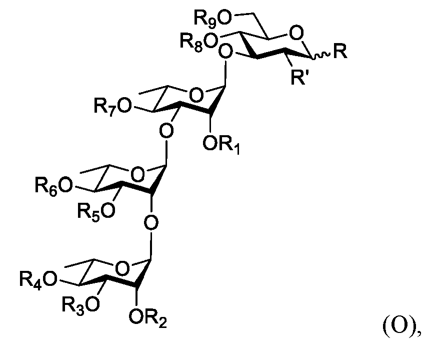 wherein :
R is O-allyl (All), O-triisopropylsilyl (TIPS), O-*tert*-butyldiphenylsilyl (TBDPS), O*-tert*butyldimethylsilyl (TBS or TBDMS), O-thexyl dimethyl silyl (TDS), *para*-methoxyphenyl (PMP) or SR₀, with R₀ being as the compound is a thioglycoside;
R' is NHC(O)CCl₃, NHC(O)CHCl₂, NAc₂, NHTroc with Troc being 2,2,2-trichloroethoxycarbonyl, a carbamate such as NHCbz, with Cbz being carboxybenzyl, NAlloc, with Alloc being allyloxycarbonyl, NHC(O)CF₃, tetrachlorophtalimido, phtalimido, or azido;
R₁ is chloroacetyl (CA), bromoacetyl (BA), or acetyl (Ac), and R₂ is levulinoyl (Lev), fluorenylmethoxycarbonyl (Fmoc), or pentafluorophenyl ester (PFP);
or R₂ is chloroacetyl (CA), bromoacetyl (BA), or acetyl (Ac), and R₁ is levulinoyl (Lev), fluorenylmethoxycarbonyl (Fmoc), or pentafluorophenyl ester (PFP);
R₄, R₅ and R₆ are independently chosen from arylmethyl protecting groups, in particular benzyl (Bn), *para*-chlorobenzyl (PCB), *para*-bromobenzyl (PBB), *para*-nitrobenzyl or *ortho-*nitrobenzyl;
R₃, R₇ and R₈/R₉ are protecting groups that are orthogonal to each other and orthogonal to R, R₁, R₂, R₄, R₅, R₆ and R'.

2. A tetrasaccharide compound of following formula (I₀) : 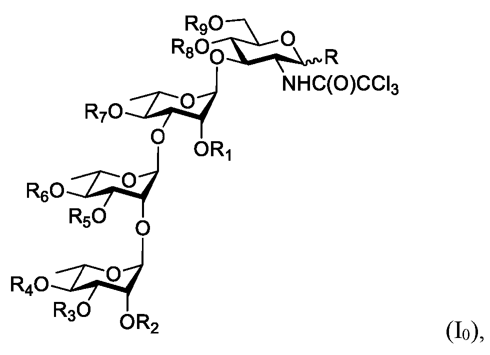 wherein :
R is O-allyl (All), O-triisopropylsilyl (TIPS), O-*tert*-butyldiphenylsilyl (TBDPS), O*-tert*butyldimethylsilyl (TBS or TBDMS), O-thexyl dimethyl silyl (TDS), *para*-methoxyphenyl (PMP) or SR₀, with R₀ being as the compound is a thioglycoside;
R₁ is chloroacetyl (CA), bromoacetyl (BA), or acetyl (Ac), and R₂ is levulinoyl (Lev), fluorenylmethoxycarbonyl (Fmoc), or pentafluorophenyl ester (PFP);
or R₂ is chloroacetyl (CA), bromoacetyl (BA), or acetyl (Ac), and R₁ is levulinoyl (Lev), fluorenylmethoxycarbonyl (Fmoc), or pentafluorophenyl ester (PFP);
R₃ is *tert*-butyldimethyl silyl (TBS), triethylsilyl (TES), O-triisopropylsilyl (TIPS), 2-methylnaphthyl (Nap), *para*-methoxybenzyl (PMB), *para*-bromobenzyl (PBB), *para-*chlorobenzyl (PCB),*para*-nitrobenzyl, *ortho*-nitrobenzyl, 2-pyridylmethyl (picolinyl), picoloyl ester (pico), or allyl (All);
R₄, R5 and R₆ are benzyl (Bn), or XXX;
R₇ is 2-methylnaphthyl (Nap), *para*-methoxybenzyl (PMB), *para*-bromobenzyl (PBB), *para-*chlorobenzyl (PCB), *tert*-butyldimethyl silyl (TBS), triethylsilyl (TES), O-triisopropylsilyl (TIPS), *para*-nitrobenzyl, *ortho*-nitrobenzyl, 2-pyridylmethyl (picolinyl), picoloyl ester (pico), allyloxycarbonyl (Alloc) or allyl (All);
none or only one of R, OR₃ and OR₇ being OAll;
R₇ being different from PBB and PCB when at least one of R₄, R₅ and R₆ is PBB or PCB;
with R₃ being TBS or TES, and R being different from TIPS, TBDPS, TBS and TDS, when R₇ is Nap, PMB or PBB,
with R₃ being Nap, PMB or PBB when R₇ is TBS or TES, and R being different from TIPS, TBDPS, TBS and TDS;
with R₃ being Nap and R₇ being PMB or PBB, or R₇ being Nap and R₃ being PMB or PBB, when R is TIPS, TBDPS, TBS or TDS,
with R₃ being TBS, TES, TIPS, PMB, PCB, PBB, *para*-nitrobenzyl, *ortho*-nitrobenzyl, picolinyl, or pico, when R₇ is Nap, and R₃ being Nap, PMB, PCB or PBB, *para*-nitrobenzyl, *ortho*-nitrobenzyl, picolinyl, or pico, when R₇ is TBS, TES, or TIPS;
and with R₇ being TBS, TES, TIPS, PMB, PCB, PBB, *para*-nitrobenzyl, *ortho*-nitrobenzyl, picolinyl, or pico, when R₃ is Nap, and R₇ being Nap, PMB, PCB or PBB, *para*-nitrobenzyl, *ortho*-nitrobenzyl, picolinyl, or pico, when R₃ is TBS, TES, or TIPS;
R₈ and R₉ form together a benzylidene acetal (Bzl), cyclohexylidene acetal or isopropylidene acetal, or, when XXX is not Nap, *para*-nitrobenzylidene acetal or naphthylidene acetal;
or of following formula (I) : 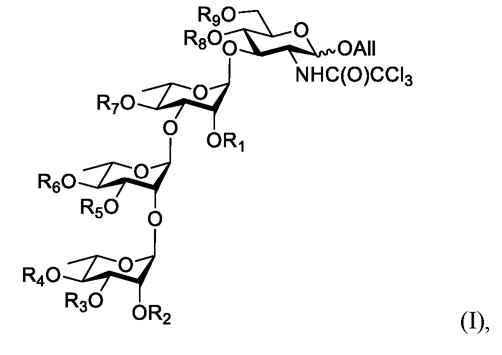 wherein :
All is allyl;
R₁ is chloroacetyl (CA), bromoacetyl (BA), or acetyl (Ac);
R₂ is levulinoyl (Lev) or fluorenylmethoxycarbonyl (Fmoc) or pentafluorophenyl ester (PFP), in particular Lev or Fmoc, even more particularly Lev;
R₃ is *tert*-butyldimethyl silyl (TBS), triethylsilyl (TES), 2-methylnaphthyl (Nap), *para-*methoxyphenyl (PMB) or *para*-bromobenzyl (PBB);
R₄, R₅ and R₆ are independently chosen from arylmethyl protecting groups, in particular benzyl (Bn), *para*-chlorobenzyl (PCB), *para*-bromobenzyl (PBB), *para*-nitrobenzyl or *ortho-*nitrobenzyl;
R₇ is 2-methylnaphthyl (Nap), *para*-methoxyphenyl (PMB), *para*-bromobenzyl (PBB), *tert-*butyldimethyl silyl (TBS) or triethylsilyl (TES);
R₇ being different from PBB and PCB when at least one of R₄, R₅ and R₆ is PBB or PCB; with R₃ being TBS or TES, and R being different from TIPS, TBDPS, TBS and TDS, when R₇ is Nap, PMB or PBB,
with R₃ being Nap, PMB or PBB when R₇ is TBS or TES, and R being different from TIPS, TBDPS, TBS and TDS;
with R₃ being Nap and R₇ being PMB or PBB, or R₇ being Nap and R₃ being PMB or PBB, when R is TIPS, TBDPS, TBS or TDS,
with R₃ being TBS, TES, TIPS, PMB, PCB, PBB, *para*-nitrobenzyl, *ortho*-nitrobenzyl, picolinyl, or pico, when R₇ is Nap, and R₃ being Nap, PMB, PCB or PBB, *para*-nitrobenzyl, *ortho*-nitrobenzyl, picolinyl, or pico, when R₇ is TBS, TES, or TIPS;
and with R₇ being TBS, TES, TIPS, PMB, PCB, PBB, *para*-nitrobenzyl, *ortho*-nitrobenzyl, picolinyl, or pico, when R₃ is Nap, and R₇ being Nap, PMB, PCB or PBB, *para*-nitrobenzyl, *ortho*-nitrobenzyl, picolinyl, or pico, when R₃ is TBS, TES, or TIPS;
R₈ and R₉ form together a benzylidene acetal (Bzl), cyclohexylidene acetal or isopropylidene acetal, or, when XXX is not Nap, *para*-nitrobenzylidene acetal or naphthylidene acetal,
or of following formula (I₁) : 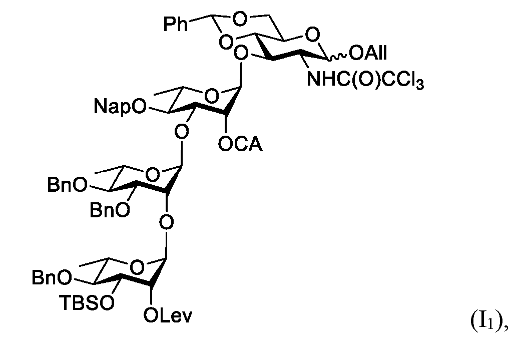 wherein :
All is allyl;
CA is chloroacetyl;
Lev is levulinoyl;
TBS is *tert*-butyldimethyl silyl;
Bn is benzyl;
Nap is 2-methylnaphthyl.

3. A process of preparation of a compound according to claim 2, comprising a step of contacting a donor of formula (I_{A}) with an acceptor of formula (I_{B}) to yield said compound of formula (I),
said formula (I_{A}) being as follows: 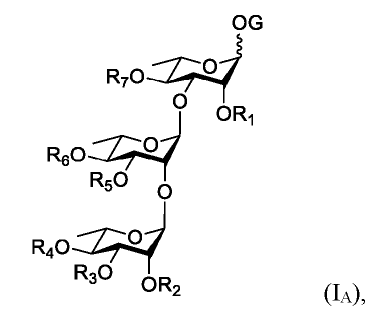 Wherein:
G is an activating group, in particular *N-*phenyltrifluoroacetimidyl (PTFA) or trichloroacetimidyl (TCA);
R₁-R₇ being as defined above;
said formula (I_{B}) being as follows: 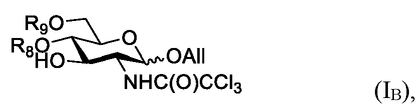
R₈-R₉ being as defined above.

4. A penta- or hexasaccharide compound of following formula (II) : 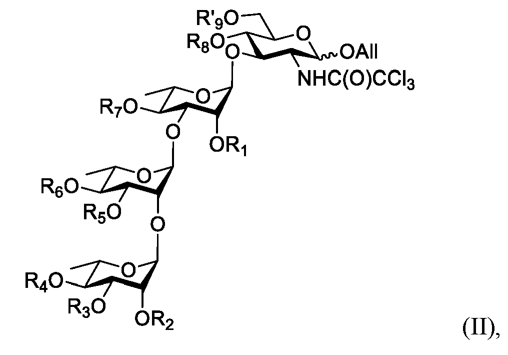 wherein :
one or two of R₈, R₇ and R₃ represent(s) a protected residue α-D-Gl*cp*-;
the other R₁-R₇ groups are as defined in claim 1;
when Rs represents a protected residue α-D-Gl*cp*-, R'₉ is a protecting groups that is orthogonal R₂, R₃ and R₇, and R₃ may also represent Bn;
and when R₈ does not represent a protected residue α-D-Gl*cp*-, R₈ and R'₉ form together a benzylidene acetal (Bzl) or isopropylidene acetal.

5. A pentasaccharide compound of following formula (II) : 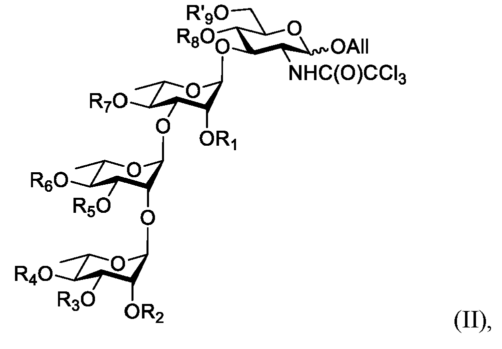 wherein :
R₈, R₇ or R₃ represents a protected residue α-D-Gl*cp*-;
the other R₁-R₇ groups are as defined in claim 1;
when R₈ represents a protected residue α-D-Gl*cp*-, R'₉ is chosen from Bn,Nap, PMB, PBB, and,
when R₃ is not TBS or TES, TBDPS, TES, TBS and thexyl (TDS), notably TBDPS and TES, and wherein R₃ may also represent Bn,
and when R₈ does not represent a protected residue α-D-Gl*cp*-, R₈ and R₉ form together a benzylidene acetal (Bzl), cyclohexylidene acetal or isopropylidene acetal, or, when R₃ and R₇ are not Nap, *para*-nitrobenzylidene acetal or naphthylidene acetal.

6. A process of preparation of a pentasaccharide SF1b'-OAll according to claim 5, wherein:
SF1b is ABC(E)D, with A is 2)-α-L-Rha*p*-(1→ , B is 2)-α-L-Rha*p*-(1→ , C is 3)-α-L-Rha*p*-(1→ , D is 3)-α-D-Gl*cp*NAc-(1→ or 3)-β-D-Gl*cp*NAc-(1→ , E represents a residue α-D-Glc*p*- ;
SF1b being in particular *O*-acetylated in position 2c; and
' denotes that the pentasaccharide is protected as defined below,
said process comprising the following steps:
(iii) A step of deprotection of the R₈ group of a compound of formula (I), in particular by deprotecting both R₈ and R₉ using for example SnCl₄, camphorsulfonic acid (CSA) or trifluoroacetic acid (TFA), and then by protecting the obtained compound with a R'₉ group using for example R'₉-Br and Taylor reagent, R'₉-Br with Bu₂SnO activation, R'₉-TCA or R'₉-PTFA, R'₉ being as defined above, to obtain an acceptor compound of following formula: 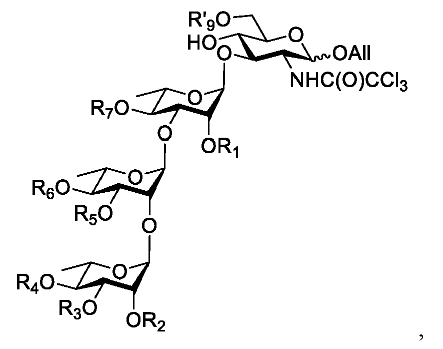
wherein R₁-R₇ and R'₉ are as defined above;
(iv) A step of reacting the acceptor obtain in step (i) with the donor compound E-G of following formula: 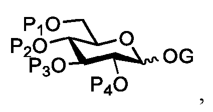 wherein :
P₁, P₂, P₃ and P₄ are independently chosen from arylmethyl protecting groups, in particular Bn, PCB, PBB, PMB, *para*-nitrobenzyl or *ortho*-nitrobenzyl, and Nap,
G is an activating group, in particular *N*-phenyltrifluoroacetimidyl (PTFA), or trichloroacetimidyl (TCA),
to yield the pentasaccharide SF1b'-OAll of following formula: 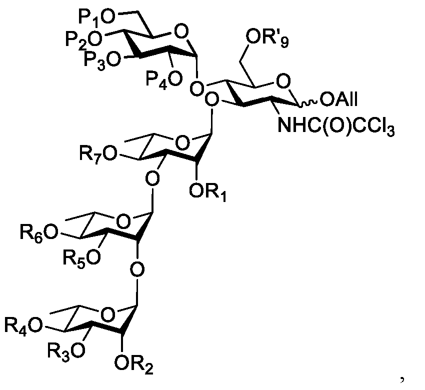
Wherein R₁-R₇ and R'₉, P₁, P₂, P₃ and P₄ are as defined above;
or of a pentasaccharide SF2a'-OAll according to claim 5, wherein:
SF2a is AB(E)CD, with A is 2)-α-L-Rha*p*-(1→ , B is 2)-α-L-Rha*p*-(1→ , C is 3)-α-L-Rha*p*-(1→ , D is 3)-α -D-Glc*p*NAc-(1→ or 3)-β-D-Glc*p*NAc-(1→ , E represents a residue α-D-Glc*p*- ; and ' denotes that the pentasaccharide is protected as defined below,
said process comprising the following steps:
(iii) A step of deprotection of the R₇ group of a compound of formula (I), for example using 2,3-dichloro-5,6-dicyano-p-benzoquinone (DDQ) and optionally β-pinene when R₇ is Nap, to obtain an acceptor compound of following formula: 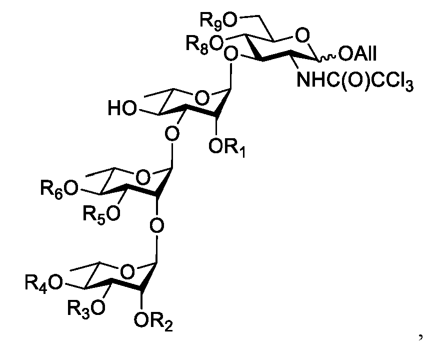 wherein R₁-R₆ and R₈-R₉ are as defined above ;
(iv) A step of reacting the acceptor obtain in step (i) with the donor compound E-G of following formula: 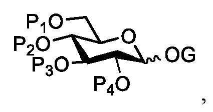 wherein :
P₁, P₂, P₃ and P₄ are as defined above,
G is an activating group, in particular *N*-phenyltrifluoroacetimidyl (PTFA), or trichloroacetimidyl (TCA),
to yield the pentasaccharide SF2a'-OAll of following formula: 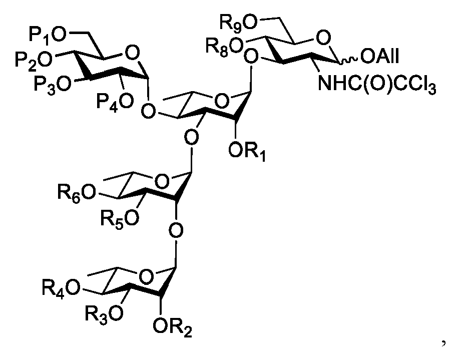
wherein R₁-R₆, R₈-R₉, P₁, P₂, P₃ and P₄ are as defined above;
or of a pentasaccharide SF3a'-OAll according to claim 5, wherein:
SF3a is (E)ABCD, with A is 2)-α-L-Rha*p*-(1→ , B is 2)-α-L-Rha*p*-(1→ , C is 3)-α-L-Rha*p*-(1→ , D is 3)-α -D-Glc*p*NAc-(1→ or 3)-β-D-Glc*p*NAc-(1→ , E represents a residue α-D-Glc*p*- ;
SF3a being in particular *O*-acetylated in position 2c; and
' denotes that the pentasaccharide is protected as defined below,
said process comprising the following steps:
(iii) A step of deprotection of the R₃ group of a compound of formula (I), for example using triethylamine trihydrofluoride (TEA.3HF) when R₃ is TBS, to obtain an acceptor compound of following formula: 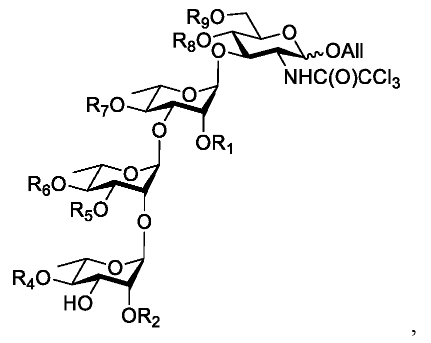 wherein R₁-R₂, and R₄-R₉ are as defined above;
(iv) A step of reacting the acceptor obtain in step (i) with the donor compound E-G of following formula: 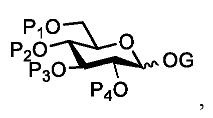 wherein :
P₁, P₂, P₃ and P₄ are as defined above,
G is an activating group, in particular *N*-phenyltrifluoroacetimidyl (PTFA), or trichloroacetimidyl (TCA),
to yield the pentasaccharide SF3a'-OAll of following formula:
wherein R₁-R₂, R₄-R₉, P₁, P₂, P₃ and P₄ are as defined above.

7. A pentasaccharide acceptor compound of following formula (III_{A}) : 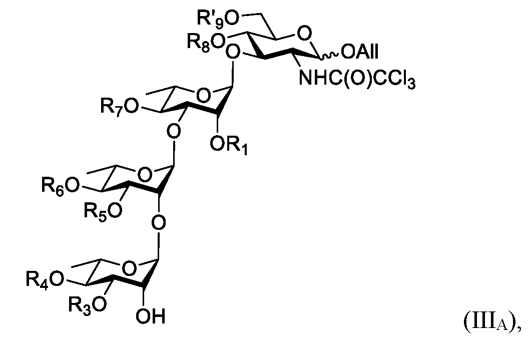 wherein :
R₈, R₇ or R₃ represents a protected residue α-D-Glc*p*- ;
the other R₁, R₃-R₈ and R'₉ being as defined above.

8. A process of preparation of a pentasaccharide acceptor compound of formula (III_{A}) according to claim 7, comprising a step of deprotection of group R₂ of a compound of formula (II), in particular using hydrazine acetate or hydrazine in a mixture pyridine/acetic acid, in particular in a 1:1 (v/v) pyridine/acetic acid mixture when R₂ is Lev.

9. A pentasaccharide compound of following formula (III_{DHF}) : 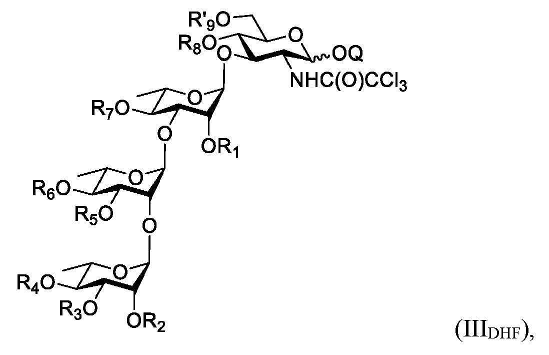 wherein :
Q is H, G or LZ,
G is an activating group, in particular N phenyltrifluoroacetimidyl (PTFA), or
trichloroacetimidyl (TCA),
L, which is optionally protected is:
- a single bond,
- a divalent C₁-C₁₂ alkyl chain optionally interrupted by one or more heteroatoms, notably selected from an oxygen atom, a sulphur atom or a nitrogen atom, said nitrogen and sulphur atoms being optionally oxidized, and the nitrogen atom being optionally involved in an acetamide bond, or
- a divalent C₁-C₁₂ alkyl chain substituted by at least one -OH group, being in particular of the following formula -(CH₂-CH₂-C(OH))_{q}-(CH₂-CH₂)ᵢ, wherein i is 0 or 1 and q ranges from 1 to 10,
Z is a terminal function or group, optionally protected, able to form a covalent bond with a compound enabling to extend said LZ chain, a carrier and/or a solid support, or a multivalent scaffold; an anchor; a mono-, oligo- or polysaccharide; or a dye or fluorescent residue in particular a pentasaccharide donor compound of following formula (III_{D}) : 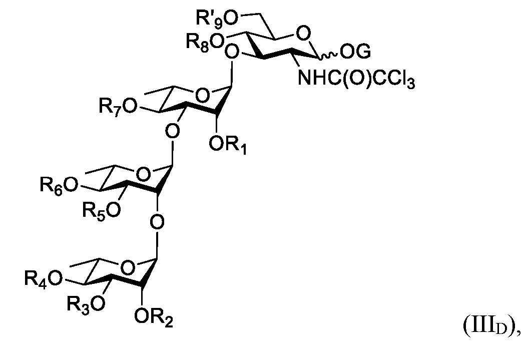 wherein :
G is an activating group, in particular N-phenyltrifluoroacetimidyl (PTFA), or trichloroacetimidyl (TCA),
Rs, R₇ or R₃ represents a protected residue α-D-Glc*p*- ;
the other R₁-R₈ and R'₉ groups are as defined above;
or in particular a pentasaccharide compound of following formula (III_{H}) : 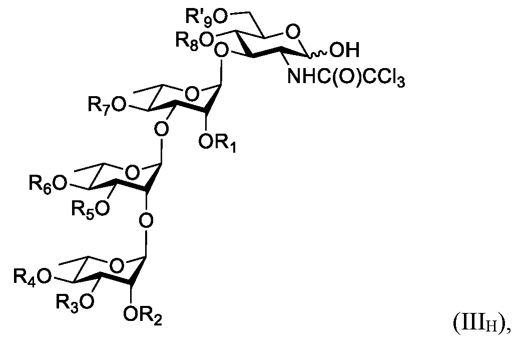 wherein :
Rs, R₇ or R₃ represents a protected residue α-D-Glc*p*- ;
the other R₁-R₈ and R'₉ groups are as defined above;
or in particular a functionalized pentasaccharide compound of following formula (III_{F}) : 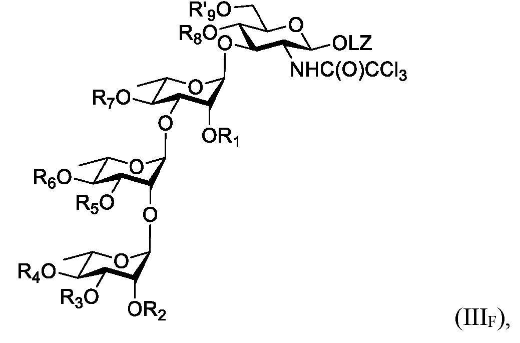 wherein :
L and Z are as defined above;
Rs, R₇ or R₃ represents a protected residue α-D-Glc*p*- ;
the other R₁-R₈ and R'₉ groups are as defined above,
or in particular a functionalized pentasaccharide acceptor compound of following formula (III_{AF}) : 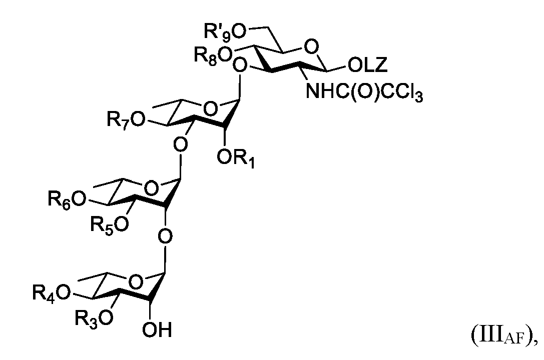
wherein Rs, R₇ or R₃ represents a protected residue α-D-Glc*p*- ;
the other R₁-R₈ and R'₉ groups, L and Z are as defined above.

10. A process of preparation of:
a pentasaccharide donor compound of formula (III_{D}) according to claim 9, comprising a first step of deprotection of the All group, in particular using PdCl₂ and then N-iodosuccinimide (NIS), or using iridium, to obtain a compound of formula (III_{H}), and a second step of contacting the obtained compound with a compound of type G-LG wherein G is as defined in claim 5, and LG is a leaving group, for example PTFA-Cl when G is PTFA,
or a functionalized pentasaccharide compound of following formula (III_{F}), comprising a step of contacting a pentasaccharide donor compound of formula (III_{D}) with a compound of formula HO-LZ', wherein Z' is Z, optionally protected, or a group enabling to form Z, in particular in presence of TMSOTf, TfOH, TBSOTf, AgOTf, Tf₂O, Bi(OTf)₃, Yb(OTf)₃, I₂/Et₃SiH, TMSB(C₆F₅)₄, acid-washed molecular sieves, ZnBr₂, or BF₃-Et₂O, notably in catalytical conditions,
or of a functionalized pentasaccharide acceptor compound of following formula (III_{AF}), comprising a step of deprotection of the group R₂ of a compound of formula (III_{F}), in particular using hydrazine acetate or hydrazine in a mixture pyridine/acetic acid, in particular in a 1:1 (v/v) pyridine/acetic acid mixture when R₂ is Lev.

11. A polysaccharide compound of formula R₂-P'-OQ', wherein P' is constituted of or comprises at least two units, and in particular at most nine or ten units, chosen from -SF1b'-, - SF2a'-, and/or -SF3a'- of following formulae: 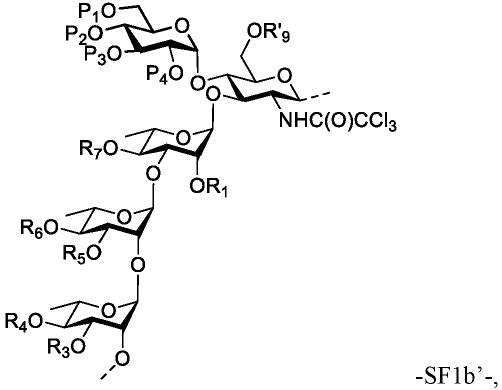 R₁ being in particular Ac, more particularly with P' being constituted of or comprising at least two units chosen from -SF1b'-, -SF2a'-, and -SF3a'-, at least two of these units being different, 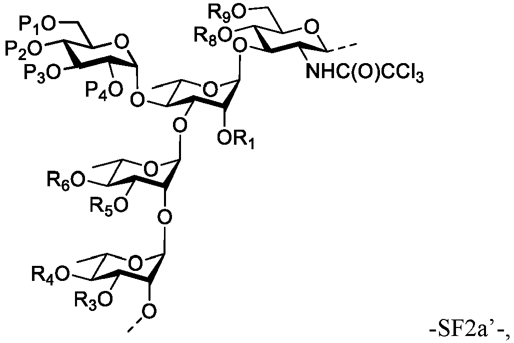 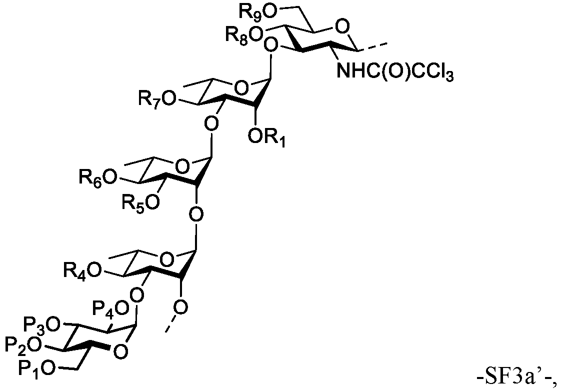 R₁ being in particular Ac, more particularly with P' being constituted of or comprising at least two units chosen from -SF1b'-, -SF2a'-, and -SF3a'-, at least two of these units being different,
wherein R₁-R₉ and R'₉, P₁, P₂, P₃, and P₄ are as defined above,
and wherein Q' is H, All, or LZ as defined above.

12. The polysaccharide compound according to claim 11, wherein P' is constituted of or comprises at least two units chosen from -SF1b'-, -SF2a'-, -SF3a'- and -ABCD'- of following formula: 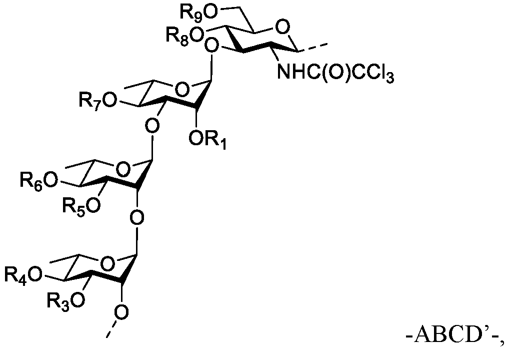
wherein R₁ and R₃-R₉ are as defined above,
P' comprising at least one unit, in particular at least two units chosen from -SF1b'-, -SF2a'-, - SF3a'-, and at least one and -ABCD'- unit, in particular one -ABCD'- unit, more particularly between two units chosen from -SF1b'-, -SF2a'-, -SF3a'-.

13. A process of preparation of a polysaccharide as defined in claim 11 or 12, comprising:
**a)** a step of contacting a pentasaccharide acceptor compound of formula (III_{A}) or (III_{AF}), or a ABCD acceptor, and a pentasaccharide donor compound of formula (III_{D}) or a ABCD donor;
and optionally:
**b)** a step:
- of converting the polysaccharide obtained in a previous step into an acceptor by deprotecting the R₂ group, in particular using hydrazine acetate or hydrazine in a mixture pyridine/acetic acid, in particular in a 1:1 (v/v) pyridine/acetic acid mixture when R₂ is Lev; or
- of converting the polysaccharide obtained in a previous step into a donor by first deprotecting the All group, in particular in particular using PdCl₂ and then N-iodosuccinimide (NIS), and then of contacting the obtained compound with a compound of type D'-LG wherein D' is a donor group and LG is a leaving group, for example PTFA-Cl when D' is PTFA;
and
**c)** a step of contacting the acceptor or donor obtained in step b) with a pentasaccharide donor compound of formula (III_{D}) or a ABCD donor; or a pentasaccharide acceptor compound of formula (III_{A}) or (III_{AF}) or a ABCD acceptor, respectively, or an acceptor or donor obtained in one of the previous steps, respectively; step b) and c) being repeated if necessary;
and/or
**d)** a step of functionalizing if necessary the polysaccharide obtained in a previous step by deprotecting the All group, in particular using PdCl₂ and then N-iodosuccinimide (NIS), contacting the obtained compound with a compound of type D'-LG wherein D' is a donor group and LG is a leaving group, for example PTFA-Cl when D' is PTFA, and then contacting the obtained donor with a compound of formula HO-LZ, in particular in presence of TMSOTf, TfOH, TBSOTf, AgOTf, Tf₂O, Bi(OTf)₃, Yb(OTf)₃, I₂/Et₃SiH, TMSB(C₆F₅)₄, acid-washed molecular sieves, ZnBr₂, or BF₃-Et₂O, notably in catalytical conditions;
step d) taking place after step c), or after one of steps c) if step c) is repeated), in particular after last step c).

14. A process of preparation of a glycoconjugate of formula H-P-OLZ', wherein:
P is constituted of or comprises at least two units chosen from -SF1b-, -SF2a-, -SF3a- and - ABCD-, wherein:
SF1b is ABC(E)D, SF2a is AB(E)CD, SF3a is (E)ABCD, with A is 2)-α-L-Rha*p*-(1→ , B is 2)-α-L-Rha*p*-(1→ , C is 3)-α-L-Rha*p*-(1→ , D is 3)-α-D-Rha*p*-(1→,or, when D is in terminal position, 3)-α-L-Rha*p*-(1→ or 3)-α-D-Glc*p*NAc-(1→, E represents a residue α-D-Glc*p*-;
L is:
- a single bond,
- a divalent C₁-C₁₂ alkyl, C₂-C₁₂ alkenyl or C₂-C₁₂ alkynyl chain optionally interrupted by one or more heteroatoms, notably selected from an oxygen atom, a sulphur atom or a nitrogen atom, said nitrogen and sulphur atoms being optionally oxidized, and the nitrogen atom being optionally involved in an acetamide bond, or
- a divalent C₁-C₁₂ alkyl, C₂-C₁₂ alkenyl or C₂-C₁₂ alkynyl chain substituted by at least one -OH group, being in particular of the following formula -(CH₂-CH₂-C(OH))_{q}-(CH₂-CH₂)ᵢ, wherein i is 0 or 1 and q ranges from 1 to 10,
Z' is Z₁ or F₁-L₂-Z₂,
Z₁ is a terminal function or group, optionally protected, able to form a covalent bond with a carrier and/or a solid support, or a multivalent scaffold; an anchor; a mono-, oligo- or
polysaccharide; or a dye or fluorescent residue.
F₁ is any group enabling to bond the linker L to the linker L₂, F₁ being in particular chosen from the -C(=O)-, -C(=O)-C(=O)-, -C(=O)-C(=O)-NH-, -NHC(=O)-C(=O)-, -NHC(=O)-C(=O)-NH-, -C(=O)-C(H)=N-NH-, -NH-C(=O)-C(H)=N-NH-, ester, amide, amine, -CH₂-, ether, thioether, imine, thio-succinimide, oxime, hydrazone, hydrazonamide, -C(=O)CH₂-NH-, -NH-CH₂-C(=O)-, triazole functions or groups, and from the following: 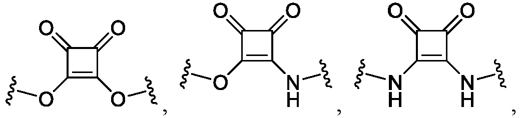
L₂ is a single bond, divalent C₁-C₁₂ alkyl, C₂-C₁₂ alkenyl or C₂-C₁₂ alkynyl chain optionally interrupted by one or more heteroatoms, notably selected from an oxygen atom, a sulphur atom or a nitrogen atom, said nitrogen and sulphur atoms being optionally oxidized, and the nitrogen atom being optionally involved in an acetamide bond,
Z₂ is Z₁ or F₂-L₃-Z₁,
F₂ is any group enabling to bond the linker L to the linker L₃, F₁ being in particular chosen from the -C(=O)-, -C(=O)-C(=O)-, -C(=O)-C(=O)-NH-, -NHC(=O)-C(=O)-, -NHC(=O)-C(=O)-NH-, -C(=O)-C(H)=N-NH-, -NH-C(=O)-C(H)=N-NH-, ester, amide, amine, -CH₂-, ether, thioether, imine, thio-succinimide, oxime, hydrazone, hydrazonamide, -C(=O)CH₂-NH-, -NH-CH₂-C(=O)-, triazole functions or groups, and from the following: 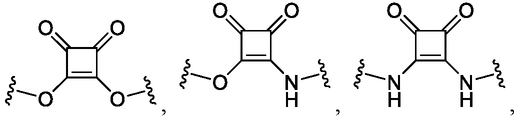
L₃ is single bond, a divalent C₁-C₁₂ alkyl, C₂-C₁₂ alkenyl or C₂-C₁₂ alkynyl chain optionally interrupted by one or more heteroatoms, notably selected from an oxygen atom, a sulphur atom or a nitrogen atom, said nitrogen and sulphur atoms being optionally oxidized, and the nitrogen atom being optionally involved in an acetamide bond,
said process comprising :
**a)** One or more steps of full deprotection of a polysaccharide of formula R₂-P'-LZ as defined above, to obtain a H-P-OLZ₁ compound or a H-P-OLF₁ compound, L and Z₁ being as defined above, F₁' being a precursor of F₁ as defined above;
**b)** Optionally, a step of contacting the fully deprotected polysaccharide obtained in the previous step with:
- a compound of following formula F₁"-L₂-Z₁, F₁" being a precursor of F₁ as defined above, L₂ and Z₁ being as defined above, or
- a compound of following formula F₁"-L₂-F₂', F₁" being a precursor of F₁ as defined above, F₂' being a precursor of F₂ as defined above, L₂ being as defined above, followed by contacting the obtained compound with a compound of following formula F₂"-L₃-Z₁, wherein F₂" is a precursor of F₂ as defined above, and L₃ and Z₁ being as defined above,

15. A glycoconjugate of formula H-P-OLZ', as defined above, wherein P is constituted of or comprises:
- at least two different units chosen from -SF1b-, -SF2a-, -SF3a-;
- at least one -SF1b- unit, with LZ' being different from propyl when P is constituted of or comprises one -SF1b- unit; or
- at least one -ABCD- unit, in particular one -ABCD- unit, and at least one unit chosen from -SF1b-, -SF2a-, -SF3a-, and
And wherein:
- the ABCD units are optionally substituted by at least one phosphoethanolamine (PEtN-modified); and/or
- the units, in particular the -SF1b-, -SF2a-, -SF3a- units are optionally acetylated, said acetylation being partial or stoichiometric, -SF3a- being in particular stoichiometrically acetylated in position 2c, -SF1b- being in particular acetylated, more particularly partially, in position 2c.
